# EUROPEAN PATENT SPECIFICATION

(11) **EP 2 446 900 B1**
(45) Date of publication and mention of the grant of the patent: **29.03.2017**
(21) Application number: 10184424.9
(22) Date of filing: 09.06.2005
(51) Int. Cl.: A61K 39/395, A61P 3/10, A61K 31/00, A61K 38/02, A61P 5/00, C07K 16/40

(54) **Methods for treating conditions associated with MASP-2 dependent complement activation**
Verfahren zur Behandlung von Leiden in Zusammenhang mit MASP-2-abhängiger Komplementaktivierung
Procédés pour le traitement d'états pathologiques liés à l'activation du complément MASP-2 dépendant

(43) Date of publication of application: 02.05.2012
(62) Divisional of application: 05765604.3
(73) Proprietor: Omeros Corporation, Seattle, WA 98119 (US); UNIVERSITY OF LEICESTER, Leicester, Leicestershire LE1 7RH (GB)
(72) Inventor: Schwaeble, Hans-Wilhelm, Mountsorrel, Leicestershire LE12 7AF (GB); Stover, Cordula M., Wigston Meadows LE18 3QF (GB); Tedford, Clark E., Poulsbo WA 98370 (US); Parent, James B., Bainbridge Island WA 98110 (US); Fujita, Teizo, Fukushima 960-8157 (JP)
(74) Representative: Cole, William Gwyn

(56) References cited:
- WO-A-2004/075837
- WO-A-2004/106384
- HANSEN TROELS K ET AL: "Association between mannose-binding lectin and vascular complications in type 1 diabetes.", DIABETES JUN 2004 LNKD- PUBMED:15161763, vol. 53, no. 6, June 2004 (2004-06), pages 1570-1576, XP002671481, ISSN: 0012-1797
- ZHANG JING ET AL: "Early complement activation and decreased levels of glycosylphosphatidylinositol-anchored complement inhibitors in human and experimental diabetic retinopathy", DIABETES, AMERICAN DIABETES ASSOCIATION, US, vol. 51, no. 12, 1 December 2002 (2002-12-01), pages 3499-3504, XP002324813, ISSN: 0012-1797, DOI: 10.2337/DIABETES.51.12.3499
- OSTERGAARD ET AL: "Complement activation and diabetic vascular complications", CLINICA CHIMICA ACTA, ELSEVIER BV, AMSTERDAM, NL, vol. 361, no. 1-2, 5 July 2005 (2005-07-05), pages 10-19, XP005092820, ISSN: 0009-8981, DOI: 10.1016/J.CCCN.2005.04.028
- VORUP-JENSEN T ET AL: "DISTINCT PATHWAYS OF MANNAN-BINDING LECTIN (MBL)- AND C1-COMPLEX AUTOACTIVATION REVEALED BY RECONSTITUION OF MBL WITH RECOMBINANT MBL-ASSOCIATED SERINE PROTEASE-2", JOURNAL OF IMMUNOLOGY, THE WILLIAMS AND WILKINS CO. BALTIMORE, US, vol. 165, no. 4, 15 August 2000 (2000-08-15), pages 2093-2100, XP001183499, ISSN: 0022-1767
- COLLARD CHARLES D ET AL: "Endothelial oxidative stress activates the lectin complement pathway: Role of cytokeratin 1", AMERICAN JOURNAL OF PATHOLOGY, vol. 159, no. 3, September 2001 (2001-09), pages 1045-1054, XP002378477, ISSN: 0002-9440
- GERL VERENA B. ET AL: 'Extensive deposits of complement C3d and C5b-9 in the choriocapillaris of eyes of patients with diabetic retinopathy.' INVESTIGATIVE OPHTHALMOLOGY & VISUAL SCIENCE APR 2002 vol. 43, no. 4, April 2002, pages 1104 - 1108 ISSN: 0146-0404
- NICOLOFF GEORGE ET AL: "Circulating immune complexes among diabetic children.", CLINICAL & DEVELOPMENTAL IMMUNOLOGY MAR 2004, vol. 11, no. 1, March 2004 (2004-03), pages 61-66, ISSN: 1740-2522
- ROSOKLIJA G.B. ET AL: 'Local activation of the complement system in endoneurial microvessels of diabetic neuropathy.' ACTA NEUROPATHOLOGICA JAN 2000 vol. 99, no. 1, January 2000, pages 55 - 62 ISSN: 0001-6322

## Description

### FIELD OF THE INVENTION

The present invention relates to methods of inhibiting the adverse effects of MASP-2-dependent complement activation.

### BACKGROUND OF THE INVENTION

The complement system provides an early acting mechanism to initiate and amplify the inflammatory response to microbial infection and other acute insults (Liszewski, M.K. and J.P. Atkinson, 1993, in Fundamental Immunology, Third Edition, edited by W.E. Paul, Raven Press, Ltd., New York). While complement activation provides a valuable first-line defense against potential pathogens, the activities of complement that promote a protective inflammatory response can also represent a potential threat to the host (Kalli, K.R., et al., Springer Semin. Immunopathol. 15:417-431, 1994; Morgan, B.P., Eur. J. Clinical Investig. 24:219-228, 1994). For example, C3 and C5 proteolytic products recruit and activate neutrophils. These activated cells are indiscriminate in their release of destructive enzymes and may cause organ damage. In addition, complement activation may cause the deposition of lytic complement components on nearby host cells as well as on microbial targets, resulting in host cell lysis.

The complement system has been implicated as contributing to the pathogenesis of numerous acute and chronic disease states, including: myocardial infarction, revascularization following stroke, ARDS, reperfusion injury, septic shock, capillary leakage following thermal bums, postcardiopulmonary bypass inflammation, transplant rejection, rheumatoid arthritis, multiple sclerosis, myasthenia gravis, and Alzheimer's disease. In almost all of these conditions, complement is not the cause but is one of several factors involved in pathogenesis. Nevertheless, complement activation may be a major pathological mechanism and represents an effective point for clinical control in many of these disease states. The growing recognition of the importance of complement-mediated tissue injury in a variety of disease states underscores the need for effective complement inhibitory drugs. No drugs have been approved for human use that specifically target and inhibit complement activation.

Currently, it is widely accepted that the complement system can be activated through three distinct pathways: the classical pathway, the lectin pathway, and the alternative pathway. The classical pathway is usually triggered by antibody bound to a foreign particle (i.e., an antigen) and thus requires prior exposure to that antigen for the generation of specific antibody. Since activation of the classical pathway is associated with development of an immune response, the classical pathway is part of the acquired immune system. In contrast, both the lectin and alternative pathways are independent of clonal immunity and are part of the innate immune system.

The first step in activation of the classical pathway is the binding of a specific recognition molecule, C1q, to antigen-bound IgG and IgM. The activation of the complement system results in the sequential activation of serine protease zymogens. C1q is associated with the Clr and Cls serine protease proenzymes as a complex called Cl and, upon binding of C1q to an immune complex, autoproteolytic cleavage of the Arg-Ile site of Clr is followed by Clr activation of Cls, which thereby acquires the ability to cleave C4 and C2. The cleavage of C4 into two fragments, designated C4a and C4b, allows the C4b fragments to form covalent bonds with adjacent hydroxyl or amino groups and the subsequent generation of C3 convertase (C4b2b) through noncovalent interaction with the C2b fragment of activated C2. C3 convertase (C4b2b) activates C3 leading to generation of the C5 convertase (C4b2b3b) and formation of the membrane attack complex (C5b-9) that can cause microbial lysis. The activated forms of C3 and C4 (C3b and C4b) are covalently deposited on the foreign target surfaces, which are recognized by complement receptors on multiple phagocytes.

Independently, the first step in activation of the complement system by the lectin pathway is also the binding of specific recognition molecules, which is followed by the activation of associated serine proteases. However, rather than the binding of immune complexes by C1q, the recognition molecules in the lectin pathway are carbohydrate-binding proteins (mannan-binding lectin (MBL), H-ficolin, M-ficolin and L-ficolin) (Lu, J., et al., Biochim. Biophys. Acta 1572:387-400, 2002; Holmskov et al., Annu. Rev. Immunol. 21: 547-578 (2003); Teh et al., Immunology 101: 225-232 (2000)). Ikeda et al. first demonstrated that, like C1q, MBL could activate the complement system upon binding to yeast mannan-coated erythrocytes in a C4-dependent manner (Ikeda, K., et al., J. Biol. Chem. 262:7451-7454, 1987). MBL, a member of the collectin protein family, is a calcium-dependent lectin that binds carbohydrates with 3- and 4-hydroxy groups oriented in the equatorial plane of the pyranose ring. Prominent ligands for MBL are thus D-mannose and N-acetyl-D-glucosamine, while carbohydrates not fitting this steric requirement have undetectable affinity for MBL (Weis, W.I., et al., Nature 360:127-134, 1992). The interaction between MBL and monovalent sugars is extremely weak, with dissociation constants typically in the 2 mM range. MBL achieves tight, specific binding to glycan ligands by interaction with multiple monosaccharide residues simultaneously (Lee, R.T., et al., Archiv. Biochem. Biophys. 299:129-136, 1992). MBL recognizes the carbohydrate patterns that commonly decorate microorganisms such as bacteria, yeast, parasites and certain viruses. In contrast, MBL does not recognize D-galactose and sialic acid, the penultimate and ultimate sugars that usually decorate "mature" complex glycoconjugates present on mammalian plasma and cell surface glycoproteins. This binding specificity is thought to help protect from self activation. However, MBL does bind with high affinity to clusters of high-mannose "precursor" glycans on N-linked glycoproteins and glycolipids sequestered in the endoplasmic reticulum and Golgi of mammalian cells (Maynard, Y., et al., J. Biol. Chem. 257:3788-3794, 1982). Therefore, damaged cells are potential targets for lectin pathway activation via MBL binding.

The ficolins possess a different type of lectin domain than MBL, called the fibrinogen-like domain. Ficolins bind sugar residues in a Ca⁺⁺-independent manner. In humans, three kinds of ficolins, L-ficolin, M-ficolin and H-ficolin, have been identified. Both serum ficolins L-ficolin and H-ficolin have in common a specificity for N-acetyl-D-glucosamine; however, H-ficolin also binds N-acetyl-D-galactosamine. The difference in sugar specificity of L-ficolin, H-ficolin and MBL means that the different lectins may be complementary and target different, though overlapping, glycoconjugates. This concept is supported by the recent report that, of the known lectins in the lectin pathway, only L-ficolin binds specifically to lipoteichoic acid, a cell wall glycoconjugate found on all Gram-positive bacteria (Lynch, N.J., et al., J. Immunol. 172:1198-1202, 2004). The collectins (i.e., MBL) and the ficolins bear no significant similarity in amino acid sequence. However, the two groups of proteins have similar domain organizations and, like C1q, assemble into oligomeric structures, which maximize the possibility of multisite binding. The serum concentrations of MBL are highly variable in healthy populations and this is genetically controlled by the polymorphism/mutations in both the promoter and coding regions of the MBL gene. As an acute phase protein, the expression of MBL is further upregulated during inflammation. L-ficolin is present in serum at similar concentrations as MBL. Therefore, the L-ficolin arm of the lectin pathway is potentially comparable to the MBL arm in strength. MBL and ficolins can also function as opsonins, which require interaction of these proteins with phagocyte receptors (Kuhlman, M., et al., J. Exp. Med. 169:1733, 1989; Matsushita, M., et al., J. Biol. Chem. 271:2448-54, 1996). However, the identities of the receptor(s) on phagocytic cells have not been established.

Human MBL forms a specific and high affinity interaction through its collagen-like domain with unique Clr/Cls-like serine proteases, termed MBL-associated serine proteases (MASPs). To date, three MASPs have been described. First, a single enzyme "MASP" was identified and characterized as the enzyme responsible for the initiation of the complement cascade (i.e., cleaving C2 and C4) (Ji, Y.H., et al., J. Immunol. 150:571-578, 1993). Later, it turned out that MASP is in fact a mixture of two proteases: MASP-1 and MASP-2 (Thiel, S., et al., Nature 386:506-510, 1997). However, it was demonstrated that the MBL-MASP-2 complex alone is sufficient for complement activation (Vorup-Jensen, T., et al., J. Immunol. 165:2093-2100, 2000). Furthermore, only MASP-2 cleaved C2 and C4 at high rates (Ambrus, G., et al., J. Immunol. 170:1374-1382, 2003). Therefore, MASP-2 is the protease responsible for activating C4 and C2 to generate the C3 convertase, C4b2b. This is a significant difference from the Cl complex, where the coordinated action of two specific serine proteases (C1r and C1s) leads to the activation of the complement system. Recently, a third novel protease, MASP-3, has been isolated (Dahl, M.R., et al., Immunity 15:127-35, 2001). MASP-1 and MASP-3 are alternatively spliced products of the same gene. The biological functions of MASP-1 and MASP-3 remain to be resolved.

MASPs share identical domain organizations with those of Clr and Cls, the enzymatic components of the Cl complex (Sim, R.B., et al., Biochem. Soc. Trans. 28:545, 2000). These domains include an N-terminal Clr/Cls/sea urchin Vegf/bone morphogenic protein (CUB) domain, an epidermal growth factor-like domain, a second CUB domain, a tandem of complement control protein domains, and a serine protease domain. As in the C1 proteases, activation of MASP-2 occurs through cleavage of an Arg-Ile bond adjacent to the serine protease domain, which splits the enzyme into disulfide-linked A and B chains, the latter consisting of the serine protease domain. Recently, a genetically determined deficiency of MASP-2 was described (Stengaard-Pedersen, K., et al., New Eng. J. Med. 349:554-560, 2003). The mutation of a single nucleotide leads to an Asp-Gly exchange in the CUB1 domain and renders MASP-2 incapable of binding to MBL.

MBL is also associated with a nonenzymatic protein referred to as MBL-associated protein of 19 kDa (MAp19) (Stover, C.M., J. Immunol. 162:3481-90, 1999) or small MBL-associated protein (sMAP) (Takahashi, M., et al., Int. Immunol. 11:859-863, 1999). MAp19 is formed by alternative splicing of the *MASP 2* gene product and comprises the first two domains of MASP-2, followed by an extra sequence of four unique amino acids. The *MASP 1* and *MASP 2* genes are located on chromosomes 3 and 1, respectively (Schwaeble, W., et al., Immunobiology 205:455-466, 2002).

Several lines of evidence suggest that there are different MBL-MASPs complexes and a large fraction of the total MASPs in serum is not complexed with MBL (Thiel, S., et al., J. Immunol. 165:878-887, 2000). Both H- and L-ficolin are associated with MASP and activate the lectin complement pathway, as does MBL (Dahl, M.R., et al., Immunity 15:127-35, 2001; Matsushita, M., et al., J. Immunol. 168:3502-3506, 2002). Both the lectin and classical pathways form a common C3 convertase (C4b2b) and the two pathways converge at this step.

The lectin pathway is widely thought to have a major role in host defense against infection. Strong evidence for the involvement of MBL in host defense comes from analysis of patients with decreased serum levels of functional MBL (Kilpatrick, D.C., Biochim. Biophys. Acta 1572:401-413, 2002). Such patients display susceptibility to recurrent bacterial and fungal infections. These symptoms are usually evident early in life, during an apparent window of vulnerability as maternally derived antibody titer wanes, but before a full repertoire of antibody responses develops. This syndrome often results from mutations at several sites in the collagenous portion of MBL, which interfere with proper formation of MBL oligomers. However, since MBL can function as an opsonin independent of complement, it is not known to what extent the increased susceptibility to infection is due to impaired complement activation.

Although there is extensive evidence implicating both the classical and alternative complement pathways in the pathogenesis of non-infectious human diseases, the role of the lectin pathway is just beginning to be evaluated. Recent studies provide evidence that activation of the lectin pathway can be responsible for complement activation and related inflammation in ischemia/reperfusion injury. Collard et al. (2000) reported that cultured endothelial cells subjected to oxidative stress bind MBL and show deposition of C3 upon exposure to human serum (Collard, C.D., et al., Am. J. Pathol. 156:1549-1556, 2000). In addition, treatment of human sera with blocking anti-MBL monoclonal antibodies inhibited MBL binding and complement activation. These findings were extended to a rat model of myocardial ischemia-reperfusion in which rats treated with a blocking antibody directed against rat MBL showed significantly less myocardial damage upon occlusion of a coronary artery than rats treated with a control antibody (Jordan, J.E., et al., Circulation 104:1413-1418, 2001). The molecular mechanism of MBL binding to the vascular endothelium after oxidative stress is unclear; a recent study suggests that activation of the lectin pathway after oxidative stress may be mediated by MBL binding to vascular endothelial cytokeratins, and not to glycoconjugates (Collard, C.D., et al., Am. J. Pathol. 159:1045-1054, 2001). Other studies have implicated the classical and alternative pathways in the pathogenesis of ischemia/reperfusion injury and the role of the lectin pathway in this disease remains controversial (Riedermann, N.C., et al., Am. J. Pathol. 162:363-367, 2003).

In contrast to the classical and lectin pathways, no initiators of the alternative pathway have been found to fulfill the recognition functions that C1q and lectins perform in the other two pathways. Currently it is widely accepted that the alternative pathway is spontaneously triggered by foreign or other abnormal surfaces (bacteria, yeast, virally infected cells, or damaged tissue). There are four plasma proteins directly involved in the alternative pathway: C3, factors B and D, and properdin. Proteolytic generation of C3b from native C3 is required for the alternative pathway to function. Since the alternative pathway C3 convertase (C3bBb) contains C3b as an essential subunit, the question regarding the origin of the first C3b via the alternative pathway has presented a puzzling problem and has stimulated considerable research.

C3 belongs to a family of proteins (along with C4 and α-2 macroglobulin) that contain a rare posttranslational modification known as a thioester bond. The thioester group is composed of a glutamine whose terminal carbonyl group is bound to the sulfhydryl group of a cysteine three amino acids away. This bond is unstable and the electrophilic carbonyl group of glutamine can form a covalent bond with other molecules via hydroxyl or amino groups. The thioester bond is reasonably stable when sequestered within a hydrophobic pocket of intact C3. However, proteolytic cleavage of C3 to C3a and C3b results in exposure of the highly reactive thioester bond on C3b and by this mechanism C3b covalently attaches to a target. In addition to its well-documented role in covalent attachment of C3b to complement targets, the C3 thioester is also thought to have a pivotal role in triggering the alternative pathway. According to the widely accepted "tick-over theory", the alternative pathway is initiated by the generation of a fluid-phase convertase, iC3Bb, which is formed from C3 with hydrolyzed thioester (iC3; C3(H₂O)) and factor B (Lachmann, P.J., et al., Springer Semin. Immunopathol. 7:143-162, 1984). The C3b-like iC3 is generated from native C3 by a slow spontaneous hydrolysis of the internal thioester in the protein (Pangburn, M.K., et al., J. Exp. Med. 154:856-867, 1981). Through the activity of the iC3Bb convertase, C3b molecules are deposited on the target surface thereby initiating the alternative pathway.

Very little is known about the initiators of activation of the alternative pathway. Activators are thought to include yeast cell walls (zymosan), many pure polysaccharides, rabbit erythrocytes, certain immunoglobulins, viruses, fungi, bacteria, animal tumor cells, parasites, and damaged cells. The only feature common to these activators is the presence of carbohydrate, but the complexity and variety of carbohydrate structures has made it difficult to establish the shared molecular determinants, which are recognized.

The alternative pathway can also provide a powerful amplification loop for the lectin/classical pathway C3 convertase (C4b2b) since any C3b generated can participate with factor B in forming additional alternative pathway C3 convertase (C3bBb). The alternative pathway C3 convertase is stabilized by the binding of properdin. Properdin extends the alternative pathway C3 convertase half-life six to ten fold. Addition of C3b to the C3 convertase leads to the formation of the alternative pathway C5 convertase.

All three pathways (i.e., the classical, lectin and alternative) have been thought to converge at C5, which is cleaved to form products with multiple proinflammatory effects. The converged pathway has been referred to as the terminal complement pathway. C5a is the most potent anaphylatoxin, inducing alterations in smooth muscle and vascular tone, as well as vascular permeability. It is also a powerful chemotaxin and activator of both neutrophils and monocytes. C5a-mediated cellular activation can significantly amplify inflammatory responses by inducing the release of multiple additional inflammatory mediators, including cytokines, hydrolytic enzymes, arachidonic acid metabolites and reactive oxygen species. C5 cleavage leads to the formation of C5b-9, also known as the membrane attack complex (MAC). There is now strong evidence that sublytic MAC deposition may play an important role in inflammation in addition to its role as a lytic pore-forming complex.

Hansen Troels et al, Diabetes Jun 2004, vol 53, no 6, pages 1570-1576 (2004) discloses the involvement of MBL in diseases.

### SUMMARY OF THE INVENTION

The invention provides a MASP-2 inhibitory anti-MASP-2 antibody or fragment thereof that specifically binds to a portion of SEQ ID NO:6 and selectively inhibits MASP-2-dependent complement activation without substantially inhibiting C1q-dependent complement activation for use in the treatment of a subject suffering from diabetic angiopathy.

Also described herein is the use of such an antibody for the treatment of diabetic neuropahy.

Described herein is a method of inhibiting the adverse effects of MASP-2-dependent complement activation in a living subject. The method includes the step of administering to a subject in need thereof, an amount of a MASP-2 inhibitory agent effective to inhibit MASP-2-dependent complement activation. In this context, the phrase "MASP-2-dependent complement activation" refers to alternative pathway complement activation that occurs via the lectin-dependent MASP-2 system. In another aspect of the invention, the MASP-2 inhibitory agent inhibits complement activation via the lectin-dependent MASP-2 system without substantially inhibiting complement activation via the classical or C1q-dependent system, such that the C1q-dependent system remains functional.

Described herein the MASP-2 inhibitory agent is an anti-MASP-2 antibody or fragment thereof. In further embodiments, the anti-MASP-2 antibody has reduced effector function. In some embodiments, the MASP-2 inhibitory agent is a MASP-2 inhibitory peptide or a non-peptide MASP-2 inhibitor.

Described herein are compositions for inhibiting the adverse effects of MASP-2-dependent complement activation, comprising a therapeutically effective amount of a MASP-2 inhibitory agent and a pharmaceutically acceptable carrier. Methods are also provided for manufacturing a medicament for use in inhibiting the adverse effects of MASP-2-dependent complement activation in living subjects in need thereof, comprising a therapeutically effective amount of a MASP-2 inhibitory agent in a pharmaceutical carrier. Methods are also provided for manufacturing medicaments for use in inhibiting MASP-2-dependent complement activation for treatment of each of the conditions, diseases and disorders described herein below.

The methods, compositions and medicaments of the invention are useful for inhibiting the adverse effects of MASP-2-dependent complement activation *in vivo* in mammalian subjects, including humans suffering from an acute or chronic pathological condition or injury as further described herein. Such conditions and injuries include without limitation MASP-2 mediated complement activation in associated autoimmune disorders and/or inflammatory conditions.

Described herein methods are provided for the treatment of ischemia reperfusion injuries by treating a subject experiencing ischemic reperfusion, including without limitation, after aortic aneurysm repair, cardiopulmonary bypass, vascular reanastomosis in connection with, for example, organ transplants (e.g., heart, lung, liver, kidney) and/or extremity/digit replantation, stroke, myocardial infarction, hemodynamic resuscitation following shock and/or surgical procedures, with a therapeutically effective amount of a MASP-2 inhibitory agent in a pharmaceutical carrier.

Described herein methods are provided for the inhibition of atherosclerosis by treating a subject suffering from or prone to atherosclerosis with a therapeutically effective amount of a MASP-2 inhibitory agent in a pharmaceutical carrier.

Described herein methods are provided for inhibiting MASP-2-dependent complement activation in a subject experiencing a vascular condition, including without limitation cardiovascular conditions, cerebrovascular conditions, peripheral (e.g., musculoskeletal) vascular conditions, renovascular conditions, mesenteric/enteric vascular, and revascularization to transplants and/or replants, by treating such patient with a therapeutically effective amount of a MASP-2 inhibitory agent. Such conditions include without limitation the treatment of: vasculitis, including Henoch-Schonlein purpura nephritis, systemic lupus erythematosus-associated vasculitis, vasculitis associated. with rheumatoid arthritis (also called malignant rheumatoid arthritis), immune complex vasculitis, and Takayasu's disease; dilated cardiomyopathy; diabetic angiopathy; Kawasaki's disease (arteritis); venous gas embolus (VGE); and/or restenosis following stent placement, rotational atherectomy and/or percutaneous transluminal coronary angioplasty (PTCA).

Described herein methods are provided for inhibiting MASP-2-dependent complement activation in a subject suffering from inflammatory gastrointestinal disorders, including but not limited to pancreatitis, diverticulitis and bowel disorders including Crohn's disease, ulcerative colitis, and irritable bowel syndrome.

Described herein methods are provided for inhibiting MASP-2-dependent complement activation in a subject suffering from a pulmonary condition including but not limited to acute respiratory distress syndrome, transfusion-related acute lung injury, ischemia/reperfusion acute lung injury, chronic obstructive pulmonary disease, asthma, Wegener's granulomatosis, antiglomerular basement membrane disease (Goodpasture's disease), meconium aspiration syndrome, bronchiolitis obliterans syndrome, idiopathic pulmonary fibrosis, acute lung injury secondary to burn, non-cardiogenic pulmonary edema, transfusion-related respiratory depression, and emphysema.

Described herein methods are provided for inhibiting MASP-2-dependent complement activation in a subject that has undergone, is undergoing or will undergo an extracorporeal reperfusion procedure, including but not limited to hemodialysis, plasmapheresis, leukopheresis, extracorporeal membrane oxygenation (ECMO), heparin-induced extracorporeal membrane oxygenation LDL precipitation (HELP) and cardiopulmonary bypass (CPB).

Described herein methods are provided for inhibiting MASP-2-dependent complement activation in a subject suffering from a musculoskeletal condition, including but not limited to osteoarthritis, rheumatoid arthritis, juvenile rheumatoid arthritis, gout, neuropathic arthropathy, psoriatic arthritis, ankylosing spondylitis or other spondyloarthropathies and crystalline arthropathies, or systemic lupus erythematosus (SLE).

Described herein methods are provided for inhibiting MASP-2-dependent complement activation in a subject suffering from renal conditions including but not limited to mesangioproliferative glomerulonephritis, membranous glomerulonephritis, membranoproliferative glomerulonephritis (mesangiocapillary glomerulonephritis), acute postinfectious glomerulonephritis (poststreptococcal glomerulonephritis), cryoglobulinemic glomerulonephritis, lupus nephritis, Henoch-Schonlein purpura nephritis or IgA nephropathy.

Described herein methods are provided for inhibiting MASP-2-dependent complement activation in a subject suffering from a skin condition, including but not limited to psoriasis, autoimmune bullous dermatoses, eosinophilic spongiosis, bullous pemphigoid, epidermolysis bullosa acquisita and herpes gestationis and other skin disorders, or from a thermal or chemical burn injury involving capillary leakage.

Described herein methods are provided for inhibiting MASP-2-dependent complement activation in a subject that has received an organ or other tissue transplant, including but not limited to allotransplantation or xenotransplantation of whole organs (e.g., kidney, heart, liver, pancreas, lung, cornea, etc.) or grafts (e.g., valves, tendons, bone marrow, etc.).

Described herein methods are provided for inhibiting MASP-2-dependent complement activation in a subject suffering from a central nervous system disorder or injury or a peripheral nervous system disorder or injury, including but not limited to multiple sclerosis (MS), myasthenia gravis (MG), Huntington's disease (HD), amyotrophic lateral sclerosis (ALS), Guillain Barre syndrome, reperfusion following stroke, degenerative discs, cerebral trauma, Parkinson's disease (PD), Alzheimer's disease (AD), Miller-Fisher syndrome, cerebral trauma and/or hemorrhage, demyelination and meningitis.

Described herein methods are provided for inhibiting MASP-2-dependent complement activation in a subject suffering from a blood disorder including but not limited to sepsis or a condition resulting from sepsis including without limitation severe sepsis, septic shock, acute respiratory distress syndrome resulting from sepsis, and systemic inflammatory response syndrome. Related methods are provided for the treatment of other blood disorders, including hemorrhagic shock, hemolytic anemia, autoimmune thrombotic thrombocytopenic purpura (TTP), hemolytic uremic syndrome (HUS) or other marrow/blood destructive conditions.

Described herein methods are provided for inhibiting MASP-2-dependent complement activation in a subject suffering from a urogenital condition including but not limited to painful bladder disease, sensory bladder disease, chronic abacterial cystitis and interstitial cystitis, male and female infertility, placental dysfunction and miscarriage and pre-eclampsia.

Described herein methods are provided for inhibiting MASP-2-dependent complement activation in a subject suffering from nonobese diabetes (Type-1 diabetes or Insulin dependent diabetes mellitus) or from angiopathy, neuropathy or retinopathy complications of Type-1 or Type-2 (adult onset) diabetes.

Described herein methods are provided for inhibiting MASP-2-dependent complement activation in a subject being treated with chemotherapeutics and/or radiation therapy, including without limitation for the treatment of cancerous conditions, by administering a MASP-2 inhibitor to such a patient perichemotherapeutically or periradiation therapy, i.e., before and/or during and/or after the administration of chemotherapeutic(s) and/or radiation therapy. Perichemotherapeutic or periradiation therapy administration of MASP-2 inhibitors may be useful for reducing the side-effects of chemotherapeutic or radiation therapy. In a still further aspect of the invention, methods are provided for the treatment of malignancies by administering a MASP-2 inhibitory agent in a pharmaceutically acceptable carrier to a patient suffering from a malignancy.

Described herein methods are provided for inhibiting MASP-2-dependent complement activation in a subject suffering from an endocrine disorder, by administering a therapeutically effective amount of a MASP-2 inhibitory agent in a pharmaceutical carrier to such a subject. Conditions subject to treatment in accordance with the present invention include, by way of nonlimiting example, Hashimoto's thyroiditis, stress, anxiety and other potential hormonal disorders involving regulated release of prolactin, growth or insulin-like growth factor, and adrenocorticotropin from the pituitary.

Described herein methods are provided for inhibiting MASP-2-dependent complement activation in a subject suffering from age-related macular degeneration or other complement mediated ophthalmologic condition by administering a therapeutically effective amount of a MASP-2 inhibitory agent in a pharmaceutical carrier to a subject suffering from such a condition.

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
The foregoing aspects and many of the attendant advantages of this invention will become more readily appreciated as the same become better understood by reference to the following detailed description, when taken in conjunction with the accompanying drawings, wherein:

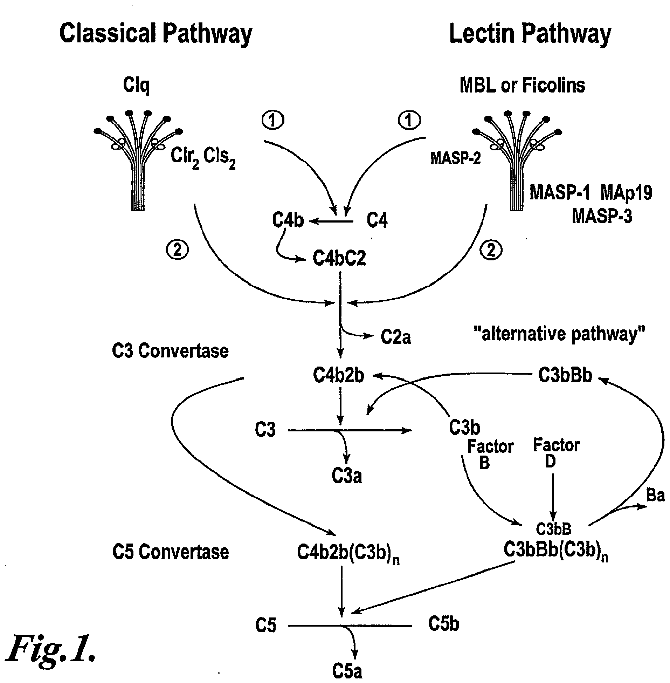

   FIGURE 1 is a flowchart illustrating the new discovery that the alternative complement pathway requires lectin pathway-dependent MASP-2 activation for complement activation;
   FIGURE 2 is a diagram illustrating the genomic structure of human MASP-2;
   FIGURE 3A is a schematic diagram illustrating the domain structure of human MASP-2 protein;
   FIGURE 3B is a schematic diagram illustrating the domain structure of human MAp19 protein;
   FIGURE 4 is a diagram illustrating the murine MASP-2 knockout strategy;
   FIGURE 5 is a diagram illustrating the human MASP-2 minigene construct;
   FIGURE 6A presents results demonstrating that MASP-2-deficiency leads to the loss of lectin-pathway-mediated C4 activation as measured by lack of C4b deposition on mannan;
   FIGURE 6B presents results demonstrating that MASP-2-deficiency leads to the loss of lectin-pathway-mediated C4 activation as measured by lack of C4b deposition on zymosan;
   FIGURE 6C presents results demonstrating the relative C4 activation levels of serum samples obtained from MASP-2+/-; MASP-2-/- and wild-type strains as measure by C4b deposition on mannan and on zymosan;
   FIGURE 7A presents results demonstrating that MASP-2-deficiency leads to the loss of both lectin-pathway-mediated and alternative pathway mediated C3 activation as measured by lack of C3b deposition on mannan;
   FIGURE 7B presents results demonstrating that MASP-2-deficiency leads to the loss of both lectin-pathway-mediated and alternative pathway mediated C3 activation as measured by lack of C3b deposition on zymosan;
   FIGURE 8 presents results demonstrating that the addition of murine recombinant MASP-2 to MASP-2-/- serum samples recovers lectin-pathway-mediated C4 activation in a protein concentration dependant manner, as measured by C4b deposition on mannan;
   FIGURE 9 presents demonstrating that the classical pathway is functional in the MASP-2-/- strain; and
   FIGURE 10 presents results demonstrating that the MASP-2-dependent complement activation system is activated in the ischemia/reperfusion phase following abdominal aortic aneurysm repair.

### DESCRIPTION OF THE SEQUENCE LISTING

SEQ ID NO:1 human MAp19 cDNA
SEQ ID NO:2 human MAp19 protein (with leader)
SEQ ID NO:3 human MAp19 protein (mature)
SEQ ID NO:4 human MASP-2 cDNA
SEQ ID NO:5 human MASP-2 protein (with leader)
SEQ ID NO:6 human MASP-2 protein (mature)
SEQ ID NO:7 human MASP-2 gDNA (exons 1-6)

### ANTIGENS: (IN REFERENCE TO THE MASP-2 MATURE PROTEIN)

SEQ ID NO:8 CUBI sequence (aa 1-121)
SEQ ID NO:9 CUBEGF sequence (aa 1-166)
SEQ ID NO:10 CUBEGFCUBII (aa 1-293)
SEQ ID NO:11 EGF region (aa 122-166)
SEQ ID NO:12 serine protease domain (aa 429 - 671)
SEQ ID N0:13 serine protease domain inactive (aa 610-625 with Ser618 to Ala mutation)
SEQ ID NO:14 TPLGPKWPEPVFGRL (CUB1 peptide)
SEQ ID NO: 15
   TAPPGYRLRLYFTHFDLELSHLCEYDFVKLSSGAKVLATLC GQ (CUBI peptide)
SEQ ID NO:16 TFRSDYSN (MBL binding region core)
SEQ ID NO: 17 FYSLGSSLDITFRSDYSNEKPFTGF (MBL binding region)
SEQ ID NO:18 IDECQVAPG (EGF PEPTIDE)
SEQ ID NO:19 ANMLCAGLESGGKDSCRGDSGGALV (serine protease binding core)

### PEPTIDE INHIBITORS:

SEQ ID NO:20 MBL full length cDNA
SEQ ID NO:21 MBL full length protein
SEQ ID NO:22 OGK-X-GP (consensus binding)
SEQ ID NO:23 OGKLG
SEQ ID NO:24 GLR GLQ GPO GKL GPO G
SEQ ID NO:25 GPO GPO GLR GLQ GPO GKL GPO GPO GPO
SEQ ID NO:26 GKDGRDGTKGEKGEPGQGLRGLQGPOGKLGPOG
SEQ ID NO:27 GAOGSOGEKGAOGPQGPOGPOGKMGPKGEOGDO (human h-ficolin)
SEQ ID NO:28 GCOGLOGAOGDKGEAGTNGKRGERGPOGPOGKAGPOGPN GAOGEO (human ficolin p35)
SEQ ID NO:29 LQRALEILPNRVTIKANRPFLVFI (C4 cleavage site)

### EXPRESSION INHIBITORS:

SEQ ID NO:30 cDNA of CUBI-EGF domain (nucleotides 22-680 of SEQ ID NO:4)
SEQ ID NO:31
   5' CGGGCACACCATGAGGCTGCTGACCCTCCTGGGC 3'
   Nucleotides 12-45 of SEQ ID NO:4 including the MASP-2 translation start site (sense)
SEQ ID NO:32
   5'GACATTACCTTCCGCTCCGACTCCAACGAGAAG3' Nucleotides 361-396 of SEQ ID NO:4 encoding a region comprising the MASP-2 MBL binding site (sense)
SEQ ID NO:33
   5'AGCAGCCCTGAATACCCACGGCCGTATCCCAAA3' Nucleotides 610-642 of SEQ ID NO:4 encoding a region comprising the CUBII domain

### CLONING PRIMERS:

SEQ ID NO:34 CGGGATCCATGAGGCTGCTGACCCTC (5' PCR for CUB)
SEQ ID NO:35 GGAATTCCTAGGCTGCATA (3' PCR FOR CUB)
SEQ ID NO:36 GGAATTCCTACAGGGCGCT (3' PCR FOR CUBIEGF)
SEQ ID NO:37 GGAATTCCTAGTAGTGGAT (3' PCR FOR CUBIEGFCUBII)
SEQ ID NOS:38-47 are cloning primers for humanized antibody
SEQ ID NO:48 is 9 aa peptide bond

### EXPRESSION VECTOR:

SEQ ID NO:49 is the MASP-2 minigene insert
SEQ ID NO: 50 is the murine MASP-2 cDNA
SEQ ID NO: 51 is the murine MASP-2 protein (w/leader)
SEQ ID NO: 52 is the mature murine MASP-2 protein
SEQ ID NO: 53 the rat MASP-2 cDNA
SEQ ID NO: 54 is the rat MASP-2 protein (w/ leader)
SEQ ID NO: 55 is the mature rat MASP-2 protein
SEQ ID NO: 56-59 are the oligonucleotides for site-directed mutagenesis of human MASP-2 used to generate human MASP-2A
SEQ ID NO: 60-63 are the oligonucleotides for site-directed mutagenesis of murine MASP-2 used to generate murine MASP-2A
SEQ ID NO: 64-65 are the oligonucleotides for site-directed mutagenesis of rat MASP-2 used to generate rat MASP-2A

### DETAILED DESCRIPTION

The present invention is based upon the surprising discovery by the present inventors that MASP-2 is needed to initiate alternative complement pathway activation. Through the use of a knockout mouse model of MASP-2-/-, the present inventors have shown that it is possible to inhibit alternative complement pathway activation via the lectin mediated MASP-2 pathway while leaving the classical pathway intact, thus establishing the lectin-dependent MASP-2 activation as a requirement for alternative complement activation in absence of the classical pathway. Also described is the use of MASP-2 as a therapeutic target for inhibiting cellular injury associated with lectin-mediated alternative complement pathway activation while leaving the classical (C1q-dependent) pathway component of the immune system intact.

### I. DEFINITIONS

Unless specifically defined herein, all terms used herein have the same meaning as would be understood by those of ordinary skill in the art of the present invention. The following definitions are provided in order to provide clarity with respect to the terms as they are used in the specification and claims to describe the present invention.

As used herein, the term "MASP-2-dependent complement activation" refers to alternative pathway complement activation that occurs via lectin-dependent MASP-2 activation.

As used herein, the term "alternative pathway" refers to complement activation that is triggered, for example, by zymosan from fungal and yeast cell walls, lipopolysaccharide (LPS) from Gram negative outer membranes, and rabbit erythrocytes, as well as from many pure polysaccharides, rabbit erythrocytes, viruses, bacteria, animal tumor cells, parasites and damaged cells, and which has traditionally been thought to arise from spontaneous proteolytic generation of C3b from complement factor C3.

As used herein, the term "lectin pathway" refers to complement activation that occurs via the specific binding of serum and non-serum carbohydrate-binding proteins including mannan-binding lectin (MBL) and the ficolins.

As used herein, the term "classical pathway" refers to complement activation that is triggered by antibody bound to a foreign particle and requires binding of the recognition molecule C1q.

As used herein, the term "MASP-2 inhibitory agent" refers to any agent that binds to or interacts with MASP-2 and effectively inhibits MASP-2-dependent complement activation, including anti-MASP-2 antibodies and MASP-2 binding fragments thereof, natural and synthetic peptides, small molecules, soluble MASP-2 receptors, expression inhibitors and isolated natural inhibitors. MASP-2 inhibitory agents useful in the method of the invention may reduce MASP-2-dependent complement activation by greater than 20%, such as greater than 50%, such as greater than 90%. In one embodiment, the MASP-2 inhibitory agent reduces MASP-2-dependent complement activation by greater than 90% (i.e., resulting in MASP-2 complement activation of only 10% or less).

As used herein, the term "antibody" encompasses antibodies and antibody fragments thereof, derived from any antibody-producing mammal (e.g., mouse, rat, rabbit, and primate including human), that specifically bind to MASP-2 polypeptides or portions thereof. Exemplary antibodies include polyclonal, monoclonal and recombinant antibodies; multispecific antibodies (e.g., bispecific antibodies); humanized antibodies; murine antibodies; chimeric, mouse-human, mouse-primate, primate-human monoclonal antibodies; and anti-idiotype antibodies, and may be any intact molecule or fragment thereof.

As used herein, the term "antibody fragment" refers to a portion derived from or related to a full-length anti-MASP-2 antibody, generally including the antigen binding or variable region thereof. Illustrative examples of antibody fragments include Fab, Fab', F(ab)₂, F(ab')₂ and Fv fragments, scFv fragments, diabodies, linear antibodies, single-chain antibody molecules and multispecific antibodies formed from antibody fragments.

As used herein, a "single-chain Fv" or "scFv" antibody fragment comprises the V_{H} and V_{L} domains of an antibody, wherein these domains are present in a single polypeptide chain. Generally, the Fv polypeptide further comprises a polypeptide linker between the V_{H} and V_{L} domains, which enables the scFv to form the desired structure for antigen binding.

As used herein, a "chimeric antibody" is a recombinant protein that contains the variable domains and complementarity-determining regions derived from a non-human species (e.g., rodent) antibody, while the remainder of the antibody molecule is derived from a human antibody.

As used herein, a "humanized antibody" is a chimeric antibody that comprises a minimal sequence that conforms to specific complementarity-determining regions derived from non-human immunoglobulin that is transplanted into a human antibody framework. Humanized antibodies are typically recombinant proteins in which only the antibody complementarity-determining regions are of non-human origin.

As used herein, the term "mannan-binding lectin" ("MBL") is equivalent to mannan-binding protein ("MBP").

As used herein, the "membrane attack complex" ("MAC") refers to a complex of the terminal five complement components (C5-C9) that inserts into and disrupts membranes. Also referred to as C5b-9.

As used herein, "a subject" includes all mammals, including without limitation humans, non-human primates, dogs, cats, horses, sheep, goats, cows, rabbits, pigs and rodents.

As used herein, the amino acid residues are abbreviated as follows: alanine (Ala;A), asparagine (Asn;N), aspartic acid (Asp;D), arginine (Arg;R), cysteine (Cys;C), glutamic acid (Glu;E), glutamine (Gln;Q), glycine (Gly;G), histidine (His;H), isoleucine (Ile;I), leucine (Leu;L), lysine (Lys;K), methionine (Met;M), phenylalanine (Phe;F), proline (Pro;P), serine (Ser;S), threonine (Thr;T), tryptophan (Trp;W), tyrosine (Tyr;Y), and valine (Val;V).

In the broadest sense, the naturally occurring amino acids can be divided into groups based upon the chemical characteristic of the side chain of the respective amino acids. By "hydrophobic" amino acid is meant either Ile, Leu, Met, Phe, Trp, Tyr, Val, Ala, Cys or Pro. By "hydrophilic" amino acid is meant either Gly, Asn, Gln, Ser, Thr, Asp, Glu, Lys, Arg or His. This grouping of amino acids can be further subclassed as follows. By "uncharged hydrophilic" amino acid is meant either Ser, Thr, Asn or Gln. By "acidic" amino acid is meant either Glu or Asp. By "basic" amino acid is meant either Lys, Arg or His.

As used herein the term "conservative amino acid substitution" is illustrated by a substitution among amino acids within each of the following groups: (1) glycine, alanine, valine, leucine, and isoleucine, (2) phenylalanine, tyrosine, and tryptophan, (3) serine and threonine, (4) aspartate and glutamate, (5) glutamine and asparagine, and (6) lysine, arginine and histidine.

The term "oligonucleotide" as used herein refers to an oligomer or polymer of ribonucleic acid (RNA) or deoxyribonucleic acid (DNA) or mimetics thereof. This term also covers those oligonucleobases composed of naturally-occurring nucleotides, sugars and covalent internucleoside (backbone) linkages as well as oligonucleotides having non-naturally-occurring modifications.

### II. THE ALTERNATIVE PATHWAY: A NEW UNDERSTANDING

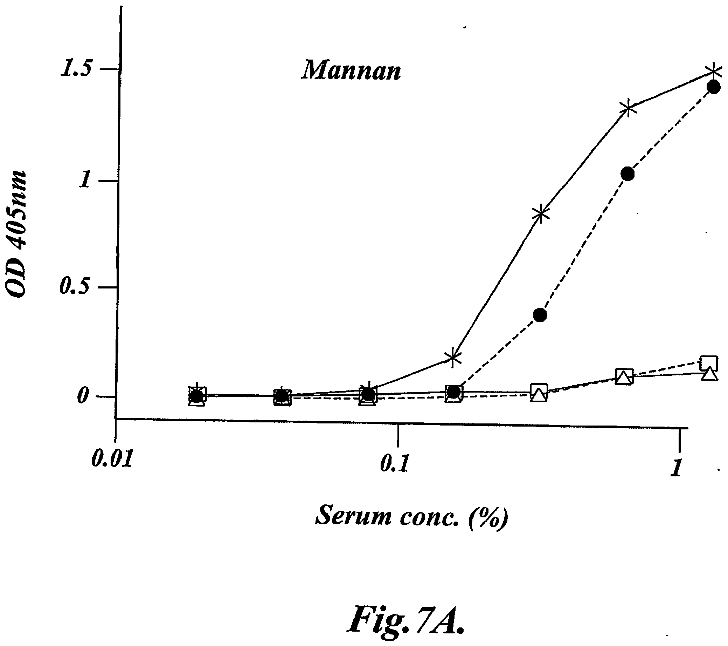

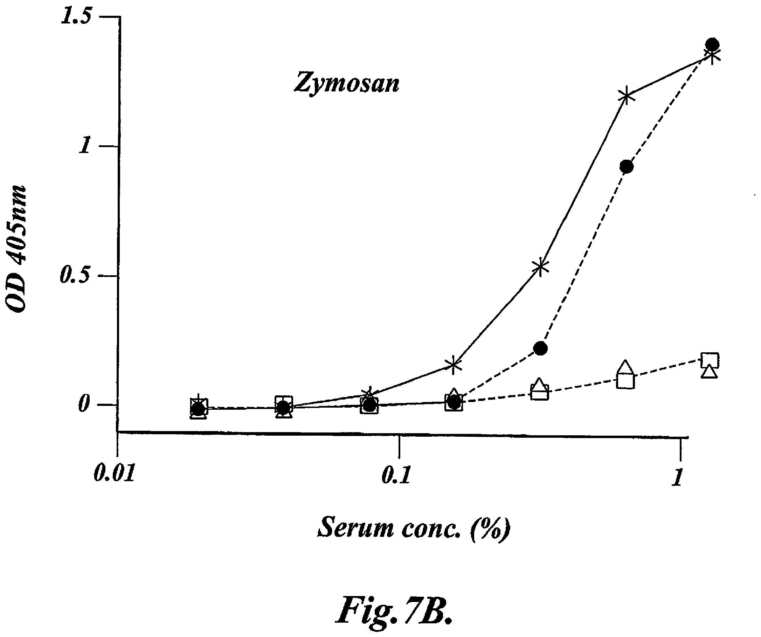

The alternative pathway of complement was first described by Louis Pillemer and his colleagues in early 1950s based on studies in which zymosan made from yeast cell walls was used to activate complement (Pillemer, L. et al., J. Exp. Med. 103:1-13, 1956; Lepow, I.H., J. Immunol. 125:471-478, 1980). Ever since then, zymosan is considered as the canonical example of a specific activator of the alternative pathway in human and rodent serum (Lachmann, P.J., et al., Springer Semin. Immunopathol. 7:143-162, 1984; Van Dijk, H., et al., J. Immunol. Methods 85:233-243, 1985; Pangburn, M.K., Methods in Enzymol. 162:639-653, 1988). A convenient and widely used assay for alternative pathway activation is to incubate serum with zymosan coated onto plastic wells and to determine the amount of C3b deposition onto the solid phase following the incubation. As expected, there is substantial C3b deposition onto zymosan-coated wells following incubation with normal mouse serum (Figure 7B). However, incubation of serum from homozygous MASP-2-deficient mice with zymosan-coated wells results in a substantial reduction in C3b deposition compared to that of normal serum. Furthermore, use of serum from mice heterozygous for deficiency in the *MASP 2* gene in this assay results in levels of C3b deposition that are intermediate between those obtained with serum from homozygous MASP-2-deficient mice and normal mouse serum. Parallel results are also obtained using wells coated with mannan, another polysaccharide known to activate the alternative pathway (Figure 7A). Since the normal and MASP-2 deficient mice share the same genetic background, except for the *MASP 2* gene, these unexpected results demonstrate that MASP-2 plays an essential role in activation of the alternative pathway.

These results provide strong evidence that the alternative pathway is not an independent, stand-alone pathway of complement activation as described in essentially all current medical textbooks and recent review articles on complement. The current and widely held scientific view is that the alternative pathway is activated on the surface of certain particulate targets (microbes, zymosan, rabbit erythrocytes) through the amplification of spontaneous "tick-over" C3 activation. However, the absence of significant alternative pathway activation in serum from MASP-2 knockout mice by two well-known "activators" of the alternative pathway makes it unlikely that the "tick-over theory" describes an important physiological mechanism for complement activation.

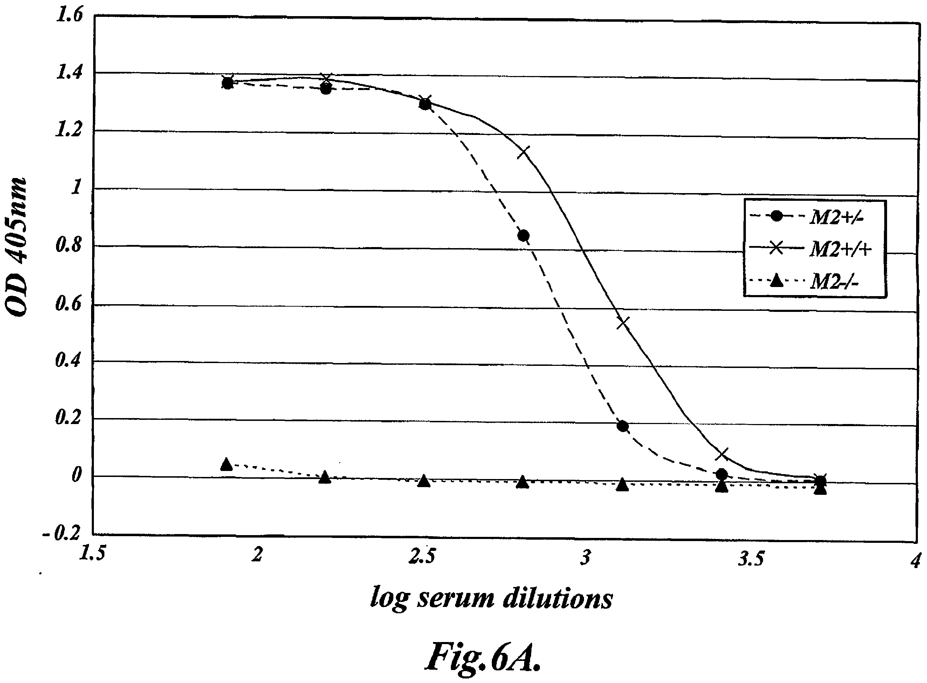

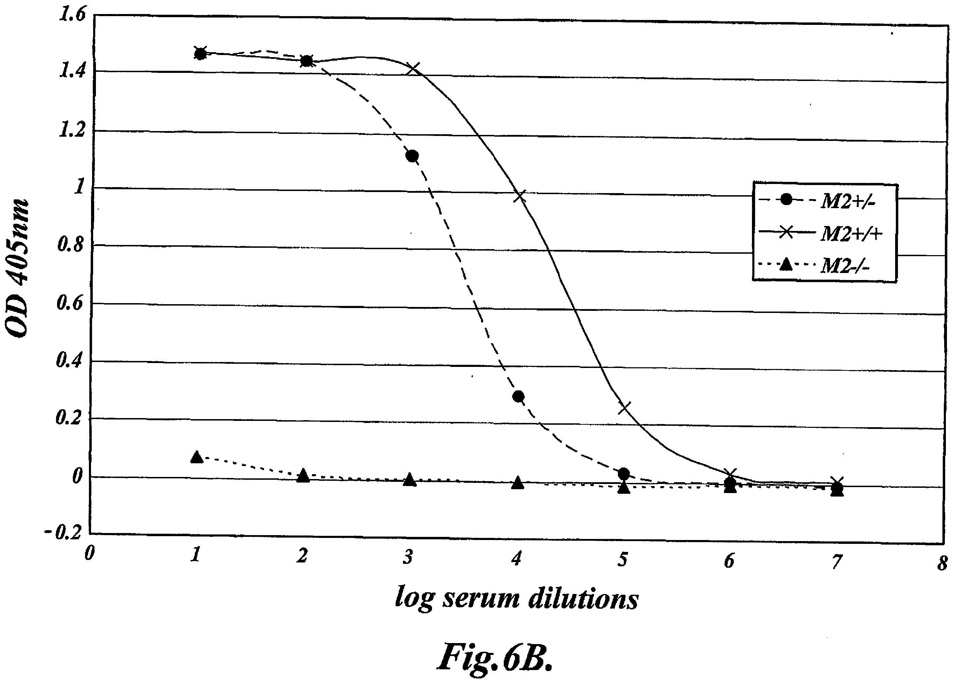

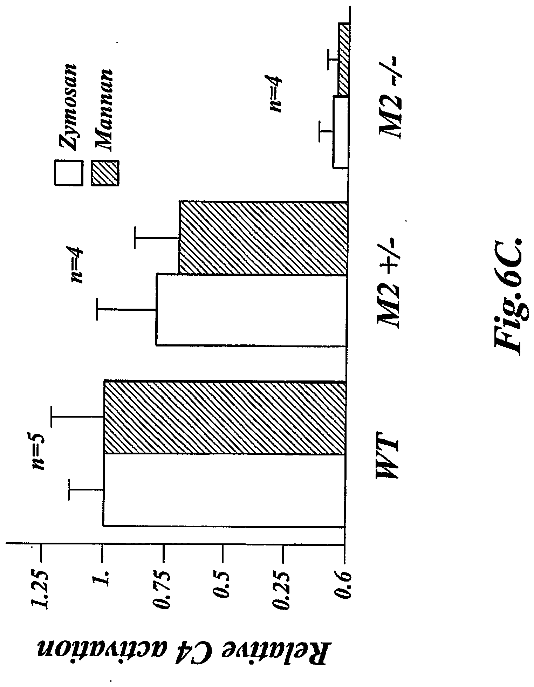

Since MASP-2 protease is known to have a specific and well-defined role as the enzyme responsible for the initiation of the lectin complement cascade, these results implicate activation of the lectin pathway by zymosan and mannan as a critical first step for subsequent activation of the alternative pathway. C4b is an activation product generated by the lectin pathway but not by the alternative pathway. Consistent with this concept, incubation of normal mouse serum with zymosan- or mannan-coated wells results in C4b deposition onto the wells and this C4b deposition is substantially reduced when the coated wells are incubated with serum from MASP-2-deficient mice (Figures 6A, 6B and 6C).

The alternative pathway, in addition to its widely accepted role as an independent pathway for complement activation, can also provide an amplification loop for complement activation initially triggered via the classical and lectin pathways (Liszewski, M.K. and J.P. Atkinson, 1993, in Fundamental Immunology, Third Edition, edited by W.E. Paul, Raven Press, Ltd., New York; Schweinie, J.E., et al., J. Clin. Invest. 84:1821-1829, 1989). In this alternative pathway-mediated amplification mechanism, C3 convertase (C4b2b) generated by activation of either the classical or lectin complement cascades cleaves C3 into C3a and C3b, and thereby provides C3b that can participate in forming C3bBb, the alternative pathway C3 convertase. The likely explanation for the absence of alternative pathway activation in MASP-2 knockout serum is that the lectin pathway is required for initial complement activation by zymosan, mannan, and other putative "activators" of the alternative pathway, while the alternative pathway plays a crucial role for amplifying complement activation. In other words, the alternative pathway is a feedforward amplification loop dependent upon the lectin and classical complement pathways for activation, rather than an independent linear cascade.

Rather than the complement cascade being activated through three distinct pathways (classical, alternative and lectin pathways) as previously envisioned, our results indicate that it is more accurate to view complement as being composed of two major systems, which correspond, to a first approximation, to the innate (lectin) and acquired (classical) wings of the complement immune defense system. Lectins (MBP, M-ficolin, H-ficolin, and L-ficolin) are the specific recognition molecules that trigger the innate complement system and the system includes the lectin pathway and the associated alternative pathway amplification loop. C1q is the specific recognition molecule that triggers the acquired complement system and the system includes the classical pathway and associated alternative pathway amplification loop. We refer to these two major complement activation systems as the lectin-dependent complement system and the C1q-dependent complement system, respectively.

In addition to its essential role in immune defense, the complement system contributes to tissue damage in many clinical conditions. Thus, there is a pressing need to develop therapeutically effective complement inhibitors to prevent these adverse effects. With recognition that complement is composed of two major complement activation systems comes the realization that it would be highly desirable to specifically inhibit only the complement activation system causing a particular pathology without completely shutting down the immune defense capabilities of complement. For example, in disease states in which complement activation is mediated predominantly by the lectin-dependent complement system, it would be advantageous to specifically inhibit only this system. This would leave the C1q-dependent complement activation system intact to handle immune complex processing and to aid in host defense against infection.

The preferred protein component to target in the development of therapeutic agents to specifically inhibit the lectin-dependent complement system is MASP-2. Of all the protein components of the lectin-dependent complement system (MBL, H-ficolin, M-ficolin, L-ficolin, MASP-2, C2-C9, Factor B, Factor D, and properdin), only MASP-2 is both unique to the lectin-dependent complement system and required for the system to function. The lectins (MBL, H-ficolin, M-ficolin and L-ficolin) are also unique components in the lectin-dependent complement system. However, loss of any one of the lectin components would not necessarily inhibit activation of the system due to lectin redundancy. It would be necessary to inhibit all four lectins in order to guarantee inhibition of the lectin-dependent complement activation system. Furthermore, since MBL and the ficolins are also known to have opsonic activity independent of complement, inhibition of lectin function would result in the loss of this beneficial host defense mechanism against infection. In contrast, this complement-independent lectin opsonic activity would remain intact if MASP-2 was the inhibitory target. An added benefit of MASP-2 as the therapeutic target to inhibit the lectin-dependent complement activation system is that the plasma concentration of MASP-2 is among the lowest of any complement protein (≈ 500 ng/ml); therefore, correspondingly low concentrations of high-affinity inhibitors of MASP-2 may be required to obtain full inhibition (Moller-Kristensen, M., et al., J. Immunol Methods 282:159-167, 2003).

### III. ROLE OF MASP-2 IN VARIOUS DISEASES AND CONDITIONS AND THERAPEUTIC METHODS USING MASP-2 INHIBITORY AGENTS VASCULAR DISEASES AND CONDITIONS

The endothelium is largely exposed to the immune system and is particularly vulnerable to complement proteins that are present in plasma. Complement-mediated vascular injury has been shown to contribute to the pathophysiology of several diseases of the cardiovascular system, including atherosclerosis (Seifert, P.S., et al., Atherosclerosis 73:91-104, 1988), ischemia-reperfusion injury (Weisman, H.F., Science 249:146-51, 1990) and myocardial infarction (Tada, T., et al., Virchows Arch 430:327-332, 1997). Evidence suggests that complement activation may extend to other vascular conditions.

For example, there is evidence that complement activation contributes to the pathogenesis of many forms of vasculitis, including: Henoch-Schonlein purpura nephritis, systemic lupus erythematosus-associated vasculitis, vasculitis associated with rheumatoid arthritis (also called malignant rheumatoid arthritis), immune complex vasculitis, and Takayasu's disease. Henoch-Schonlein purpura nephritis is a form of systemic vasculitis of the small vessels with immune pathogenesis, in which activation of complement through the lectin pathway leading to C5b-9-induced endothelial damage is recognized as an important mechanism (Kawana, S., et al., Arch. Dermatol. Res. 282:183-7, 1990; Endo, M., et al., Am J. Kidney Dis. 35:401-7, 2000). Systemic lupus erythematosus (SLE) is an example of systemic autoimmune diseases that affects multiple organs, including skin, kidneys, joints, serosal surfaces, and central nervous system, and is frequently associated with severe vasculitis. IgG anti-endothelial antibodies and IgG complexes capable of binding to endothelial cells are present in the sera of patients with active SLE, and deposits of IgG immune complexes and complement are found in blood vessel walls of patients with SLE vasculitis (Cines, D.B., et al., J. Clin. Invest. 73:611-25, 1984). Rheumatoid arthritis associated with vasculitis, also called malignant rheumatoid arthritis (Tomooka, K., Fukuoka Igaku Zasshi 80:456-66, 1989), immune-complex vasculitis, vasculitis associated with hepatitis A, leukocytoclastic vasculitis, and the arteritis known as Takayasu's disease, form another pleomorphic group of human diseases in which complement-dependent cytotoxicity against endothelial and other cell types plays a documented role (Tripathy, N.K., et al., J. Rheumatol. 28:805-8, 2001).

Evidence also suggests that complement activation plays a role in dilated cardiomyopathy. Dilated cardiomyopathy is a syndrome characterized by cardiac enlargement and impaired systolic function of the heart. Recent data suggests thatongoing inflammation in the myocardium may contribute to the development of disease. C5b-9, the terminal membrane attack complex of complement, is known to significantly correlate with immunoglobulin deposition and myocardial expression of TNF-alpha. In myocardial biopsies from 28 patients with dilated cardiomyopathy, myocardial accumulation of C5b-9 was demonstrated, suggesting that chronic immunoglobulin-mediated complement activation in the myocardium may contribute in part to the progression of dilated cardiomyopathy (Zwaka, T.P., et al., Am. J. Pathol. 161(2):449-57, 2002).

There is described the treatment of a vascular condition, including cardiovascular conditions, cerebrovascular conditions, peripheral (e.g., musculoskeletal) vascular conditions, renovascular conditions, and mesenteric/enteric vascular conditions, by administering a composition comprising a therapeutically effective amount of a MASP-2 inhibitory agent in a pharmaceutical carrier. Conditions for which the invention is believed to be suited include, without limitation: vasculitis, including Henoch-Schonlein purpura nephritis, systemic lupus erythematosus-associated vasculitis, vasculitis associated with rheumatoid arthritis (also called malignant rheumatoid arthritis), immune complex vasculitis, and Takayasu's disease; dilated cardiomyopathy; diabetic angiopathy; Kawasaki's disease (arteritis); and venous gas embolus (VGE). Also, given that complement activation occurs as a result of luminal trauma and the foreign-body inflammatory response associated with cardiovascular interventional procedures, it is believed that the MASP-2 inhibitory compositions of the present invention may also be used in the inhibition of restenosis following stent placement, rotational atherectomy and/or percutaneous transluminal coronary angioplasty (PTCA), either alone or in combination with other restenosis inhibitory agents such as are disclosed in U.S. Patent No. 6,492,332 to Demopulos.

The MASP-2 inhibitory agent may be administered to the subject by intra-arterial, intravenous, intramuscular, intrathecal, intracranial, subcutaneous or other parenteral administration, and potentially orally for non-peptidergic inhibitors. Administration may be repeated periodically as determined by a physician for optimal therapeutic effect. For the inhibition of restenosis, the MASP-2 inhibitory composition may be administered before and/or during and/or after the placement of a stent or the atherectomy or angioplasty procedure. Alternately, the MASP-2 inhibitory composition may be coated on or incorporated into the stent.

### DIABETES AND DIABETIC CONDITIONS

Diabetic retinal microangiopathy is characterized by increased permeability, leukostasis, microthrombosis, and apoptosis of capillary cells, all of which could be caused or promoted by activation of complement. Glomerular structures and endoneurial microvessels of patients with diabetes show signs of complement activation. Decreased availability or effectiveness of complement inhibitors in diabetes has been suggested by the findings that high glucose *in vitro* selectively decreases on the endothelial cell surface the expression of CD55 and CD59, the two inhibitors that are glycosylphosphatidylinositol (GPI)-anchored membrane proteins, and that CD59 undergoes nonenzymatic glycation that hinders its complement-inhibitory function.

Studies by Zhang et al. (Diabetes 51:3499-3504, 2002), investigated complement activation as a feature of human nonproliferative diabetic retinopathy and its association with changes in inhibitory molecules. It was found that deposition of C5b-9, the terminal product of complement activation, occurs in the wall of retinal vessels of human eye donors with type-2 diabetes, but not in the vessels of age-matched nondiabetic donors. C1q and C4, the complement components unique to the classical pathway, were not detected in the diabetic retinas, which indicates that C5b-9 was generated via the alternative pathway. The diabetic donors showed a prominent reduction in the retinal levels of CD55 and CD59, the two complement inhibitors linked to the plasma membrane by GPI anchors. Similar complement activation in retinal vessels and selective reduction in the levels of retinal CD55 and CD59 were observed in rats with a 10 week duration of streptozotocin-induced diabetes. Thus, diabetes appears to cause defective regulation of complement inhibitors and complement activation that precede most other manifestations of diabetic retinal microangiopathy.

Gerl et al. (Investigative Ophthalmology and Visual Science 43:1104-08, 2000) determined the presence of activated complement components in eyes affected by diabetic retinopathy. Immunohistochemical studies found extensive deposits of complement C5b-9 complexes that were detected in the choriocapillaris immediately underlying the Bruch membrane and densely surrounding the capillaries in all 50 diabetic retinopathy specimens. Staining for C3d positively correlated with C5b-9 staining, indicative of the fact that complement activation had occurred *in situ.* Furthermore, positive staining was found for vitronectin, which forms stable complexes with extracellular C5b-9. In contrast, there was no positive staining for C-reactive protein (CRP), mannan-binding lectin (MBL), C1q, or C4, indicating that complement activation did not occur through a C4-dependent pathway. Thus, the presence of C3d, C5b-9, and vitronectin indicates that complement activation occurs to completion, possibly through the alternative pathway in the choriocapillaris in eyes affected by diabetic retinopathy. Complement activation may be a causative factor in the pathologic sequelae that can contribute to ocular tissue disease and visual impairment. Therefore, the use of a complement inhibitor may be an effective therapy to reduce or block damage to microvessels that occurs in diabetes.

Insulin dependent diabetes mellitus (IDDM, also referred to as Type-I diabetes) is an autoimmune disease associated with the presence of different types of autoantibodies (Nicoloff et al., Clin. Dev. Immunol. 11:61-66, 2004). The presence of these antibodies and the corresponding antigens in the circulation leads to the formation of circulating immune complexes (CIC), which are known to persist in the blood for long periods of time. Deposition of CIC in the small blood vessels has the potential to lead to microangiopathy with debilitating clinical consequences. A correlation exists between CIC and the development of microvascular complications in diabetic children. These findings suggest that elevated levels of CIC IgG are associated with the development of early diabetic nephropathy and that an inhibitor of the complement pathway may be effective at blocking diabetic nephropathy (Kotnik, et al., Croat. Med. J. 44:707-11, 2003). In addition, the formation of downstream complement proteins and the involvement of the alternative pathway is likely to be a contributory factor in overall islet cell function in IDDM, and the use of a complement inhibitor to reduce potential damage or limit cell death is expected (Caraher, et al., J. Endocrinol. 162:143-53, 1999).

Described herein are methods provided for inhibiting MASP-2-dependent complement activation in a subject suffering from nonobese diabetes (IDDM) or from angiopathy, neuropathy or retinopathy complications of IDDM or adult onset (Type-2) diabetes, by administering a composition comprising a therapeutically effective amount of a MASP-2 inhibitor in a pharmaceutical carrier. The MASP-2 inhibitory agent may be administered to the subject systemically, such as by intra-arterial, intravenous, intramuscular, subcutaneous or other parenteral administration, or potentially by oral administration for non-peptidergic agents. Alternatively, administration may be by local delivery to the site of angiopathic, neuropathic or retinopathic symptoms. The MASP-2 inhibitory agent may be administered periodically over an extended period of time for treatment or control of a chronic condition, or by a single or series of administrations for treatment of an acute condition.

### IV. MASP-2 INHIBITORY AGENTS

Described herein are methods of inhibiting the adverse effects of MASP-2-dependent complement activation. MASP-2 inhibitory agents are administered in an amount effective to inhibit MASP-2-dependent complement activation in a living subject. In practice, representative MASP-2 inhibitory agents include: molecules that inhibit the biological activity of MASP-2 (such as small molecule inhibitors, anti-MASP-2 antibodies or blocking peptides which interact with MASP-2 or interfere with a protein-protein interaction), and molecules that decrease the expression of MASP-2 (such as MASP-2 antisense nucleic acid molecules, MASP-2 specific RNAi molecules and MASP-2 ribozymes), thereby preventing MASP-2 from activating the alternative complement pathways. The MASP-2 inhibitory agents can be used alone as a primary therapy or in combination with other therapeutics as an adjuvant therapy to enhance the therapeutic benefits of other medical treatments.

The inhibition of MASP-2-dependent complement activation is characterized by at least one of the following changes in a component of the complement system that occurs as a result of administration of a MASP-2 inhibitory agent in accordance with the methods of the invention: the inhibition of the generation or production of MASP-2-dependent complement activation system products C4b, C3a, C5a and/or C5b-9 (MAC) (measured, for example, as described in Example 2), the reduction of alternative complement activation assessed in a hemolytic assay using unsensitized rabbit or guinea pig red blood cells , the reduction of C4 cleavage and C4b deposition (measured, for example as described in Example 2), or the reduction of C3 cleavage and C3b deposition (measured, for example, as described in Example 2).

Accordingly, MASP-2 inhibitory agents are utilized that are effective in inhibiting the MASP-2-dependent complement activation system. MASP-2 inhibitory agents useful in the practice of this aspect of the method include, for example, anti-MASP-2 antibodies and fragments thereof, MASP-2 inhibitory peptides, small molecules, MASP-2 soluble receptors and expression inhibitors. MASP-2 inhibitory agents may inhibit the MASP-2-dependent complement activation system by blocking the biological function of MASP-2. For example, an inhibitory agent may effectively block MASP-2 protein-to-protein interactions, interfere with MASP-2 dimerization or assembly, block Ca²⁺ binding, interfere with the MASP-2 serine protease active site, or may reduce MASP-2 protein expression.

The MASP-2 inhibitory agents selectively inhibit MASP-2 complement activation, leaving the C1q-dependent complement activation system functionally intact.

In one embodiment, a MASP-2 inhibitory agent useful in the methods of the invention is a specific MASP-2 inhibitory agent that specifically binds to a polypeptide comprising SEQ ID NO:6 with an affinity of at least 10 times greater than to other antigens in the complement system. In another embodiment, a MASP-2 inhibitory agent specifically binds to a polypeptide comprising SEQ ID NO:6 with a binding affinity of at least 100 times greater than to other antigens in the complement system. The binding affinity of the MASP-2 inhibitory agent can be determined using a suitable binding assay.

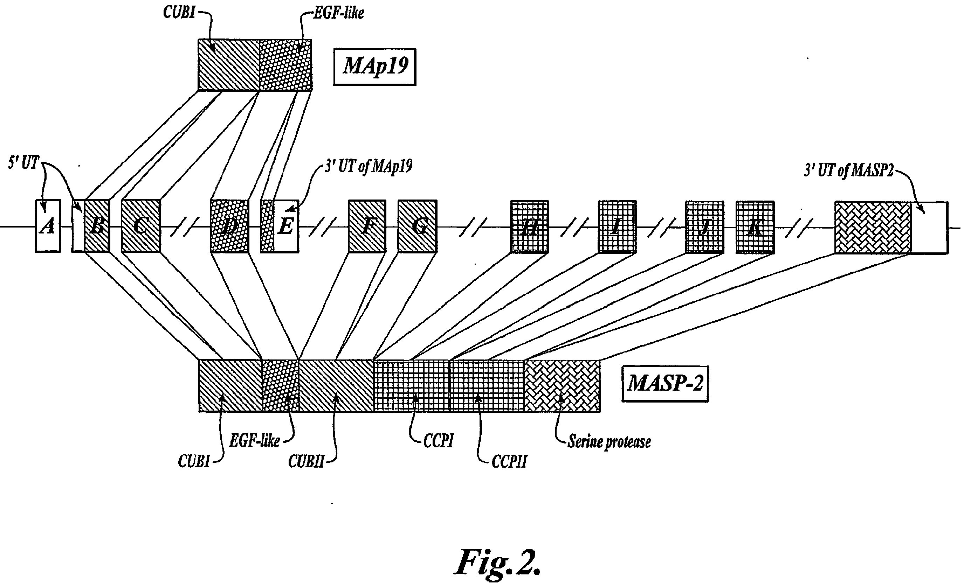

The MASP-2 polypeptide exhibits a molecular structure similar to MASP-1, MASP-3, and C1r and C1s, the proteases of the C1 complement system. The cDNA molecule set forth in SEQ ID NO:4 encodes a representative example of MASP-2 (consisting of the amino acid sequence set forth in SEQ ID NO:5) and provides the human MASP-2 polypeptide with a leader sequence (aa 1-15) that is cleaved after secretion, resulting in the mature form of human MASP-2 (SEQ ID NO:6). As shown in FIGURE 2, the human *MASP 2* gene encompasses twelve exons. The human MASP-2 cDNA is encoded by exons B, C, D, F, G, H, I, J, K AND L. An alternative splice results in a 20 kDa protein termed MBL-associated protein 19 ("MAp19") (SEQ ID NO:2), encoded by (SEQ ID NO:1) arising from exons B, C, D and E as shown in FIGURE 2. The cDNA molecule set forth in SEQ ID NO:50 encodes the murine MASP-2 (consisting of the amino acid sequence set forth in SEQ ID NO:51) and provides the murine MASP-2 polypeptide with a leader sequence that is cleaved after secretion, resulting in the mature form of murine MASP-2 (SEQ ID NO:52). The cDNA molecule set forth in SEQ ID NO:53 encodes the rat MASP-2 (consisting of the amino acid sequence set forth in SEQ ID NO:54) and provides the rat MASP-2 polypeptide with a leader sequence that is cleaved after secretion, resulting in the mature form of rat MASP-2 (SEQ ID NO:55).

Those skilled in the art will recognize that the sequences disclosed in SEQ ID NO:4, SEQ ID NO:50 and SEQ ID NO:53 represent single alleles of human, murine and rat MASP-2 respectively, and that allelic variation and alternative splicing are expected to occur. Allelic variants of the nucleotide sequences shown in SEQ ID NO:4, SEQ ID NO:50 and SEQ ID NO:53, including those containing silent mutations and those in which mutations result in amino acid sequence changes, are within the scope of the present invention. Allelic variants of the MASP-2 sequence can be cloned by probing cDNA or genomic libraries from different individuals according to standard procedures.

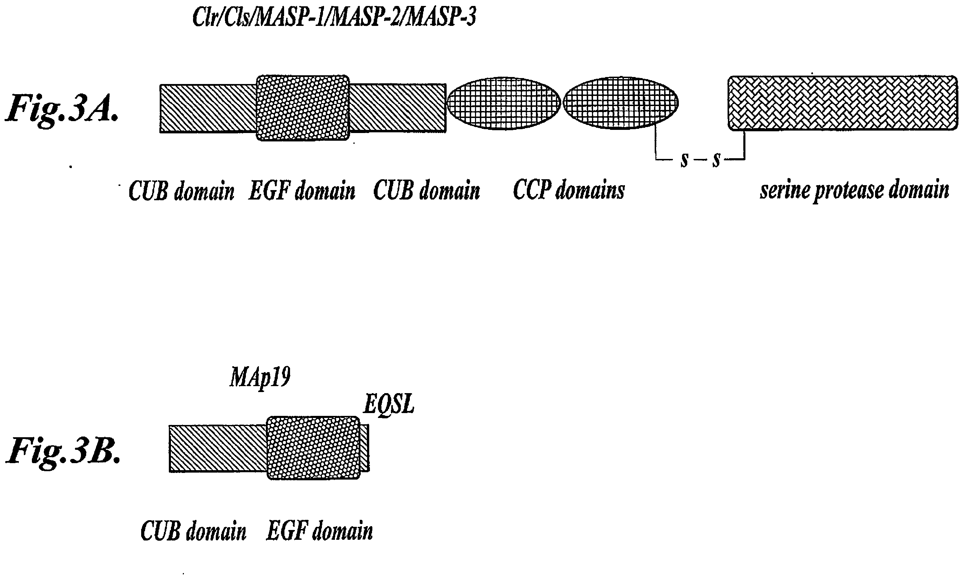

The domains of the human MASP-2 protein (SEQ ID NO:6) are shown in FIGURE 3A and include an N-terminal C1r/C1s/sea urchin Vegf/bone morphogenic protein (CUBI) domain (aa 1-121 of SEQ ID NO:6), an epidermal growth factor-like domain (aa 122-166), a second CUBI domain (aa 167-293), as well as a tandem of complement control protein domains and a serine protease domain. Alternative splicing of the *MASP 2* gene results in MAp19 shown in FIGURE 3B. MAp19 is a nonenzymatic protein containing the N-terminal CUB1-EGF region of MASP-2 with four additional residues (EQSL) derived from exon E as shown in FIGURE 2.

Several proteins have been shown to bind to, or interact with MASP-2 through protein-to-protein interactions. For example, MASP-2 is known to bind to, and form Ca²⁺ dependent complexes with, the lectin proteins MBL, H-ficolin and L-ficolin. Each MASP-2/lectin complex has been shown to activate complement through the MASP-2-dependent cleavage of proteins C4 and C2 (Ikeda, K., et al., J. Biol. Chem. 262:7451-7454, 1987; Matsushita, M., et al., J. Exp. Med. 176:1497-2284, 2000; Matsushita, M., et al., J. Immunol. 168:3502-3506, 2002). Studies have shown that the CUB1-EGF domains of MASP-2 are essential for the association of MASP-2 with MBL (Thielens, N.M., et al., J. Immunol. 166:5068, 2001). It has also been shown that the CUB1EGFCUBII domains mediate dimerization of MASP-2, which is required for formation of an active MBL complex (Wallis, R., et al., J. Biol. Chem. 275:30962-30969, 2000). Therefore, MASP-2 inhibitory agents can be identified that bind to or interfere with MASP-2 target regions known to be important for MASP-2-dependent complement activation.

### ANTI-MASP-2 ANTIBODIES

In some embodiments of this aspect of the invention, the MASP-2 inhibitory agent comprises an anti-MASP-2 antibody that inhibits the MASP-2-dependent complement activation system. The anti-MASP-2 antibodies useful in this aspect of the invention include polyclonal, monoclonal or recombinant antibodies derived from any antibody producing mammal and may be multispecific, chimeric, humanized, anti-idiotype, and antibody fragments. Antibody fragments include Fab, Fab', F(ab)₂, F(ab')₂, Fv fragments, scFv fragments and single-chain antibodies as further described herein.

Several anti-MASP-2 antibodies have been described in the literature, some of which are listed below in TABLE 1. These previously described anti-MASP-2 antibodies can be screened for the ability to inhibit the MASP-2-dependent complement activation system using the assays described herein. Once an anti-MASP-2 antibody is identified that functions as a MASP-2 inhibitory agent, it can be used to produce anti-idiotype antibodies and used to identify other MASP-2 binding molecules as further described below.

**TABLE 1: MASP-2 SPECIFIC ANTIBODIES FROM THE LITERATURE**

| **Antigen** | **Antibody Type** | **Reference** |
|---|---|---|
| Recombinant MASP-2 | Rat Polyclonal | Peterson, S.V., et al., Mol. Immunol. 37:803-811,2000 |
| Recombinant human CCP1/2-SP fragment (MoAb 8B5) | Rat MoAb (subclass IgG1) | Moller-Kristensen, M., et al., J. of Immunol. Methods 282:159-167, 2003 |
| Recombinant human MAp19 (MoAb 6G12) (cross reacts with MASP-2) | Rat MoAb (subclass IgG1) | Moller-Kristensen, M., et al., J. of Immunol. Methods 282:159-167, 2003 |
| MASP-2 | Mouse MoAb | Peterson, S.V., et al., Mol. Immunol. 35:409 |

### ANTI-MASP-2 ANTIBODIES WITH REDUCED EFFECTOR FUNCTION

In some embodiments of this aspect of the invention, the anti-MASP-2 antibodies have reduced effector function in order to reduce inflammation that may arise from the activation of the classical complement pathway. The ability of IgG molecules to trigger the classical complement pathway has been shown to reside within the Fc portion of the molecule (Duncan, A.R., et al., Nature 332:738-740 1988). IgG molecules in which the Fc portion of the molecule has been removed by enzymatic cleavage are devoid of this effector function (see Harlow, Antibodies: A Laboratory Manual, Cold Spring Harbor Laboratory, New York, 1988). Accordingly, antibodies with reduced effector function can be generated as the result of lacking the Fc portion of the molecule by having a genetically engineered Fc sequence that minimizes effector function, or being of either the human IgG₂ or IgG₄ isotype.

Antibodies with reduced effector function can be produced by standard molecular biological manipulation of the Fc portion of the IgG heavy chains as described in Example 9 herein and also described in Jolliffe, et al., Int'l Rev. Immunol. 10:241-250, 11993, and Rodrigues, et al., J. Immunol. 151:6954-6961, 1998. Antibodies with reduced effector function also include human IgG2 and IgG4 isotypes that have a reduced ability to activate complement and/or interact with Fc receptors (Ravetch, J.V., et al., Annu. Rev. Immunol. 9:457-492, 1991; Isaacs, J.D., et al., J. Immunol. 148:3062-3071, 1992; van de Winkel, J.G., et al., Immunol. Today 14:215-221, 1993). Humanized or fully human antibodies specific to human MASP-2 comprised of IgG2 or IgG4 isotypes can be produced by one of several methods known to one of ordinary skilled in the art, as described in Vaughan, T.J., et al., Nature Biotechnical 16:535-539, 1998.

### PRODUCTION OF ANTI-MASP-2 ANTIBODIES

Anti-MASP-2 antibodies can be produced using MASP-2 polypeptides (e.g., full length MASP-2) or using antigenic MASP-2 epitope-bearing peptides (e.g., a portion of the MASP-2 polypeptide). Immunogenic peptides may be as small as five amino acid residues. For example, the MASP-2 polypeptide including the entire amino acid sequence of SEQ ID NO:6 may be used to induce anti-MASP-2 antibodies useful in the method of the invention. Particular MASP-2 domains known to be involved in protein-protein interactions, such as the CUBI, and CUBIEGF domains, as well as the region encompassing the serine-protease active site, may be expressed as recombinant polypeptides as described in Example 5 and used as antigens. In addition, peptides comprising a portion of at least 6 amino acids of the MASP-2 polypeptide (SEQ ID NO:6) are also useful to induce MASP-2 antibodies. Additional examples of MASP-2 derived antigens useful to induce MASP-2 antibodies are provided below in TABLE 2. The MASP-2 peptides and polypeptides used to raise antibodies may be isolated as natural polypeptides, or recombinant or synthetic peptides and catalytically inactive recombinant polypeptides, such as MASP-2A, as further described in Examples 5-7. In some embodiments of this aspect of the invention, anti-MASP-2 antibodies are obtained using a transgenic mouse strain as described in Examples 8 and 9 and further described below.

Antigens useful for producing anti-MASP-2 antibodies also include fusion polypeptides, such as fusions of MASP-2 or a portion thereof with an immunoglobulin polypeptide or with maltose-binding protein. The polypeptide immunogen may be a full-length molecule or a portion thereof. If the polypeptide portion is hapten-like, such portion may be advantageously joined or linked to a macromolecular carrier (such as keyhole limpet hemocyanin (KLH), bovine serum albumin (BSA) or tetanus toxoid) for immunization.

**TABLE 2: MASP-2 DERIVED ANTIGENS**

| **SEQ ID NO:** | **Amino Acid Sequence** |
|---|---|
| SEQ ID NO:6 | Human MASP-2 protein |
| SEQ ID NO:51 | Murine MASP-2 protein |
| SEQ ID NO:8 | CUBI domain of human MASP-2 (aa 1-121 of SEQ ID NO:6) |
| SEQ ID NO:9 | CUBIEGF domains of human MASP-2 (aa 1-166 of SEQ ID NO:6) |
| SEQ ID NO: 10 | CUBIEGFCUBII domains of human MASP-2 (aa 1-293 of SEQ ID NO:6) |
| SEQ ID NO:11 | EGF domain of human MASP-2 (aa 122-166 of SEQ ID NO:6) |
| SEQ ID NO:12 | Serine-Protease domain of human MASP-2 (aa 429-671 of SEQ ID NO:6) |
| SEQ ID NO:13 | Serine-Protease inactivated mutant form (aa 610-625 of SEQ ID NO:6 with mutated Ser 618) |
| GKDSCRGDAGGALVFL | |
| SEQ ID NO: 14 | Human CUBI peptide |
| TPLGPKWPEPVFGRL | |
| SEQ ID NO:15: | Human CUBI peptide |
| | |
| SEQ ID NO: 16: | MBL binding region in human CUBI domain |
| TFRSDYSN | |
| SEQ ID NO: 17: | MBL binding region in human CUBI domain |
| | |
| SEQ ID NO: 18 | EGF peptide |
| IDECQVAPG | |
| SEQ ID NO: 19 | Peptide from serine-protease active site |
| | |

### POLYCLONAL ANTIBODIES

Polyclonal antibodies against MASP-2 can be prepared by immunizing an animal with MASP-2 polypeptide or an immunogenic portion thereof using methods well known to those of ordinary skill in the art. See, for example, Green, et al., "Production of Polyclonal Antisera," in Immunochemical Protocols (Manson, ed.), page 105, and as further described in Example 6. The immunogenicity of a MASP-2 polypeptide can be increased through the use of an adjuvant, including mineral gels, such as aluminum hydroxide or Freund's adjuvant (complete or incomplete), surface active substances such as lysolecithin, pluronic polyols, polyanions, oil emulsions, keyhole limpet hemocyanin and dinitrophenol. Polyclonal antibodies are typically raised in animals such as horses, cows, dogs, chicken, rats, mice, rabbits, guinea pigs, goats, or sheep. Alternatively, an anti-MASP-2 antibody useful in the present invention may also be derived from a subhuman primate. General techniques for raising diagnostically and therapeutically useful antibodies in baboons may be found, for example, in Goldenberg et al., International Patent Publication No. WO 91/11465, and in Losman, M.J., et al., Int. J. Cancer 46:310, 1990. Sera containing immunologically active antibodies are then produced from the blood of such immunized animals using standard procedures well known in the art.

### MONOCLONAL ANTIBODIES

In some embodiments, the MASP-2 inhibitory agent is an anti-MASP-2 monoclonal antibody. Anti-MASP-2 monoclonal antibodies are highly specific, being directed against a single MASP-2 epitope. As used herein, the modifier "monoclonal" indicates the character of the antibody as being obtained from a substantially homogenous population of antibodies, and is not to be construed as requiring production of the antibody by any particular method. Monoclonal antibodies can be obtained using any technique that provides for the production of antibody molecules by continuous cell lines in culture, such as the hybridoma method described by Kohler, G., et al., Nature 256:495, 1975, or they may be made by recombinant DNA methods (see, e.g., U.S. Patent No.4,816,567 to Cabilly). Monoclonal antibodies may also be isolated from phage antibody libraries using the techniques described in Clackson, T., et al., Nature 352:624-628, 1991, and Marks, J.D., et al., J. Mol. Biol. 222:581-597, 1991. Such antibodies can be of any immunoglobulin class including IgG, IgM, IgE, IgA, IgD and any subclass thereof.

For example, monoclonal antibodies can be obtained by injecting a suitable mammal (e.g., a BALB/c mouse) with a composition comprising a MASP-2 polypeptide or portion thereof. After a predetermined period of time, splenocytes are removed from the mouse and suspended in a cell culture medium. The splenocytes are then fused with an immortal cell line to form a hybridoma. The formed hybridomas are grown in cell culture and screened for their ability to produce a monoclonal antibody against MASP-2. An example further describing the production of anti-MASP-2 monoclonal antibodies is provided in Example 7. (See also Current Protocols in Immunology, Vol. 1., John Wiley & Sons, pages 2.5.1-2.6.7, 1991.)

Human monoclonal antibodies may be obtained through the use of transgenic mice that have been engineered to produce specific human antibodies in response to antigenic challenge. In this technique, elements of the human immunoglobulin heavy and light chain locus are introduced into strains of mice derived from embryonic stem cell lines that contain targeted disruptions of the endogenous immunoglobulin heavy chain and light chain loci. The transgenic mice can synthesize human antibodies specific for human antigens, such as the MASP-2 antigens described herein, and the mice can be used to produce human MASP-2 antibody-secreting hybridomas by fusing B-cells from such animals to suitable myeloma cell lines using conventional Kohler-Milstein technology as further described in Example 7. Transgenic mice with a human immunoglobulin genome are commercially available (e.g., from Abgenix, Inc., Fremont, CA, and Medarex, Inc., Annandale, N.J.). Methods for obtaining human antibodies from transgenic mice are described, for example, by Green, L.L., et al., Nature Genet. 7:13, 1994; Lonberg, N., et al., Nature 368:856, 1994; and Taylor, L.D., et al., Int. Immun. 6:579, 1994.

Monoclonal antibodies can be isolated and purified from hybridoma cultures by a variety of well-established techniques. Such isolation techniques include affinity chromatography with Protein-A Sepharose, size-exclusion chromatography, and ionexchange chromatography (see, for example, Coligan at pages 2.7.1-2.7.12 and pages 2.9.1-2.9.3; Baines et al., "Purification of Immunoglobulin G (IgG)," in Methods in Molecular Biology, The Humana Press, Inc., Vol. 10, pages 79-104, 1992).

Once produced, polyclonal, monoclonal or phage-derived antibodies are first tested for specific MASP-2 binding. A variety of assays known to those skilled in the art may be utilized to detect antibodies which specifically bind to MASP-2. Exemplary assays include Western blot or immunoprecipitation analysis by standard methods (e.g., as described in Ausubel et al.), immunoelectrophoresis, enzyme-linked immuno-sorbent assays, dot blots, inhibition or competition assays and sandwich assays (as described in Harlow and Land, Antibodies: A Laboratory Manual, Cold Spring Harbor Laboratory Press, 1988). Once antibodies are identified that specifically bind to MASP-2, the anti-MASP-2 antibodies are tested for the ability to function as a MASP-2 inhibitory agent in one of several assays such as, for example, a lectin-specific C4 cleavage assay (described in Example 2), a C3b deposition assay (described in Example 2) or a C4b deposition assay (described in Example 2).

The affinity of anti-MASP-2 monoclonal antibodies can be readily determined by one of ordinary skill in the art (see, e.g., Scatchard, A., NY Acad. Sci. 51:660-672, 1949). In one embodiment, the anti-MASP-2 monoclonal antibodies useful for the methods of the invention bind to MASP-2 with a binding affinity of <100 nM, preferably <10 nM and most preferably <2 nM.

### CHIMERIC/HUMANIZED ANTIBODIES

Monoclonal antibodies useful in the method of the invention include chimeric antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies (U.S. Patent No. 4,816,567 to Cabilly, and Morrison, S.L., et al., Proc. Nat'l Acad. Sci. USA 81:6851-6855, 1984).

One form of a chimeric antibody useful in the invention is a humanized monoclonal anti-MASP-2 antibody. Humanized forms of non-human (e.g., murine) antibodies are chimeric antibodies, which contain minimal sequence derived from non-human immunoglobulin. Humanized monoclonal antibodies are produced by transferring the non-human (e.g., mouse) complementarity determining regions (CDR), from the heavy and light variable chains of the mouse immunoglobulin into a human variable domain. Typically, residues of human antibodies are then substituted in the framework regions of the non-human counterparts. Furthermore, humanized antibodies may comprise residues that are not found in the recipient antibody or in the donor antibody. These modifications are made to further refine antibody performance. In general, the humanized antibody will comprise substantially all of at least one, and typically two variable domains, in which all or substantially all of the hypervariable loops correspond to those of a non-human immunoglobulin and all or substantially all of the Fv framework regions are those of a human immunoglobulin sequence. The humanized antibody optionally also will comprise at least a portion of an immunoglobulin constant region (Fc), typically that of a human immunoglobulin. For further details, see Jones, P.T., et al., Nature 321:522-525, 1986; Reichmann, L., et al., Nature 332:323-329, 1988; and Presta, Curr. Op. Struct. Biol. 2:593-596, 1992.

The humanized antibodies useful in the invention include human monoclonal antibodies including at least a MASP-2 binding CDR3 region. In addition, the Fc portions may be replaced so as to produce IgA or IgM as well as human IgG antibodies. Such humanized antibodies will have particular clinical utility because they will specifically recognize human MASP-2 but will not evoke an immune response in humans against the antibody itself. Consequently, they are better suited for *in vivo* administration in humans, especially when repeated or long-term administration is necessary.

An example of the generation of a humanized anti-MASP-2 antibody from a murine anti-MASP-2 monoclonal antibody is provided herein in Example 10. Techniques for producing humanized monoclonal antibodies are also described, for example, by Jones, P.T., et al., Nature 321:522, 1986; Carter, P., et al., Proc. Nat'l. Acad. Sci. USA 89:4285, 1992; Sandhu, J.S., Crit. Rev. Biotech. 12:437, 1992; Singer, I.I., et al., J. Immun. 150:2844, 1993; Sudhir (ed.), Antibody Engineering Protocols, Humana Press, Inc., 1995; Kelley, "Engineering Therapeutic Antibodies," in Protein Engineering: Principles and Practice, Cleland et al. (eds.), John Wiley & Sons, Inc., pages 399-434, 1996; and by U.S. Patent No. 5,693,762 to Queen, 1997. In addition, there are commercial entities that will synthesize humanized antibodies from specific murine antibody regions, such as Protein Design Labs (Mountain View, CA).

### RECOMBINANT ANTIBODIES

Anti-MASP-2 antibodies can also be made using recombinant methods. For example, human antibodies can be made using human immunoglobulin expression libraries (available for example, from Stratagene, Corp., La Jolla, CA) to produce fragments of human antibodies (V_{H}, V_{L}, Fv, Fd, Fab or F(ab')₂). These fragments are then used to construct whole human antibodies using techniques similar to those for producing chimeric antibodies.

### ANTI-IDIOTYPE ANTIBODIES

Once anti-MASP-2 antibodies are identified with the desired inhibitory activity, these_antibodies can be used to generate anti-idiotype antibodies that resemble a portion of MASP-2 using techniques that are well known in the art. See, e.g., Greenspan, N.S., et al., FASEB J. 7:437, 1993. For example, antibodies that bind to MASP-2 and competitively inhibit a MASP-2 protein interaction required for complement activation can be used to generate anti-idiotypes that resemble the MBL binding site on MASP-2 protein and therefore bind and neutralize a binding ligand of MASP-2 such as, for example, MBL.

### IMMUNOGLOBULIN FRAGMENTS

The MASP-2 inhibitory agents useful in the method of the invention encompass not only intact immunoglobulin molecules but also the well known fragments including Fab, Fab', F(ab)₂, F(ab')₂ and Fv fragments, scFv fragments, diabodies, linear antibodies, single-chain antibody molecules and multispecific antibodies formed from antibody fragments.

It is well known in the art that only a small portion of an antibody molecule, the paratope, is involved in the binding of the antibody to its epitope (see, e.g., Clark, W.R., The Experimental Foundations of Modern Immunology, Wiley & Sons, Inc., NY, 1986). The pFc' and Fc regions of the antibody are effectors of the classical complement pathway, but are not involved in antigen binding. An antibody from which the pFc' region has been enzymatically cleaved, or which has been produced without the pFc' region, is designated an F(ab')₂ fragment and retains both of the antigen binding sites of an intact antibody. An isolated F(ab')₂ fragment is referred to as a bivalent monoclonal fragment because of its two antigen binding sites. Similarly, an antibody from which the Fc region has been enzymatically cleaved, or which has been produced without the Fc region, is designated a Fab fragment, and retains one of the antigen binding sites of an intact antibody molecule.

Antibody fragments can be obtained by proteolytic hydrolysis, such as by pepsin or papain digestion of whole antibodies by conventional methods. For example, antibody fragments can be produced by enzymatic cleavage of antibodies with pepsin to provide a 5S fragment denoted F(ab')₂. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using pepsin produces two monovalent Fab fragments and an Fc fragment directly. These methods are described, for example, U.S. Patent No. 4,331,647 to Goldenberg; Nisonoff, A., et al., Arch. Biochem. Biophys. 89:230, 1960; Porter, R.R., Biochem. J. 73:119, 1959; Edelman, et al., in Methods in Enzymology, 1:422, Academic Press, 1967; and by Coligan at pages 2.8.1-2.8.10 and 2.10.-2.10.4.

In some embodiments, the use of antibody fragments lacking the Fc region are preferred to avoid activation of the classical complement pathway which is initiated upon binding Fc to the Fcγ receptor. There are several methods by which one can produce a MoAb that avoids Fcγ receptor interactions. For example, the Fc region of a monoclonal antibody can be removed chemically using partial digestion by proteolytic enzymes (such as ficin digestion), thereby generating, for example, antigen-binding antibody fragments such as Fab or F(ab)₂ fragments (Mariani, M., et al., Mol. Immunol. 28:69-71, 1991). Alternatively, the human γ4 IgG isotype, which does not bind Fcγ receptors, can be used during construction of a humanized antibody as described herein. Antibodies, single chain antibodies and antigen-binding domains that lack the Fc domain can also be engineered using recombinant techniques described herein.

### SINGLE-CHAIN ANTIBODY FRAGMENTS

Alternatively, one can create single peptide chain binding molecules specific for MASP-2 in which the heavy and light chain Fv regions are connected. The Fv fragments may be connected by a peptide linker to form a single-chain antigen binding protein (scFv). These single-chain antigen binding proteins are prepared by constructing a structural gene comprising DNA sequences encoding the V_{H} and V_{L} domains which are connected by an oligonucleotide. The structural gene is inserted into an expression vector, which is subsequently introduced into a host cell, such as *E. coli.* The recombinant host cells synthesize a single polypeptide chain with a linker peptide bridging the two V domains. Methods for producing scFvs are described for example, by Whitlow, et al., "Methods: A Companion to Methods in Enzymology" 2:97, 1991; Bird, et al., Science 242:423, 1988; U.S. Patent No. 4,946,778 to Ladner; Pack, P., et al., Bio/Technology 11:1271, 1993.

As an illustrative example, a MASP-2 specific scFv can be obtained by exposing lymphocytes to MASP-2 polypeptide *in vitro* and selecting antibody display libraries in phage or similar vectors (for example, through the use of immobilized or labeled MASP-2 protein or peptide). Genes encoding polypeptides having potential MASP-2 polypeptide binding domains can be obtained by screening random peptide libraries displayed on phage or on bacteria such as *E. coli.* These random peptide display libraries can be used to screen for peptides which interact with MASP-2. Techniques for creating and screening such random peptide display libraries are well known in the art (U.S. Patent No. 5,223,409 to Lardner; U.S. Patent No.4,946,778 to Ladner; U.S. Patent No. 5,403,484 to Lardner; U.S. Patent No. 5,571,698 to Lardner; and Kay et al., Phage Display of Peptides and Proteins Academic Press, Inc., 1996) and random peptide display libraries and kits for screening such libraries are available commercially, for instance from CLONTECH Laboratories, Inc. (Palo Alto, Calif.), Invitrogen Inc. (San Diego, Calif.), New England Biolabs, Inc. (Beverly, Mass.), and Pharmacia LKB Biotechnology Inc. (Piscataway, N.J.).

Another form of an anti-MASP-2 antibody fragment useful in this aspect of the invention is a peptide coding for a single complementarity-determining region (CDR) that binds to an epitope on a MASP-2 antigen and inhibits MASP-2-dependent complement activation. CDR peptides ("minimal recognition units") can be obtained by constructing genes encoding the CDR of an antibody of interest. Such genes are prepared, for example, by using the polymerase chain reaction to synthesize the variable region from RNA of antibody-producing cells (see, for example, Larrick et al., Methods: A Companion to Methods in Enzymology 2:106, 1991; Courtenay-Luck, "Genetic Manipulation of Monoclonal Antibodies," in Monoclonal Antibodies: Production, Engineering and Clinical Application, Ritter et al. (eds.), page 166, Cambridge University Press, 1995; and Ward et al., "Genetic Manipulation and Expression of Antibodies," in Monoclonal Antibodies: Principles and Applications, Birch et al. (eds.), page 137, Wiley-Liss, Inc., 1995).

The MASP-2 antibodies described herein are administered to a subject in need thereof to inhibit MASP-2-dependent complement activation. In some embodiments, the MASP-2 inhibitory agent is a high-affinity human or humanized monoclonal anti-MASP-2 antibody with reduced effector function.

### PHARMACEUTICAL CARRIERS AND DELIVERY VEHICLES

In general, the MASP-2 inhibitory agent compositions of the present invention, combined with any other selected therapeutic agents, are suitably contained in a pharmaceutically acceptable carrier. The carrier is non-toxic, biocompatible and is selected so as not to detrimentally affect the biological activity of the MASP-2 inhibitory agent (and any other therapeutic agents combined therewith). Exemplary pharmaceutically acceptable carriers for peptides are described in U.S. Patent No. 5,211,657 to Yamada. The anti-MASP-2 antibodies and inhibitory peptides useful in the invention may be formulated into preparations in solid, semi-solid, gel, liquid or gaseous forms such as tablets, capsules, powders, granules, ointments, solutions, depositories, inhalants and injections allowing for oral, parenteral or surgical administration. The invention also contemplates local administration of the compositions by coating medical devices and the like.

Suitable carriers for parenteral delivery via injectable, infusion or irrigation and topical delivery include distilled water, physiological phosphate-buffered saline, normal or lactated Ringer's solutions, dextrose solution, Hank's solution, or propanediol. In addition, sterile, fixed oils may be employed as a solvent or suspending medium. For this purpose any biocompatible oil may be employed including synthetic mono- or diglycerides. In addition, fatty acids such as oleic acid find use in the preparation of injectables. The carrier and agent may be compounded as a liquid, suspension, polymerizable or non-polymerizable gel, paste or salve.

The carrier may also comprise a delivery vehicle to sustain (i.e., extend, delay or regulate) the delivery of the agent(s) or to enhance the delivery, uptake, stability or pharmacokinetics of the therapeutic agent(s). Such a delivery vehicle may include, by way of non-limiting example, microparticles, microspheres, nanospheres or nanoparticles composed of proteins, liposomes, carbohydrates, synthetic organic compounds, inorganic compounds, polymeric or copolymeric hydrogels and polymeric micelles. Suitable hydrogel and micelle delivery systems include the PEO:PHB:PEO copolymers and copolymer/cyclodextrin complexes disclosed in WO 2004/009664 A2 and the PEO and PEO/cyclodextrin complexes disclosed in US 2002/0019369 A1. Such hydrogels may be injected locally at the site of intended action, or subcutaneously or intramuscularly to form a sustained release depot.

For intra-articular delivery, the MASP-2 inhibitory agent may be carried in above-described liquid or gel carriers that are injectable, above-described sustained-release delivery vehicles that are injectable, or a hyaluronic acid or hyaluronic acid derivative.

For oral administration of non-peptidergic agents, the MASP-2 inhibitory agent may be carried in an inert filler or diluent such as sucrose, cornstarch, or cellulose.

For topical administration, the MASP-2 inhibitory agent may be carried in ointment, lotion, cream, gel, drop, suppository, spray, liquid or powder, or in gel or microcapsular delivery systems via a transdermal patch.

Various nasal and pulmonary delivery systems, including aerosols, metered-dose inhalers, dry powder inhalers, and nebulizers, are being developed and may suitably be adapted for delivery of the present invention in an aerosol, inhalant, or nebulized delivery vehicle, respectively.

### PHARMACEUTICAL CARRIERS FOR ANTIBODIES AND PEPTIDES

More specifically with respect to anti-MASP-2 antibodies and inhibitory peptides, exemplary formulations can be parenterally administered as injectable dosages of a solution or suspension of the compound in a physiologically acceptable diluent with a pharmaceutical carrier that can be a sterile liquid such as water, oils, saline, glycerol or ethanol. Additionally, auxiliary substances such as wetting or emulsifying agents, surfactants, pH buffering substances and the like can be present in compositions comprising anti-MASP-2 antibodies and inhibitory peptides. Additional components of pharmaceutical compositions include petroleum (such as of animal, vegetable or synthetic origin), for example, soybean oil and mineral oil. In general, glycols such as propylene glycol or polyethylene glycol are preferred liquid carriers for injectable solutions.

The anti-MASP-2 antibodies and inhibitory peptides can also be administered in the form of a depot injection or implant preparation that can be formulated in such a manner as to permit a sustained or pulsatile release of the active agents.

### MODES OF ADMINISTRATION

The pharmaceutical compositions comprising MASP-2 inhibitory agents may be administered in a number of ways depending on whether a local or systemic mode of administration is most appropriate for the condition being treated. Additionally, as described herein above with respect to extracorporeal reperfusion procedures, MASP-2 inhibitory agents can be administered via introduction of the compositions of the present invention to recirculating blood or plasma. Further, the compositions of the present invention can be delivered by coating or incorporating the compositions on or into an implantable medical device.

### SYSTEMIC DELIVERY

As used herein, the terms "systemic delivery" and "systemic administration" are intended to include but are not limited to oral and parenteral routes including intramuscular (IM), subcutaneous, intravenous (IV), intra-arterial, inhalational, sublingual, buccal, topical, transdermal, nasal, rectal, vaginal and other routes of administration that effectively result in dispersement of the delivered agent to a single or multiple sites of intended therapeutic action. Preferred routes of systemic delivery for the present compositions include intravenous, intramuscular, subcutaneous and inhalational. It will be appreciated that the exact systemic administration route for selected agents utilized in particular compositions of the present invention will be determined in part to account for the agent's susceptibility to metabolic transformation pathways associated with a given route of administration. For example, peptidergic agents may be most suitably administered by routes other than oral.

MASP-2 inhibitory antibodies and polypeptides can be delivered into a subject in need thereof by any suitable means. Methods of delivery of MASP-2 antibodies and polypeptides include administration by oral, pulmonary, parenteral (e.g., intramuscular, intraperitoneal, intravenous (IV) or subcutaneous injection), inhalation (such as via a fine powder formulation), transdermal, nasal, vaginal, rectal, or sublingual routes of administration, and can be formulated in dosage forms appropriate for each route of administration.

By way of representative example, MASP-2 inhibitory antibodies and peptides can be introduced into a living body by application to a bodily membrane capable of absorbing the polypeptides, for example the nasal, gastrointestinal and rectal membranes. The polypeptides are typically applied to the absorptive membrane in conjunction with a permeation enhancer. (See, e.g., Lee, V.H.L., Crit. Rev. Ther. Drug Carrier Sys. 5:69, 1988; Lee, V.H.L., J. Controlled Release 13:213, 1990; Lee, V.H.L., Ed., Peptide and Protein Drug Delivery, Marcel Dekker, New York (1991); DeBoer, A.G., et al., J. Controlled Release, 13:241, 1990.) For example, STDHF is a synthetic derivative of fusidic acid, a steroidal surfactant that is similar in structure to the bile salts, and has been used as a permeation enhancer for nasal delivery. (Lee, W.A., Biopharm. 22, Nov./Dec. 1990.)

The MASP-2 inhibitory antibodies and polypeptides may be introduced in association with another molecule, such as a lipid, to protect the polypeptides from enzymatic degradation. For example, the covalent attachment of polymers, especially polyethylene glycol (PEG), has been used to protect certain proteins from enzymatic hydrolysis in the body and thus prolong half-life (Fuertges, F., et al., J. Controlled Release 11:139, 1990). Many polymer systems have been reported for protein delivery (Bae, Y.H., et al., J. Controlled Release 9:271, 1989; Hori, R., et al., Pharm. Res. 6:813, 1989; Yamakawa, I., et al., J. Pharm. Sci. 79:505, 1990; Yoshihiro, I., et al., J. Controlled Release 10:195, 1989; Asano, M., et al., J. Controlled Release 9:111, 1989; Rosenblatt, J., et al., J. Controlled Release 9:195, 1989; Makino, K., J. Controlled Release 12:235, 1990; Takakura, Y., et al., J. Pharm. Sci. 78:117, 1989; Takakura, Y., et al., J. Pharm. Sci. 78:219, 1989).

Recently, liposomes have been developed with improved serum stability and circulation half-times (see, e.g., U.S. Patent No. 5,741,516 to Webb). Furthermore, various methods of liposome and liposome-like preparations as potential drug carriers have been reviewed (see, e.g., U.S. Patent No. 5,567,434 to Szoka; U.S. Patent No. 5,552,157 to Yagi; U.S. Patent No. 5,565,213 to Nakamori; U.S. Patent No. 5,738,868 to Shinkarenko and U.S. Patent No. 5,795,587 to Gao).

For transdermal applications, the MASP-2 inhibitory antibodies and polypeptides may be combined with other suitable ingredients, such as carriers and/or adjuvants. There are no limitations on the nature of such other ingredients, except that they must be pharmaceutically acceptable for their intended administration, and cannot degrade the activity of the active ingredients of the composition. Examples of suitable vehicles include ointments, creams, gels, or suspensions, with or without purified collagen. The MASP-2 inhibitory antibodies and polypeptides may also be impregnated into transdermal patches, plasters, and bandages, preferably in liquid or semi-liquid form.

The compositions of the present invention may be systemically administered on a periodic basis at intervals determined to maintain a desired level of therapeutic effect. For example, compositions may be administered, such as by subcutaneous injection, every two to four weeks or at less frequent intervals. The dosage regimen will be determined by the physician considering various factors that may influence the action of the combination of agents. These factors will include the extent of progress of the condition being treated, the patient's age, sex and weight, and other clinical factors. The dosage for each individual agent will vary as a function of the MASP-2 inhibitory agent that is included in the composition, as well as the presence and nature of any drug delivery vehicle (e.g., a sustained release delivery vehicle). In addition, the dosage quantity may be adjusted to account for variation in the frequency of administration and the pharmacokinetic behavior of the delivered agent(s).

### TREATMENT REGIMENS

In prophylactic applications, the pharmaceutical compositions are administered to a subject susceptible to, or otherwise at risk of, a condition associated with MASP-2-dependent complement activation in an amount sufficient to eliminate or reduce the risk of developing symptoms of the condition. In therapeutic applications, the pharmaceutical compositions are administered to a subject suspected of, or already suffering from, a condition associated with MASP-2-dependent complement activation in a therapeutically effective amount sufficient to relieve, or at least partially reduce, the symptoms of the condition. In both prophylactic and therapeutic regimens, compositions comprising MASP-2 inhibitory agents may be administered in several dosages until a sufficient therapeutic outcome has been achieved in the subject. Application of the MASP-2 inhibitory compositions of the present invention may be carried out by a single administration of the composition, or a limited sequence of administrations, for treatment of an acute condition, e.g., reperfusion injury or other traumatic injury. Alternatively, the composition may be administered at periodic intervals over an extended period of time for treatment of chronic conditions, e.g., arthritides or psoriasis.

The uses and compositions of the present invention may be used to inhibit inflammation and related processes that typically result from diagnostic and therapeutic medical and surgical procedures. To inhibit such processes, the MASP-2 inhibitory composition of the present invention may be applied periprocedurally. As used herein "periprocedurally" refers to administration of the inhibitory composition preprocedurally and/or intraprocedurally and/or postprocedurally, i.e., before the procedure, before and during the procedure, before and after the procedure, before, during and after the procedure, during the procedure, during and after the procedure, or after the procedure. Periprocedural application may be carried out by local administration of the composition to the surgical or procedural site, such as by injection or continuous or intermittent irrigation of the site, or by systemic administration. Suitable methods for local perioperative delivery of MASP-2 inhibitory agent solutions are disclosed in US Patent Nos. 6,420,432 to Demopulos and 6,645,168 to Demopulos. Suitable methods for local delivery of chondroprotective compositions including MASP-2 inhibitory agent(s) are disclosed in International PCT Patent Application WO 01/07067 A2. Suitable methods and compositions for targeted systemic delivery of chondroprotective compositions including MASP-2 inhibitory agent(s) are disclosed in International PCT Patent Application WO 03/063799 A2.

### VI. EXAMPLES

The following examples merely illustrate the best mode now contemplated for practicing the invention, but should not be construed to limit the invention.

### EXAMPLE 1

This example describes the generation of a mouse strain deficient in MASP-2 (MASP-2-/-) but sufficient of MAp19 (MAp19+/+).

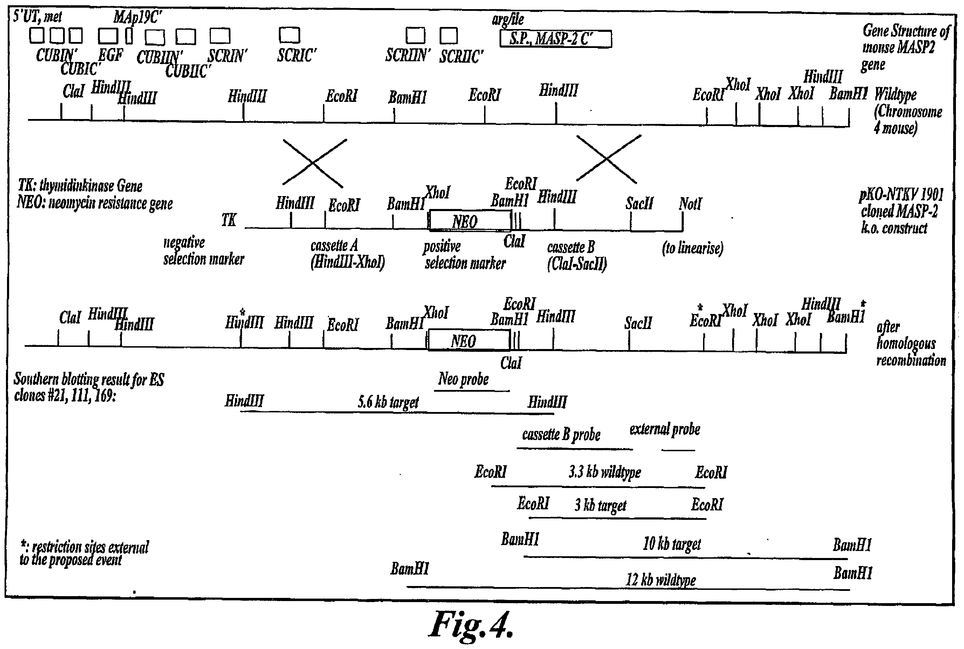

Materials and Methods: The targeting vector pKO-NTKV 1901 was designed to disrupt the three exons coding for the C-terminal end of murine MASP-2, including the exon that encodes the serine protease domain, as shown in FIGURE 4. PKO-NTKV 1901 was used to transfect the murine ES cell line E14.1a (SV129 Ola). Neomycin-resistant and Thymidine Kinase-sensitive clones were selected. 600 ES clones were screened and of these, four different clones were identified and verified by southern blot to contain the expected selective targeting and recombination event as shown in FIGURE 4. Chimeras were generated from these four positive clones by embryo transfer. The chimeras were then backcrossed in the genetic background C57/BL6 to create transgenic males. The transgenic males were crossed with females to generate F1s with 50% of the offspring showing heterozygosity for the disrupted *MASP 2* gene. The heterozygous mice were intercrossed to generate homozygous MASP-2 deficient offspring, resulting in heterozygous and wild-type mice in the ration of 1:2:1, respectively.

Results and Phenotype: The resulting homozygous MASP-2-/- deficient mice were found to be viable and fertile and were verified to be MASP-2 deficient by southern blot to confirm the correct targeting event, by Northern blot to confirm the absence of MASP-2 mRNA, and by Western blot to confirm the absence of MASP-2 protein (data not shown). The presence of MAp19 mRNA and the absence of MASP-2 mRNA was further confirmed using time-resolved RT-PCR on a LightCycler machine. The MASP-2-/- mice do continue to express MAp19, MASP-1 and MASP-3 mRNA and protein as expected (data not shown). The presence and abundance of mRNA in the MASP-2-/- mice for Properdin, Factor B, Factor D, C4, C2 and C3 was assessed by LightCycler analysis and found to be identical to that of the wild-type littermate controls (data not shown). The plasma from homozygous MASP-2-/- mice is totally deficient of lectin-pathway-mediated complement activation and alternative pathway complement activation as further described in Example 2.

Generation of a MASP-2-/- strain on a pure C57BL6 Background: The MASP-2-/- mice are back-crossed with a pure C57BL6 line for nine generations prior to use of the MASP-2-/- strain as an experimental animal model.

### EXAMPLE 2

This example demonstrates that MASP-2 is required for complement activation via the alternative and the lectin pathway.

### Methods and Materials:

Lectin pathway specific C4 Cleavage Assay: A C4 cleavage assay has been described by Petersen, et al., J. Immunol. Methods 257:107 (2001) that measures lectin pathway activation resulting from lipoteichoic acid (LTA) from *S. aureus* which binds L-ficolin. The assay described in example 11 was adapted to measure lectin pathway activation via MBL by coating the plate with LPS and mannan or zymosan prior to adding serum from MASP-2 -/- mice as described below. The assay was also modified to remove the possibility of C4 cleavage due to the classical pathway. This was achieved by using a sample dilution buffer containing 1 M NaCl, which permits high affinity binding of lectin pathway recognition components to their ligands, but prevents activation of endogenous C4, thereby excluding the participation of the classical pathway by dissociating the C1 complex. Briefly described, in the modified assay serum samples (diluted in high salt (1M NaCl) buffer) are added to ligand-coated plates, followed by the addition of a constant amount of purified C4 in a buffer with a physiological concentration of salt. Bound recognition complexes containing MASP-2 cleave the C4, resulting in C4b deposition.

### Assay Methods:

1) Nunc Maxisorb microtiter plates (Maxisorb, Nunc, cat. No. 442404, Fisher Scientific) were coated with 1 µg/ml mannan (M7504 Sigma) or any other ligand (e.g., such as those listed below) diluted in coating buffer (15 mM Na₂CO₃, 35 mM NaHCO₃, pH 9.6).
   The following reagents were used in the assay:
   a. mannan (1 µg/well mannan (M7504 Sigma) in 100 µl coating buffer):
   b. zymosan (1µg/well zymosan (Sigma) in 100µl coating buffer);
   c. LTA (1µg/well in 100 µl coating buffer or 2 µg/well in 20 µl methanol)
   d. 1 µg of the H-ficolin specific Mab 4H5 in coating buffer
   e. PSA from Aerococcus viridans (2 µg/well in 100 µl coating buffer)
   f. 100 µl/well of formalin-fixed *S. aureus* DSM20233 (OD₅₅₀=0.5) in coating buffer.
2) The plates were incubated overnight at 4°C.
3) After overnight incubation, the residual protein binding sites were saturated by incubated the plates with 0.1% HSA-TBS blocking buffer (0.1% (w/v) HSA in 10 mM Tris-CL, 140 mM NaCl, 1.5 mM NaN₃, pH 7.4) for 1-3 hours, then washing the plates 3X with TBS/tween/Ca²⁺ (TBS with 0.05% Tween 20 and 5 mM CaCl₂, 1 mM MgCl₂, pH 7.4).
4) Serum samples to be tested were diluted in MBL-binding buffer (1 M NaCl) and the diluted samples were added to the plates and incubated overnight at 4°C. Wells receiving buffer only were used as negative controls.
5) Following incubation overnight at 4°C, the plates were washed 3X with TBS/tween/Ca2+. Human C4 (100 µl/well of 1 µg/ml diluted in BBS (4 mM barbital, 145 mM NaCl, 2 mM CaCl₂, 1 mM MgCl₂, pH 7.4)) was then added to the plates and incubated for 90 minutes at 37°C. The plates were washed again 3X with TBS/tween/Ca²⁺.
6) C4b deposition was detected with an alkaline phosphatase-conjugated chicken anti-human C4c (diluted 1:1000 in TBS/tween/Ca²⁺), which was added to the plates and incubated for 90 minutes at room temperature. The plates were then washed again 3X with TBS/tween/Ca²⁺.
7) Alkaline phosphatase was detected by adding 100 µl of *p*-nitrophenyl phosphate substrate solution, incubating at room temperature for 20 minutes, and reading the OD₄₀₅ in a microtiter plate reader.

Results: FIGURE 6A-B show the amount of C4b deposition on mannan (FIGURE 6A) and zymosan (FIGURE 6B) in serum dilutions from MASP-2+/+ (crosses), MASP-2+/- (closed circles) and MASP-2-/- (closed triangles). FIGURE 6C shows the relative C4 convertase activity on plates coated with zymosan (white bars) or mannan (shaded bars) from MASP-2-/+ mice (n=5) and MASP-2-/- mice (n=4) relative to wild-type mice (n=5) based on measuring the amount of C4b deposition normalized to wild-type serum. The error bars represent the standard deviation. As shown in FIGURES 6A-C, plasma from MASP-2-/- mice is totally deficient in lectin-pathway-mediated complement activation on mannan and on zymosan coated plates. These results clearly demonstrate that MASP-2, but not MASP-1 or MASP-3, is the effector component of the lectin pathway.

### C3b deposition assay:

1) Nunc Maxisorb microtiter plates (Maxisorb, Nunc, cat. No. 442404, Fisher Scientific) are coated with 1 µg/well mannan (M7504 Sigma) or any other ligand diluted in coating buffer (15 mM Na₂CO₃, 35 mM NaHCO₃, pH 9.6) and incubated overnight at 4°C.
2) Residual protein binding sites are saturated by incubating the plate with 0.1% HSA-TBS blocking buffer (0.1% (w/v) HSA in 10 mM Tris-CL, 140 mM NaCl, 1.5 mM NaN₃, pH 7.4) for 1-3 hours.
3) Plates are washed in TBS/tw/Ca⁺⁺(TBS with 0.05% Tween 20 and 5 mM CaCl₂) and diluted BBS is added to serum samples (4 mM barbital, 145 mM NaCl, 2 mM CaCl₂, 1 mM MgCl₂, pH 7.4). Wells receiving only buffer are used as negative controls. A control set of serum samples obtained from wild-type or MASP-2-/- mice are C1q depleted prior to use in the assay. Clq-depleted mouse serum was prepared using protein-A-coupled Dynabeads (Dynal Biotech, Oslo, Norway) coated with rabbit anti-human C1q IgG (Dako, Glostrup, Denmark), according to the supplier's instructions.
4) Following incubation overnight at 4°C, and another wash with TBS/tw/ Ca⁺⁺, converted and bound C3 is detected with a polyclonal anti-human-C3c Antibody (Dako A 062) diluted in TBS/tw/ Ca⁺⁺ at 1:1000). The secondary antibody is goat anti-rabbit IgG (whole molecule) conjugated to alkaline-phosphatase (Sigma Immunochemicals A-3812) diluted 1:10,000 in TBS/tw/Ca⁺⁺. The presence of alternative complement pathway (AP) is determined by addition of 100 µl substrate solution (Sigma Fast p-Nitrophenyl Phosphate tablet sets, Sigma) and incubation at room temperature. Hydrolysis is monitored quantitatively by measuring the absorption at 405 nm in a microtiter plate reader. A standard curve is prepared for each analysis using serial dilutions of plasma/serum samples.

Results: The results shown in FIGURES 7A and 7B are from pooled serum from several mice. The crosses represent MASP-2+/+ serum, the filled circles represent C1q depleted MASP-2+/+ serum, the open squares represent MASP-2-/- serum and the open triangles represent C1q depleted MASP-2-/- serum. As shown in FIGURES 7A-B, serum from MASP-2-/- mice tested in a C3b deposition assay results in very low levels of C3 activation on mannan (FIGURE 7A) and on zymosan (FIGURE 7B) coated plates. This result clearly demonstrates that MASP-2 is required to contribute the initial C3b generation from C3 to initiate the alternative complement pathway. This is a surprising result in view of the widely accepted view that complement factors C3, factor B, factor D and properdin form an independent functional alternative pathway in which C3 can undergo a spontaneous conformational change to a "C3b-like" form which then generates a fluid phase convertase iC3Bb and deposits C3b molecules on activation surfaces such as zymosan.

### Recombinant MASP-2 reconstitutes Lectin Pathway-Dependent C4 Activation in serum from the MASP-2-/- mice

In order to establish that the absence of MASP-2 was the direct cause of the loss of lectin pathway-dependent C4 activation in the MASP-2-/- mice, the effect of adding recombinant MASP-2 protein to serum samples was examined in the C4 cleavage assay described above. Functionally active murine MASP-2 and catalytically inactive murine MASP-2A (in which the active-site serine residue in the serine protease domain was substituted for the alanine residue) recombinant proteins were produced and purified as described below in Example 5. Pooled serum from 4 MASP-2 -/- mice was pre-incubated with increasing protein concentrations of recombinant murine MASP-2 or inactive recombinant murine MASP-2A and C4 convertase activity was assayed as described above.

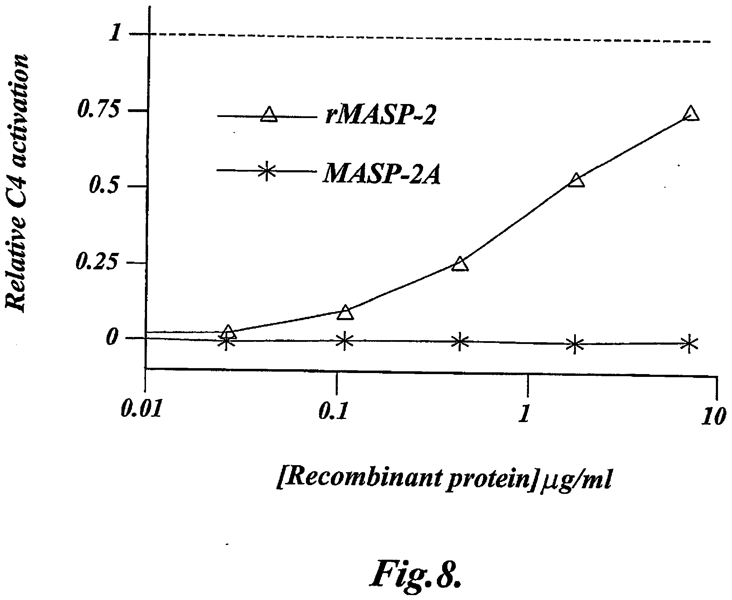

Results: As shown in FIGURE 8, the addition of functionally active murine recombinant MASP-2 protein (shown as open triangles) to serum obtained from the MASP-2 -/- mice restored lectin pathway-dependent C4 activation in a protein concentration dependent manner, whereas the catalytically inactive murine MASP-2A protein (shown as stars) did not restore C4 activation. The results shown in FIGURE 8 are normalized to the C4 activation observed with pooled wild-type mouse serum (shown as a dotted line).

### EXAMPLE 3

This example describes the generation of a transgenic mouse strain that is murine MASP-2-/-, MAp19+/+ and that expresses a human MASP-2 transgene (a murine MASP-2 knock-out and a human MASP-2 knock-in).

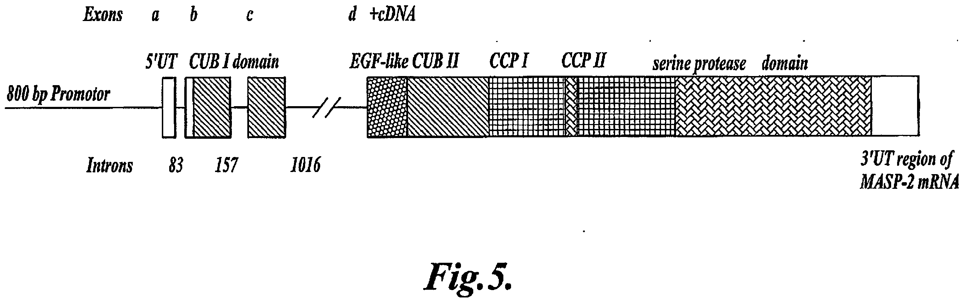

Materials and Methods: A minigene encoding human MASP-2 called "mini hMASP-2" (SEQ ID NO:49) as shown in FIGURE 5 was constructed which includes the promoter region of the human *MASP 2* gene, including the first 3 exons (exon 1 to exon 3) followed by the cDNA sequence that represents the coding sequence of the following 8 exons, thereby encoding the full-length MASP-2 protein driven by its endogenous promoter. The mini hMASP-2 construct was injected into fertilized eggs of MASP-2-/- in order to replace the deficient murine *MASP 2* gene by transgenically expressed human MASP-2.

### EXAMPLE 4

This example describes the isolation of human MASP-2 protein in proenzyme form from human serum.

Method of human MASP-2 isolation: A method for isolating MASP-2 from human serum has been described in Matsushita et al., J. Immunol. 165:2637-2642, 2000. Briefly, human serum is passed through a yeast mannan-Sepharose column using a 10 mM imidazole buffer (pH 6.0) containing 0.2 M NaCl, 20 mM CaCl₂, 0.2 mM NPGB, 20 µM *p*-APMSF, and 2% mannitol. The MASP-1 and MASP-2 proenzymes complex with MBL and elute with the above buffer containing 0.3 M mannose. To separate proenzymes MASP-1 and MASP-2 from MBL, preparations containing the complex are applied to anti-MBL-Sepharose and then MASPs are eluted with imidazole buffer containing 20 mM EDTA and 1 M NaCl. Finally, proenzymes MASP-1 and MASP-2 are separated from each other by passing through anti-MASP-1-Sepharose in the same buffer as used for the anti-MBL-Sepharose. MASP-2 is recovered in the effluents, whereas MASP-1 is eluted with 0.1 M glycine buffer (pH 2.2).

### EXAMPLE 5

This example describes the recombinant expression and protein production of recombinant full-length human, rat and murine MASP-2, MASP-2 derived polypeptides, and catalytically inactivated mutant forms of MASP-2

### Expression of Full-length human, murine and rat MASP-2:

The full length cDNA sequence of human MASP-2 (SEQ ID NO: 4) was also subcloned into the mammalian expression vector pCI-Neo (Promega), which drives eukaryotic expression under the control of the CMV enhancer/promoter region (described in Kaufman R.J. et al., Nucleic Acids Research 19:4485-90, 1991; Kaufman, Methods in Enzymology, 185:537-66 (1991)). The full length mouse cDNA (SEQ ID NO:50) and rat MASP-2 cDNA (SEQ ID NO:53) were each subcloned into the pED expression vector. The MASP-2 expression vectors were then transfected into the adherent Chinese hamster ovary cell line DXB1 using the standard calcium phosphate transfection procedure described in Maniatis et al., 1989. Cells transfected with these constructs grew very slowly, implying that the encoded protease is cytotoxic.

In another approach, the minigene construct (SEQ ID NO:49) containing the human cDNA of MASP-2 driven by its endogenous promoter is transiently transfected into Chinese hamster ovary cells (CHO). The human MASP-2 protein is secreted into the culture media and isolated as described below.

### Expression of Full-length catalytically inactive MASP-2:

Rationale: MASP-2 is activated by autocatalytic cleavage after the recognition subcomponents MBL or ficolins (either L-ficolin, H-ficolin or M-ficolin) bind to their respective carbohydrate pattern. Autocatalytic cleavage resulting in activation of MASP-2 often occurs during the isolation procedure of MASP-2 from serum, or during the purification following recombinant expression. In order to obtain a more stable protein preparation for use as an antigen, a catalytically inactive form of MASP-2, designed as MASP-2A was created by replacing the serine residue that is present in the catalytic triad of the protease domain with an alanine residue in rat (SEQ ID NO:55 Ser617 to Ala617); in mouse (SEQ ID NO:52 Ser617 to Ala617); or in human (SEQ ID NO:3 Ser618 to Ala618).

In order to generate catalytically inactive human and murine MASP-2A proteins, site-directed mutagenesis was carried out using the oligonucleotides shown in TABLE 5. The oligonucleotides in TABLE 5 were designed to anneal to the region of the human and murine cDNA encoding the enzymatically active serine and oligonucleotide contain a mismatch in order to change the serine codon into an alanine codon. For example, PCR oligonucleotides SEQ ID NOS:56-59 were used in combination with human MASP-2 cDNA (SEQ ID NO: 4) to amplify the region from the start codon to the enzymatically active serine and from the serine to the stop codon to generate the complete open reading from of the mutated MASP-2A containing the Ser618 to Ala618 mutation. The PCR products were purified after agarose gel electrophoresis and band preparation and single adenosine overlaps were generated using a standard tailing procedure. The adenosine tailed MASP-2A was then cloned into the pGEM-T easy vector, transformed into *E. coli.*

A catalytically inactive rat MASP-2A protein was generated by kinasing and annealing SEQ ID NO: 64 and SEQ ID NO: 65 by combining these two oligonucleotides in equal molar amounts, heating at 100°C for 2 minutes and slowly cooling to room temperature. The resulting annealed fragment has Pst1 and Xba1 compatible ends and was inserted in place of the PstI-XbaI fragment of the wild-type rat MASP-2 cDNA (SEQ ID NO: 53) to generate rat MASP-2A.
5 'GAGGTGACGCAGGAGGGGCATTAGTGTTT 3' (SEQ ID NO: 64)
5' CTAGAAACACTAATGCCCCTCCTGCGTCACCTCTGCA 3' (SEQ ID NO: 65)

The human, murine and rat MASP-2A were each further subcloned into either of the mammalian expression vectors pED or pCI-Neo and transfected into the Chinese Hamster ovary cell line DXB1 as described below.

In another approach, a catalytically inactive form of MASP-2 is constructed using the method described in Chen et al., J. Biol. Chem., 276(28):25894-25902, 2001. Briefly, the plasmid containing the full-length human MASP-2 cDNA (described in Thiel et al., Nature 386:506, 1997) is digested with *Xho*1 and *EcoR*1 and the MASP-2 cDNA (described herein as SEQ ID NO:4) is cloned into the corresponding restriction sites of the pFastBac1 baculovirus transfer vector (Life Technologies, NY). The MASP-2 serine protease active site at Ser618 is then altered to Ala618 by substituting the doublestranded oligonucleotides encoding the peptide region amino acid 610-625 (SEQ ID NO:13) with the native region amino acids 610 to 625 to create a MASP-2 full length polypeptide with an inactive protease domain. Construction of Expression Plasmids Containing Polypeptide Regions Derived from Human Masp-2

The following constructs are produced using the MASP-2 signal peptide (residues 1-15 of SEQ ID NO:5) to secrete various domains of MASP-2. A construct expressing the human MASP-2 CUBI domain (SEQ ID NO:8) is made by PCR amplifying the region encoding residues 1-121 of MASP-2 (SEQ ID NO:6) (corresponding to the N-terminal CUB1 domain). A construct expressing the human MASP-2 CUBIEGF domain (SEQ ID NO:9) is made by PCR amplifying the region encoding residues 1-166 of MASP-2 (SEQ ID NO:6) (corresponding to the N-terminal CUB1EGF domain). A construct expressing the human MASP-2 CUBIEGFCUBII domain (SEQ ID NO:10) is made by PCR amplifying the region encoding residues 1-293 of MASP-2 (SEQ ID NO:6) (corresponding to the N-terminal CUBIEGFCUBII domain). The above mentioned domains are amplified by PCR using Vent_{R} polymerase and pBS-MASP-2 as a template, according to established PCR methods. The 5' primer sequence of the sense primer (5'-CGGGATCCATGAGGCTGCTGACCCTC-3' SEQ ID NO:34) introduces a *Bam*HI restriction site (underlined) at the 5' end of the PCR products. Antisense primers for each of the MASP-2 domains, shown below in TABLE 5, are designed to introduce a stop codon (boldface) followed by an *Eco*RI site (underlined) at the end of each PCR product. Once amplified, the DNA fragments are digested with *Bam*HI and *Eco*RI and cloned into the corresponding sites of the pFastBac1 vector. The resulting constructs are characterized by restriction mapping and confirmed by dsDNA sequencing.

**TABLE 5: MASP-2 PCR PRIMERS**

| **MASP-2 domain** | **5' PCR Primer** | **3' PCR Primer** |
|---|---|---|
| SEQ ID NO:8 | | 5'GGAATTCCTAGGCTGCATA (SEQ ID NO:35) |
| CUBI (aa 1-121 of SEQ ID NO:6) | | |
| SEQ ID NO:9 | | |
| CUBIEGF (aa 1-166 of SEQ ID NO:6) | | |
| SEQ ID NO:10 | | |
| CUBIEGFCUBII (aa 1-293 of SEQ ID NO:6) | | |
| SEQ ID NO:4 | 5'ATGAGGCTGCTGA CCCTCCTGGGCCTT C 3' (SEQ ID NO: 56) hMASP-2_forward | 5'TTAAAATCACTAATTATGTT CTCGATC 3' (SEQ ID NO: 59) hMASP-2_reverse |
| human MASP-2 | | |
| SEQ ID NO:4 | 5'CAGAGGTGACGCA GGAGGGGCAC 3' (SEQ ID NO: 58) hMASP-2_ala_forward | 5'GTGCCCCTCCTGCGTCACCT CTG 3' (SEQ ID NO: 57) hMASP-2_ala_reverse |
| human MASP-2 cDNA | | |
| SEQ ID NO:50 | 5'ATGAGGCTACTCA TCTTCCTGG3' (SEQ ID NO: 60) mMASP-2_forward | 5'TTAGAAATTACTTATTATGT TCTCAATCC3' (SEQ ID NO: 63) mMASP-2_reverse |
| Murine MASP-2 cDNA | | |
| SEQ ID NO:50 | 5'CCCCCCCTGCGTC ACCTCTGCAG3' (SEQ ID NO: 62) mMASP-2_ala_forward | 5'CTGCAGAGGTGACGCAGGG GGGG 3' (SEQ ID NO: 61) mMASP-2_ala_reverse |
| Murine MASP-2 cDNA | | |

### Recombinant eukaryotic expression of MASP-2 and protein production of enzymatically inactive mouse, rat, and human MASP-2A

The MASP-2 and MASP-2A expression constructs described above were transfected into DXB1 cells using the standard calcium phosphate transfection procedure (Maniatis et al., 1989). MASP-2A was produced in serum-free medium to ensure that preparations were not contaminated with other serum proteins. Media was harvested from confluent cells every second day (four times in total). The level of recombinant MASP-2A averaged approximately 1.5 mg/liter of culture medium for each of the three species.

MASP-2A protein purification: The MASP-2A (Ser-Ala mutant described above) was purified by affinity chromatography on MBP-A-agarose columns. This strategy enabled rapid purification without the use of extraneous tags. MASP-2A (100-200 ml of medium diluted with an equal volume of loading buffer (50 mM Tris-Cl, pH 7.5, containing 150 mM NaCl and 25 mM CaCl₂) was loaded onto an MBP-agarose affinity column (4 ml) pre-equilibrated with 10 ml of loading buffer. Following washing with a further 10 ml of loading buffer, protein was eluted in 1 ml fractions with 50 mM Tris-Cl, pH 7.5, containing 1.25 M NaCl and 10 mM EDTA. Fractions containing the MASP-2A were identified by SDS-polyacrylamide gel electrophoresis. Where necessary, MASP-2A was purified further by ion-exchange chromatography on a MonoQ column (HR 5/5). Protein was dialysed with 50 mM Tris-Cl pH 7.5, containing 50 mM NaCl and loaded onto the column equilibrated in the same buffer. Following washing, bound MASP-2A was eluted with a 0.05-1 M NaCl gradient over 10 ml.

Results: Yields of 0.25-0.5 mg of MASP-2A protein were obtained from 200 ml of medium. The molecular mass of 77.5 kDa determined by MALDI-MS is greater than the calculated value of the unmodified polypeptide (73.5 kDa) due to glycosylation. Attachment of glycans at each of the *N*-glycosylation sites accounts for the observed mass. MASP-2A migrates as a single band on SDS-polyacrylamide gels, demonstrating that it is not proteolytically processed during biosynthesis. The weight-average molecular mass determined by equilibrium ultracentrifugation is in agreement with the calculated value for homodimers of the glycosylated polypeptide.

### PRODUCTION OF RECOMBINANT HUMAN MASP-2 POLYPEPTIDES

Another method for producing recombinant MASP-2 and MASP2A derived polypeptides is described in Thielens, N.M., et al., J. Immunol. 166:5068-5077, 2001. Briefly, the *Spodoptera frugiperda* insect cells (Ready-Plaque Sf9 cells obtained from Novagen, Madison, WI) are grown and maintained in Sf900II serum-free medium (Life Technologies) supplemented with 50 IU/ml penicillin and 50 mg/ml streptomycin (Life Technologies). The *Trichoplusia ni* (High Five) insect cells (provided by Jadwiga Chroboczek, Institut de Biologie Structurale, Grenoble, France) are maintained in TC100 medium (Life Technologies) containing 10% FCS (Dominique Dutscher, Brumath, France) supplemented with 50 IU/ml penicillin and 50 mg/ml streptomycin. Recombinant baculoviruses are generated using the Bac-to-Bac system (Life Technologies). The bacmid DNA is purified using the Qiagen midiprep purification system (Qiagen) and is used to transfect Sf9 insect cells using cellfectin in Sf900 II SFM medium (Life Technologies) as described in the manufacturer's protocol. Recombinant virus particles are collected 4 days later, titrated by virus plaque assay, and amplified as described by King and Possee, in The Baculovirus Expression System: A Laboratory Guide, Chapman and Hall Ltd., London, pp. 111-114, 1992.

High Five cells (1.75 x 10⁷ cells/175-cm² tissue culture flask) are infected with the recombinant viruses containing MASP-2 polypeptides at a multiplicity of infection of 2 in Sf900 II SFM medium at 28°C for 96 h. The supernatants are collected by centrifugation and diisopropyl phosphorofluoridate is added to a final concentration of 1 mM.

The MASP-2 polypeptides are secreted in the culture medium. The culture supernatants are dialyzed against 50 mM NaCl, 1 mM CaCl₂, 50 mM triethanolamine hydrochloride, pH 8.1, and loaded at 1.5 ml/min onto a Q-Sepharose Fast Flow column (Amersham Pharmacia Biotech) (2.8 x 12 cm) equilibrated in the same buffer. Elution is conducted by applying a1.2 liter linear gradient to 350 mM NaCl in the same buffer. Fractions containing the recombinant MASP-2 polypeptides are identified by Western blot analysis, precipitated by addition of (NH₄)₂SO₄ to 60% (w/v), and left overnight at 4°C. The pellets are resuspended in 145 mM NaCl, 1 mM CaCl₂, 50 mM triethanolamine hydrochloride, pH 7.4, and applied onto a TSK G3000 SWG column (7.5 x 600 mm) (Tosohaas, Montgomeryville, PA) equilibrated in the same buffer. The purified polypeptides are then concentrated to 0.3 mg/ml by ultrafiltration on Microsep microconcentrators (m.w. cut-off = 10,000) (Filtron, Karlstein, Germany).

### EXAMPLE 6

This example describes a method of producing polyclonal antibodies against MASP-2 polypeptides.

### Materials and Methods:

MASP-2 Antigens: Polyclonal anti-human MASP-2 antiserum is produced by immunizing rabbits with the following isolated MASP-2 polypeptides: human MASP-2 (SEQ ID NO:6) isolated from serum as described in Example 4; recombinant human MASP-2 (SEQ ID NO:6), MASP-2A containing the inactive protease domain (SEQ ID NO:13), as described in Examples 4-5; and recombinant CUBI (SEQ ID NO:8), CUBEGFI (SEQ ID NO:9), and CUBEGFCUBII (SEQ ID NO:10) expressed as described above in Example 5.

Polyclonal antibodies: Six-week old Rabbits, primed with BCG (bacillus Calmette-Guerin vaccine) are immunized by injecting 100 µg of MASP-2 polypeptide at 100 µg/ml in sterile saline solution. Injections are done every 4 weeks, with antibody titer monitored by ELISA assay as described in Example 7. Culture supernatants are collected for antibody purification by protein A affinity chromatography.

### EXAMPLE 7

This example describes a method for producing murine monoclonal antibodies against rat or human MASP-2 polypeptides.

### Materials and Methods:

Male A/J mice (Harlan, Houston, Tex.), 8-12 weeks old, are injected subcutaneously with 100 µg human or rat rMASP-2 or rMASP-2A polypeptides (made as described in Example 4 or Example 5) in complete Freund's adjuvant (Difco Laboratories, Detroit, Mich.) in 200 µl of phosphate buffered saline (PBS) pH 7.4. At two-week intervals the mice are twice injected subcutaneously with 50 µg of human or rat rMASP-2 or rMASP-2A polypeptide in incomplete Freund's adjuvant. On the fourth week the mice are injected with 50 µg of human or rat rMASP-2 or rMASP-2A polypeptide in PBS and are fused 4 days later.

For each fusion, single cell suspensions are prepared from the spleen of an immunized mouse and used for fusion with Sp2/0 myeloma cells. 5x10⁸ of the Sp2/0 and 5x10⁸ spleen cells are fused in a medium containing 50% polyethylene glycol (M.W. 1450) (Kodak, Rochester, N.Y.) and 5% dimethylsulfoxide (Sigma Chemical Co., St. Louis, Mo.). The cells are then adjusted to a concentration of 1.5x10⁵ spleen cells per 200 µl of the suspension in Iscove medium (Gibco, Grand Island, N.Y.), supplemented with 10% fetal bovine serum, 100 units/ml of penicillin, 100 µg/ml of streptomycin, 0.1 mM hypoxanthine, 0.4 µM aminopterin and 16 µM thymidine. Two hundred microliters of the cell suspension are added to each well of about twenty 96-well microculture plates. After about ten days culture supernatants are withdrawn for screening for reactivity with purified factor MASP-2 in an ELISA assay.

ELISA Assay: Wells of Immulon 2 (Dynatech Laboratories, Chantilly, Va.) microtest plates are coated by adding 50 µl of purified hMASP-2 at 50 ng/ml or rat rMASP-2 (or rMASP-2A) overnight at room temperature. The low concentration of MASP-2 for coating enables the selection of high-affinity antibodies. After the coating solution is removed by flicking the plate, 200 µl of BLOTTO (non-fat dry milk) in PBS is added to each well for one hour to block the non-specific sites. An hour later, the wells are then washed with a buffer PBST (PBS containing 0.05% Tween 20). Fifty microliters of culture supernatants from each fusion well is collected and mixed with 50 µl of BLOTTO and then added to the individual wells of the microtest plates. After one hour of incubation, the wells are washed with PBST. The bound murine antibodies are then detected by reaction with horseradish peroxidase (HRP) conjugated goat anti-mouse IgG (Fc specific) (Jackson ImmunoResearch Laboratories, West Grove, Pa.) and diluted at 1:2,000 in BLOTTO. Peroxidase substrate solution containing 0.1% 3,3,5,5 tetramethyl benzidine (Sigma, St. Louis, Mo.) and 0.0003% hydrogen peroxide (Sigma) is added to the wells for color development for 30 minutes. The reaction is terminated by addition of 50 µl of 2M H₂SO₄ per well. The Optical Density at 450 nm of the reaction mixture is read with a BioTek ELISA Reader (BioTek Instruments, Winooski, Vt.).

### MASP-2 Binding Assay:

Culture supernatants that test positive in the MASP-2 ELISA assay described above can be tested in a binding assay to determine the binding affinity the MASP-2 inhibitory agents have for MASP-2. A similar assay can also be used to determine if the inhibitory agents bind to other antigens in the complement system.

Polystyrene microtiter plate wells (96-well medium binding plates, Coming Costar, Cambridge, MA) are coated with MASP-2 (20 ng/100 µl/well, Advanced Research Technology, San Diego, CA) in phosphate-buffered saline (PBS) pH 7.4 overnight at 4°C. After aspirating the MASP-2 solution, wells are blocked with PBS containing 1% bovine serum albumin (BSA; Sigma Chemical) for 2 h at room temperature. Wells without MASP-2 coating serve as the background controls. Aliquots of hybridoma supernatants or purified anti-MASP-2 MoAbs, at varying concentrations in blocking solution, are added to the wells. Following a 2 h incubation at room temperature, the wells are extensively rinsed with PBS. MASP-2-bound anti-MASP-2 MoAb is detected by the addition of peroxidase-conjugated goat anti-mouse IgG (Sigma Chemical) in blocking solution, which is allowed to incubate for 1h at room temperature. The plate is rinsed again thoroughly with PBS, and 100 µl of 3,3',5,5'-tetramethyl benzidine (TMB) substrate (Kirkegaard and Perry Laboratories, Gaithersburg, MD) is added. The reaction of TMB is quenched by the addition of 100 µl of 1M phosphoric acid, and the plate is read at 450 nm in a microplate reader (SPECTRA MAX 250, Molecular Devices, Sunnyvale, CA).

The culture supernatants from the positive wells are then tested for the ability to inhibit complement activation in a functional assay such as the C4 cleavage assay as described in Example 2. The cells in positive wells are then cloned by limiting dilution. The MoAbs are tested again for reactivity with hMASP-2 in an ELISA assay as described above. The selected hybridomas are grown in spinner flasks and the spent culture supernatant collected for antibody purification by protein A affinity chromatography.

### EXAMPLE 8

This example describes the generation of a MASP-2-/- knockout mouse expressing human MASP-2 for use as a model in which to screen for MASP-2 inhibitory agents.

Materials and Methods: A MASP-2-/- mouse as described in Example 1 and a MASP-2-/- mouse expressing a human MASP-2 transgene construct (human MASP-2 knock-in) as described in Example 3 are crossed, and progeny that are murine MASP-2-/-, murine MAp19+, human MASP-2+ are used to identify human MASP-2 inhibitory agents.

Such animal models can be used as test substrates for the identification and efficacy of MASP-2 inhibitory agents such as human anti-MASP-2 antibodies, MASP-2 inhibitory peptides and nonpeptides, and compositions comprising MASP-2 inhibitory agents. For example, the animal model is exposed to a compound or agent that is known to trigger MASP-2-dependent complement activation, and a MASP-2 inhibitory agent is administered to the animal model at a sufficient time and concentration to elicit a reduction of disease symptoms in the exposed animal.

In addition, the murine MASP-2-/-, MAp19+, human MASP-2+ mice may be used to generate cell lines containing one or more cell types involved in a MASP-2-associated disease which can be used as a cell culture model for that disorder. The generation of continuous cell lines from transgenic animals is well known in the art, for example see Small, J.A., et al., Mol. Cell Biol., 5:642-48, 1985.

### EXAMPLE 9

This example describes a method of producing human antibodies against human MASP-2 in a MASP-2 knockout mouse that expresses human MASP-2 and human immunoglobulins.

### Materials and Methods:

A MASP-2-/- mouse was generated as described in Example 1. A mouse was then constructed that expresses human MASP-2 as described in Example 3. A homozygous MASP-2-/- mouse and a MASP-2-/- mouse expressing human MASP-2 are each crossed with a mouse derived from an embryonic stem cell line engineered to contain targeted disruptions of the endogenous immunoglobulin heavy chain and light chain loci and expression of at least a segment of the human immunoglobulin locus. Preferably, the segment of the human immunoglobulin locus includes unrearranged sequences of heavy and light chain components. Both inactivation of endogenous immunoglobulin genes and introduction of exogenous immunoglobulin genes can be achieved by targeted homologous recombination. The transgenic mammals resulting from this process are capable of functionally rearranging the immunoglobulin component sequences and expressing a repertoire of antibodies of various isotypes encoded by human immunoglobulin genes, without expressing endogenous immunoglobulin genes. The production and properties of mammals having these properties is described, for example see Thomson, A.D., Nature 148:1547-1553, 1994, and Sloane, B.F., Nature Biotechnology 14:826, 1996. Genetically engineered strains of mice in which the mouse antibody genes are inactivated and functionally replaced with human antibody genes is commercially available (e.g., XenoMouse^{®}, available from Abgenix, Fremont CA). The resulting offspring mice are capable of producing human MoAb against human MASP-2 that are suitable for use in human therapy.

### EXAMPLE 10

This example describes the generation and production of humanized murine anti-MASP-2 antibodies and antibody fragments.

A murine anti-MASP-2 monoclonal antibody is generated in Male A/J mice as described in Example 7. The murine antibody is then humanized as described below to reduce its immunogenicity by replacing the murine constant regions with their human counterparts to generate a chimeric IgG and Fab fragment of the antibody, which is useful for inhibiting the adverse effects of MASP-2-dependent complement activation in human subjects in accordance with the present invention.

### 1. Cloning of anti-MASP-2 variable region genes from murine hybridoma cells.

Total RNA is isolated from the hybridoma cells secreting anti-MASP-2 MoAb (obtained as described in Example 7) using RNAzol following the manufacturer's protocol (Biotech, Houston, Tex.). First strand cDNA is synthesized from the total RNA using oligo dT as the primer. PCR is performed using the immunoglobulin constant C region-derived 3' primers and degenerate primer sets derived from the leader peptide or the first framework region of murine V_{H} or V_{K} genes as the 5' primers. Anchored PCR is carried out as described by Chen and Platsucas (Chen, P.F., Scand. J. Immunol. 35:539-549, 1992). For cloning the V_{K} gene, double-stranded cDNA is prepared using a Not1-MAK1 primer (5'-TGCGGCCGCTGTAGGTGCTGTCTTT-3' SEQ ID NO:38). Annealed adaptors AD1 (5'-GGAATTCACTCGTTATTCTCGGA-3' SEQ ID NO:39) and AD2 (5'-TCCGAGAATAACGAGTG-3' SEQ ID NO:40) are ligated to both 5' and 3' termini of the double-stranded cDNA. Adaptors at the 3' ends are removed by Not1 digestion. The digested product is then used as the template in PCR with the AD1 oligonucleotide as the 5' primer and MAK2 (5'-CATTGAAAGCTTTGGGGTAGAAGTTGTTC-3' SEQ ID NO:41) as the 3' primer. DNA fragments of approximately 500 bp are cloned into pUC19. Several clones are selected for sequence analysis to verify that the cloned sequence encompasses the expected murine immunoglobulin constant region. The Not1-MAK1 and MAK2 oligonucleotides are derived from the V_{K} region and are 182 and 84 bp, respectively, downstream from the first base pair of the C kappa gene. Clones are chosen that include the complete V_{K} and leader peptide.

For cloning the V_{H} gene, double-stranded cDNA is prepared using the Not1 MAG1 primer (5'-CGCGGCCGCAGCTGCTCAGAGTGTAGA-3' SEQ ID NO:42). Annealed adaptors AD1 and AD2 are ligated to both 5' and 3' termini of the double-stranded cDNA. Adaptors at the 3' ends are removed by Notl digestion. The digested product are used as the template in PCR with the AD1 oligonucleotide and MAG2 (5'-CGGTAAGCTTCACTGGCTCAGGGAAATA-3' SEQ ID NO:43) as primers. DNA fragments of 500 to 600 bp in length are cloned into pUC19. The Not1-MAG1 and MAG2 oligonucleotides are derived from the murine Cγ.7.1 region, and are 180 and 93 bp, respectively, downstream from the first bp of the murine Cγ.7.1 gene. Clones are chosen that encompass the complete V_{H} and leader peptide.

### 2. Construction of Expression Vectors for Chimeric MASP-2 IgG and Fab.

The cloned V_{H} and V_{K} genes described above are used as templates in a PCR reaction to add the Kozak consensus sequence to the 5' end and the splice donor to the 3' end of the nucleotide sequence. After the sequences are analyzed to confirm the absence of PCR errors, the V_{H} and V_{K} genes are inserted into expression vector cassettes containing human C.γ1 and C. kappa respectively, to give pSV2neoV_{H}-huCγ1 and pSV2neoV-huCγ. CsCl gradient-purified plasmid DNAs of the heavy- and light-chain vectors are used to transfect COS cells by electroporation. After 48 hours, the culture supernatant is tested by ELISA to confirm the presence of approximately 200 ng/ml of chimeric IgG. The cells are harvested and total RNA is prepared. First strand cDNA is synthesized from the total RNA using oligo dT as the primer. This cDNA is used as the template in PCR to generate the Fd and kappa DNA fragments. For the Fd gene, PCR is carried out using 5'-AAGAAGCTTGCCGCCACCATGGATTGGCTGTGGAACT-3' (SEQ ID NO:44) as the 5' primer and a CH1-derived 3' primer (5'-CGGGATCCTCAAACTTTCTTGTCCACCTTGG-3' SEQ ID NO:45). The DNA sequence is confirmed to contain the complete V_{H} and the CH1 domain of human IgG1. After digestion with the proper enzymes, the Fd DNA fragments are inserted at the HindIII and BamHI restriction sites of the expression vector cassette pSV2dhfr-TUS to give pSV2dhfrFd. The pSV2 plasmid is commercially available and consists of DNA segments from various sources: pBR322 DNA (thin line) contains the pBR322 origin of DNA replication (pBR ori) and the lactamase ampicillin resistance gene (Amp); SV40 DNA, represented by wider hatching and marked, contains the SV40 origin of DNA replication (SV40 ori), early promoter (5' to the dhfr and neo genes), and polyadenylation signal (3' to the dhfr and neo genes). The SV40-derived polyadenylation signal (pA) is also placed at the 3' end of the Fd gene.

For the kappa gene, PCR is carried out using 5'-AAGAAAGCTTGCCGCCACCATGTTCTCACTAGCTCT-3' (SEQ ID NO:46) as the 5' primer and a C_{K}-derived 3' primer (5'-CGGGATCCTTCTCCCTCTAACACTCT-3' SEQ ID NO:47). DNA sequence is confirmed to contain the complete V_{K} and human C_{K} regions. After digestion with proper restriction enzymes, the kappa DNA fragments are inserted at the HindIII and BamHI restriction sites of the expression vector cassette pSV2neo-TUS to give pSV2neoK. The expression of both Fd and .kappa genes are driven by the HCMV-derived enhancer and promoter elements. Since the Fd gene does not include the cysteine amino acid residue involved in the inter-chain disulfide bond, this recombinant chimeric Fab contains non-covalently linked heavy- and light-chains. This chimeric Fab is designated as cFab.

To obtain recombinant Fab with an inter-heavy and light chain disulfide bond, the above Fd gene may be extended to include the coding sequence for additional 9 amino acids (EPKSCDKTH SEQ ID NO:48) from the hinge region of human IgG1. The BstEII-BamHI DNA segment encoding 30 amino acids at the 3' end of the Fd gene may be replaced with DNA segments encoding the extended Fd, resulting in pSV2dhfrFd/9aa.

### 3. Expression and Purification of Chimeric Anti-MASP-2 IgG

To generate cell lines secreting chimeric anti-MASP-2 IgG, NSO cells are transfected with purified plasmid DNAs of pSV2neoV_{H}-huC.γ1 and pSV2neoV-huC kappa by electroporation. Transfected cells are selected in the presence of 0.7 mg/ml G418. Cells are grown in a 250 ml spinner flask using serum-containing medium.

Culture supernatant of 100 ml spinner culture is loaded on a 10-ml PROSEP-A column (Bioprocessing, Inc., Princeton, N.J.). The column is washed with 10 bed volumes of PBS. The bound antibody is eluted with 50 mM citrate buffer, pH 3.0. Equal volume of 1 M Hepes, pH 8.0 is added to the fraction containing the purified antibody to adjust the pH to 7.0. Residual salts are removed by buffer exchange with PBS by Millipore membrane ultrafiltration (M.W. cut-off: 3,000). The protein concentration of the purified antibody is determined by the BCA method (Pierce).

### 4. Expression and purification of chimeric anti-MASP-2 Fab

To generate cell lines secreting chimeric anti-MASP-2 Fab, CHO cells are transfected with purified plasmid DNAs of pSV2dhfrFd (or pSV2dhfrFd/9aa) and pSV2neokappa, by electroporation. Transfected cells are selected in the presence of G418 and methotrexate. Selected cell lines are amplified in increasing concentrations of methotrexate. Cells are single-cell subcloned by limiting dilution. High-producing single-cell subcloned cell lines are then grown in 100 ml spinner culture using serum-free medium.

Chimeric anti-MASP-2 Fab is purified by affinity chromatography using a mouse anti-idiotypic MoAb to the MASP-2 MoAb. An anti-idiotypic MASP-2 MoAb can be made by immunizing mice with a murine anti-MASP-2 MoAb conjugated with keyhole limpet hemocyanin (KLH) and screening for specific MoAb binding that can be competed with human MASP-2. For purification, 100 ml of supernatant from spinner cultures of CHO cells producing cFab or cFab/9aa are loaded onto the affinity column coupled with an anti-idiotype MASP-2 MoAb. The column is then washed thoroughly with PBS before the bound Fab is eluted with 50 mM diethylamine, pH 11.5. Residual salts are removed by buffer exchange as described above. The protein concentration of the purified Fab is determined by the BCA method (Pierce).

The ability of the chimeric MASP-2 IgG, cFab, and cFAb/9aa to inhibit MASP-2-dependent complement pathways may be determined by using the inhibitory assays described in Example 2.

### EXAMPLE 11

This example describes an *in vitro* C4 cleavage assay used as a functional screen to identify MASP-2 inhibitory agents capable of blocking MASP-2-dependent complement activation via L-ficolin/P35, H-ficolin, M-ficolin or mannan.

C4 Cleavage Assay: A C4 cleavage assay has been described by Petersen, S.V., et al., J. Immunol. Methods 257:107, 2001, which measures lectin pathway activation resulting from lipoteichoic acid (LTA) from *S. aureus* which binds L-ficolin.

Reagents: Formalin-fixed *S. aureous* (DSM20233) is prepared as follows: bacteria is grown overnight at 37°C in tryptic soy blood medium, washed three times with PBS, then fixed for 1 h at room temperature in PBS/0.5% formalin, and washed a further three times with PBS, before being resuspended in coating buffer (15 mM Na₂Co₃, 35 mM NaHCO₃, pH 9.6).

Assay: The wells of a Nunc MaxiSorb microtiter plate (Nalgene Nunc International, Rochester, NY) are coated with: 100 µl of formalin-fixed *S. aureus* DSM20233 (OD₅₅₀ = 0.5) in coating buffer with 1 ug of L-ficolin in coating buffer. After overnight incubation, wells are blocked with 0.1% human serum albumin (HSA) in TBS (10 mM Tris-HCl, 140 mM NaCl, pH 7.4), then are washed with TBS containing 0.05% Tween 20 and 5 mM CaCl₂ (wash buffer). Human serum samples are diluted in 20 mM Tris-HCl, 1 M NaCl, 10 mM CaCl₂, 0.05% Triton X-100, 0.1% HSA, pH 7.4, which prevents activation of endogenous C4 and dissociates the C1 complex (composed of C1q, C1r and C1s). MASP-2 inhibitory agents, including anti-MASP-2 MoAbs and inhibitory peptides are added to the serum samples in varying concentrations. The diluted samples are added to the plate and incubated overnight at 4°C. After 24 hours, the plates are washed thoroughly with wash buffer, then 0.1 µg of purified human C4 (obtained as described in Dodds, A.W., Methods Enzymol. 223:46, 1993) in 100 µl of 4 mM barbital, 145 mM NaCl, 2 mM CaCl₂, 1 mM MgCl₂, pH 7.4 is added to each well. After 1.5 h at 37°C, the plates are washed again and C4b deposition is detected using alkaline phosphatase-conjugated chicken anti-human C4c (obtained from Immunsystem, Uppsala, Sweden) and measured using the colorimetric substrate p-nitrophenyl phosphate.

C4 Assay on mannan: The assay described above is adapted to measure lectin pathway activation via MBL by coating the plate with LSP and mannan prior to adding serum mixed with various MASP-2 inhibitory agents.

C4 assay on H-ficolin (Hakata Ag): The assay described above is adapted to measure lectin pathway activation via H-ficolin by coating the plate with LPS and H-ficolin prior to adding serum mixed with various MASP-2 inhibitory agents.

### EXAMPLE 12

The following assay demonstrates the presence of classical pathway activation in wild-type and MASP-2-/- mice.

Methods: Immune complexes were generated *in situ* by coating microtiter plates (Maxisorb, Nunc, cat. No. 442404, Fisher Scientific) with 0.1% human serum albumin in 10mM Tris, 140mM NaCl, pH 7.4 for 1 hours at room temperature followed by overnight incubation at 4°C with sheep anti whole serum antiserum (Scottish Antibody Production Unit, Carluke, Scottland) diluted 1:1000 in TBS/tween/Ca²⁺. Serum samples were obtained from wild-type and MASP-2-/- mice and added to the coated plates. Control samples were prepared in which C1q was depleted from wild-type and MASP-2-/- serum samples. C1q-depleted mouse serum was prepared using protein-A-coupled Dynabeads (Dynal Biotech, Oslo, Norway) coated with rabbit anti-human C1q IgG (Dako, Glostrup, Denmark), according to the supplier's instructions. The plates were incubated for 90 minutes at 37°C. Bound C3b was detected with a polyclonal anti-human-C3c Antibody (Dako A 062) diluted in TBS/tw/ Ca⁺⁺ at 1:1000. The secondary antibody is goat anti-rabbit IgG.

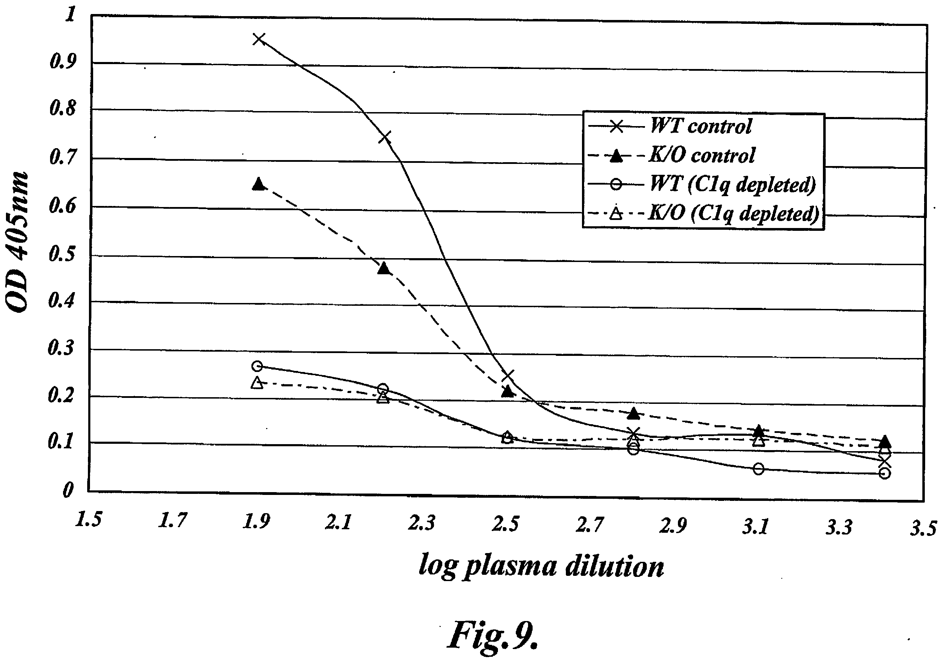

Results: FIGURE 9 shows the relative C3b deposition levels on plates coated with IgG in wild-type serum, MASP-2-/- serum, C1q-depleted wild-type and C1q-depleted MASP-2-/- serum. These results demonstrate that the classical pathway is intact in the MASP-2-/- mouse strain.

### EXAMPLE 13

The following assay is used to test whether a MASP-2 inhibitory agent blocks the classical pathway by analyzing the effect of a MASP-2 inhibitory agent under conditions in which the classical pathway is initiated by immune complexes.

Methods: To test the effect of a MASP-2 inhibitory agent on conditions of complement activation where the classical pathway is initiated by immune complexes, triplicate 50 µl samples containing 90% NHS are incubated at 37°C in the presence of 10 µg/ml immune complex (IC) or PBS, and parallel triplicate samples (+/-IC) are also included which contain 200 nM anti-properdin monoclonal antibody during the 37°C incubation. After a two hour incubation at 37°C, 13 mM EDTA is added to all samples to stop further complement activation and the samples are immediately cooled to 5°C. The samples are then stored at -70°C prior to being assayed for complement activation products (C3a and sC5b-9) using ELISA kits (Quidel, Catalog Nos. A015 and A009) following the manufacturer's instructions.

### Reference EXAMPLE 14

This example demonstrates that the lectin-dependent MASP-2 complement activation system is activated in the ischemia/reperfusion phase following abdominal aortic aneurysm repair.

Experimental Rationale and Design: Patients undergoing abdominal aortic aneurysm (AAA) repair are subject to an ischemia-reperfusion injury, which is largely mediated by complement activation. We investigated the role of the MASP-2-dependent lectin pathway of complement activation in ischemia-reperfusion injury in patients undergoing AAA repair. The consumption of mannan-binding lectin (MBL) in serum was used to measure the amount of MASP-2-dependent lectin pathway activation that occurred during reperfusion.

Patient Serum Sample Isolation: A total of 23 patients undergoing elective infrarenal AAA repair and 8 control patients undergoing major abdominal surgery were included in this study.

For the patients under going AAA repair, systemic blood samples were taken from each patient's radial artery (via an arterial line) at four defined time points during the procedure: time point 1: induction of anaesthesia; time point 2: just prior to aortic clamping; time point 3: just prior to aortic clamp removal; and time point 4: during reperfusion.

For the control patients undergoing major abdominal surgery, systemic blood samples were taken at induction of anaesthesia and at two hours after the start of the procedure.

Assay for levels of MBL: Each patient plasma sample was assayed for levels of mannan-binding lectin (MBL) using ELISA techniques.

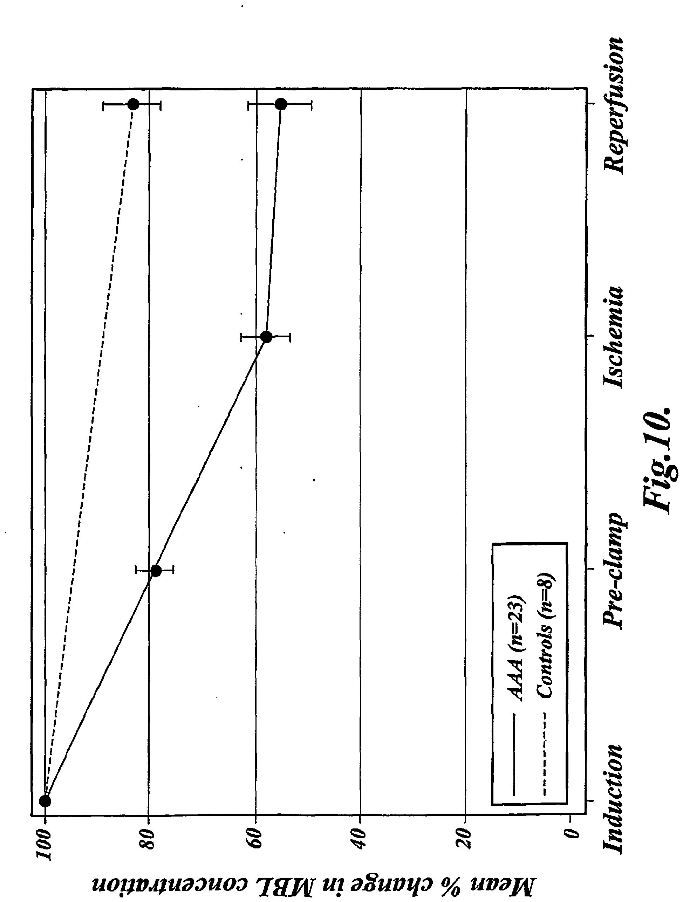

Results: The results of this study are shown in FIGURE 10, which presents a graph showing the mean percentage change in MBL levels (y axis) at each of the various time points (x axis). Starting values for MBL are 100%, with relative decreases shown thereafter. As shown in FIGURE 10, AAA patients (n=23) show a significant decrease in plasma MBL levels, averaging an approximate 41% decrease at time of ischemia/reperfusion following AAA. In contrast, in control patients (n=8) undergoing major abdominal surgery only a minor consumption of MBL was observed in the plasma samples.

The data presented provides a strong indication that the MASP-2-dependent lectin pathway of the complement system is activated in the ischemia/reperfusion phase following AAA repair. The decrease in MBL levels appears to be associated with ischaemia-reperfusion injury because the MBL levels drop significantly and rapidly when the clamped major vessel is reperfused after the end of the operation. In contrast, control sera of patients undergoing major abdominal surgery without a major ischemia-reperfusion insult only show a slight decrease in MBL plasma levels. In view of the well-established contribution of complement activation in reperfusion injury, we conclude that activation of the MASP-2-dependent lectin pathway on ischemic endothelial cells is a major factor in the pathology of ischemia/reperfusion injury. Therefore, a specific transient blockade or reduction in the MASP-2-dependent lectin pathway of complement activation would be expected to have a significant beneficial therapeutic impact to improve the outcome of clinical procedures and diseases that involve a transient ischemic insult, e.g., myocardial infarction, gut infarction, bums, transplantation and stroke.

### Reference EXAMPLE 15

This example describes the use of the MASP-2-/- strain as an animal model for testing MASP-2 inhibitory agents useful to treat Rheumatoid Arthritis.

Background and Rationale: Murine Arthritis Model: K/BxN T cell receptor (TCR) transgenic (tg) mice, is a recently developed model of inflammatory arthritis (Kouskoff, V., et al., Cell 87:811-822, 1996; Korganow, A.S., et al., Immunity 10:451-461, 1999; Matsumoto, I., et al., Science 286:1732-1735, 1999; Maccioni M. et al., J. Exp. Med. 195(8):1071-1077, 2002). The K/BxN mice spontaneously develop an autoimmune disease with most of the clinical, histological and immunological features of RA in humans (Ji, H., et al., Immunity 16:157-168, 2002). The murine disorder is joint specific, but is initiated then perpetuated by T, then B cell autoreactivity to glucose-6-phosphate isomerase ("GPI"), a ubiquitously expressed antigen. Further, transfer of serum (or purified anti-GPI Igs) from arthritic K/BxN mice into healthy animals provokes arthritis within several days. It has also been shown that polyclonal anti-GPI antibodies or a pool of anti-GPI monoclonal antibodies of the IgG1 isotype induce arthritis when injected into healthy recipients (Maccioni et al., 2002). The murine model is relevant to human RA, because serum from RA patients has also been found to contain anti-GPI antibodies, which is not found in normal individuals. A C5-deficient mouse was tested in this system and found to block the development of arthritis (Ji, H., et al., 2002, *supra*). There was also strong inhibition of arthritis in C3 null mice, implicating the alternative pathway, however, MBP-A null mice did develop arthritis. In mice however, the presence of MBP-C may compensate for the loss of MBP-A.

Based on the observations described herein that MASP-2 plays an essential role in the initiation of both the lectin and alternative pathways, the K/BxN arthritic model is useful to screen for MASP-2 inhibitory agents that are effective for use as a therapeutic agents to treat RA.

Methods: Serum from arthritic K/BxN mice is obtained at 60 days of age, pooled and injected (150-200 µl i.p.) into MASP-2-/- recipients (obtained as described in Example 1); and control littermates with or without MASP-2 inhibitory agents (MoAb, inhibitory peptides and the like as described herein) at days 0 and 2. A group of normal mice are also pretreated with a MASP-2 inhibitory agent for two days prior to receiving the injection of serum. A further group of mice receive an injection of serum at day 0, followed by a MASP-2 inhibitory agent at day 6. A clinical index is evaluated over time with one point scored for each affected paw, ½ point scored for a paw with only mild swelling. Ankle thickness is also measured by a caliper (thickness is defined as the difference from day 0 measurement).

### Reference EXAMPLE 16

This example describes an assay for inhibition of complement-mediated tissue damage in an *ex vivo* model of rabbit hearts perfused with human plasma.

Background and Rationale: Activation of the complement system contributes to hyperacute rejection of xenografts. Previous studies have shown that hyperacute rejection can occur in the absence of anti-donor antibodies via activation of the alternative pathway (Johnston, P.S., et al., Transplant Proc. 23:877-879, 1991).

Methods: To determine whether isolated anti-MASP-2 inhibitory agents such as anti-MASP-2 antibodies obtained as described in Example 7 are able to inhibit complement pathway in tissue damage, the anti-MASP-2 MoAbs and antibody fragments may be tested using an ex vivo model in which isolated rabbit hearts are perfused with diluted human plasma. This model was previously shown to cause damage to the rabbit myocardium due to the activation of the alternative complement pathway (Gralinski, M.R., et al., Immunopharmacology 34:79-88, 1996).

### Reference EXAMPLE 17

This example describes an assay that measures neutrophil activation which is useful as a measure of an effective dose of a MASP-2 inhibitory agent for the treatment of conditions associated with the lectin-dependent pathway in accordance with the methods of the invention.

Methods: A method for measuring neutrophil elastase has been described in Gupta-Bansal, R., et al., Molecular Immunol. 37:191-201, 2000. Briefly, the complex of elastase and serum α1-antitrypsin is measured with a two-site sandwich assay that utilizes antibodies against both elastase and α₁-antitrypsin. Polystyrene microtiter plates are coated with a 1:500 dilution of anti-human elastase antibody (The Binding Site, Birmingham, UK) in PBS overnight at 4°C. After aspirating the antibody solution, wells are blocked with PBS containing 0.4% HAS for 2 h at room temperature. Aliquots (100 µl) of plasma samples that are treated with or without a MASP-2 inhibitory agent are added to the wells. Following a 2 h incubation at room temperature, the wells are extensively rinsed with PBS. Bound elastase-α₁-antitrypsin complex is detected by the addition of a 1:500 dilution of peroxidase conjugated-α₁-antitrypsin antibody in blocking solution that is allowed to incubate for 1 h at room temperature. After washing the plate with PBS, 100 µl aliquots of TMB substrate are added. The reaction of TMB is quenched by the addition of 100 µl of phosphoric acid, and the plate is read at 450 nm in a microplate reader.

### Reference EXAMPLE 18

This example describes an animal model for testing MASP-2 inhibitory agents useful to treat myocardial ischemia/reperfusion.

Methods: A myocardial ischemia-reperfusion model has been described by Vakeva et al., Circulation 97:2259-2267, 1998, and Jordan et al., Circulation 104(12):1413-1418, 2001. The described model may be modified for use in MASP-2-/- and MASP-2+/+ mice as follows. Briefly, adult male mice are anesthetized. Jugular vein and trachea are cannulated and ventilation is maintained with 100% oxygen with a rodent ventilator adjusted to maintain exhaled CO₂ between 3.5% and 5%. A left thoracotomy is performed and a suture is placed 3 to 4 mm from the origin of the left coronary artery. Five minutes before ischemia, animals are given a MASP-2 inhibitory agent, such as anti-MASP-2 antibodies (e.g., in a dosage range of between .01 to 10 mg/kg). Ischemia is then initiated by tightening the suture around the coronary artery and maintained for 30 minutes, followed by four hours of reperfusion. Sham-operated animals are prepared identically without tightening the suture.

Analysis of Complement C3 Deposition: After reperfusion, samples for immunohistochemistry are obtained from the central region of the left ventricle, fixed and frozen at -80°C until processed. Tissue sections are incubated with an HRP-conjugated goat anti-rat C3 antibody. Tissue sections are analyzed for the presence of C3 staining in the presence of anti-MASP-2 inhibitory agents as compared with sham-operated control animals and MASP-2-/- animals to identify MASP-2 inhibitory agents that reduce C3 deposition *in vivo.*

### Reference EXAMPLE 19

This example describes the use of the MASP-2-/- strain as an animal model for testing MASP-2 inhibitory agents for the ability to protect transplanted tissue from ischemia/reperfusion injury.

Background/Rationale: It is known that ischemia/reperfusion injury occurs in a donor organ during transplantation. The extent of tissue damage is related to the length of ischemia and is mediated by complement, as demonstrated in various models of ischemia and through the use of complement inhibiting agents such as soluble receptor type 1 (CR1) (Weisman et al., Science 249:146-151, 1990; Mulligan et al., J. Immunol. 148:1479-1486, 1992; Pratt et al., Am. J. Path. 163(4):1457-1465, 2003). An animal model for transplantation has been described by Pratt et al., Am. J. Path. 163(4):1457-1465, which may be modified for use with the MASP-2-/- mouse model and/or for use as a MASP-2+/+ model system in which to screen MASP-2 inhibitory agents for the ability to protect transplanted tissue from ischemia/reperfusion injury. The flushing of the donor kidney with perfusion fluid prior to transplantation provides an opportunity to introduce anti-MASP-2 inhibitory agents into the donor kidney.

Methods: MASP-2-/- and/or MASP-2+/+ mice are anesthetized. The left donor kidney is dissected and the aorta is ligated cephalad and caudad to the renal artery. A portex tube catheter (Portex Ltd, Hythe, UK) is inserted between the ligatures and the kidney is perfused with 5 ml of Soltran Kidney Perfusion Solution (Baxter Health Care, UK) containing MASP-2 inhibitory agents such as anti-MASP-2 monoclonal antibodies (in a dosage range of from .01 mg/kg to 10 mg/kg) for a period of at least 5 minutes. Renal transplantation is then performed and the mice are monitored over time.

Analysis of Transplant Recipients: Kidney transplants are harvested at various time intervals and tissue sections are analyzed using anti-C3 to determine the extent of C3 deposition.

### Reference EXAMPLE 20

This example describes the use of a collagen-induced arthritis (CIA) animal model for testing MASP-2 inhibitory agents useful to treat rheumatoid arthritis (RA).

Background and Rationale: Collagen-induced arthritis (CIA) represents an autoimmune polyarthritis inducible in susceptible strains of rodents and primates after immunization with native type II collagen and is recognized as a relevant model for human rheumatoid arthritis (RA) (see Courtney et al., Nature 283: 666 (1980); Trenthan et al., J. Exp. Med. 146: 857 (1977)). Both RA and CIA are characterized by joint inflammation, pannus formation and cartilage and bone erosion. The CIA susceptible murine strain DBA/1LacJ is a developed model of CIA in which mice develop clinically severe arthritis after immunization with Bovine type II collagen (Wang et al., J. Immunol. 164: 4340-4347 (2000). A C5-deficient mouse strain was crossed with DBA/1LacJ and the resulting strain was found to be resistant to the development of CIA arthritis (Wang et al., 2000, *supra*).

Based on the observations described herein that MASP-2 plays an essential role in the initiation of both the lectin and alternative pathways, the CIA arthritic model is useful to screen for MASP-2 inhibitory agents that are effective for use as therapeutic agents to treat RA.

Methods: A MASP-2-/- mouse is generated as described in Example 1. The MASP-2-/- mouse is then crossed with a mouse derived from the DBA/1LacJ strain (The Jackson Laboratory). F1 and subsequent offspring are intercrossed to produce homozygous MASP-2-/- in the DBA/1LacJ line.

Collagen immunization is carried out as described in Wang et al., 2000, *supra.* Briefly, wild-type DBA/1LacJ mice and MASP-2-/- DBA/1LacJ mice are immunized with Bovine type II collagen (BCII) or mouse type II collagen (MCII) (obtained from Elastin Products, Owensville, MO), dissolved in 0.01 M acetic acid at a concentration of 4mg/ml. Each mouse is injected intradermally at the base of the tail with 200ug CII and 100ug mycobacteria. Mice are re-immunized after 21 days and are examined daily for the appearance of arthritis. An arthritic index is evaluated over time with respect to the severity of arthritis in each affected paw.

MASP-2 inhibitory agents are screened in the wild-type DBA/1LacJ CIA mice by injecting a MASP-2 inhibitory agent such as anti-MASP-2 monoclonal antibodies (in a dosage range of from .01 mg/kg to 10 mg/kg) at the time of collagen immunization, either systemically, or locally at one or more joints and an arthritic index is evaluated over time as described above. Anti-hMASP-2 monoclonal antibodies as therapeutic agents can be easily evaluated in a MASP-2-/-, hMASP-+/+ knock-in DBA/1LacJ CIA mouse model.

### Reference EXAMPLE 21

This example describes the use of a (NZB/W) F₁ animal model for testing MASP-2 inhibitory agents useful to treat immune-complex mediated glomerulonephritis.

Background and Rationale: New Zealand black x New Zealand white (NZB/W) F1 mice spontaneously develop an autoimmune syndrome with notable similarities to human immune-complex mediated glomerulonephritis. The NZB/W F1 mice invariably succumb to glomerulonephritis by 12 months of age. As discussed above, it has been demonstrated that complement activation plays a significant role in the pathogenesis of immune-complex mediated glomerulonephritis. It has been further shown that the administration of an anti-C5 MoAb in the NZB/W F1 mouse model resulted in significant amelioration of the course of glomerulonepthritis (Wang et al., Proc. Natl. Acad. Sci. 93: 8563-8568 (1996)). Based on the observations described herein that MASP-2 plays an essential role in the initiation of both the lectin and alternative pathways, the NZB/W F₁ animal model is useful to screen for MASP-2 inhibitory agents that are effective for use as therapeutic agents to treat glomerulonephritis.

Methods: A MASP-2-/- mouse is generated as described in Example 1. The MASP-2-/- mouse is then seperately crossed with a mouse derived both from the NZB and the NZW strains (The Jackson Laboratory). F1 and subsequent offspring are intercrossed to produce homozygous MASP-2-/- in both the NZB and NZW genetic backgrounds. To determine the role of MASP-2 in the pathogenesis of glomerulonephritis in this model, the development of this disease in F1 individuals resulting from crosses of either wild-type NZB x NZW mice or MASP-2-/-NZB x MASP-2-/-NZW mice are compared. At weekly intervals urine samples will be collected from the MASP-2+/+ and MASP-2-/- F 1 mice and urine protein levels monitored for the presence of anti-DNA antibodies (as described in Wang et al., 1996, supra). Histopathological analysis of the kidneys is also carried out to monitor the amount of mesangial matrix deposition and development of glomerulonephritis.

The NZB/W F1 animal model is also useful to screen for MASP-2 inhibitory agents that are effective for use as therapeutic agents to treat glomerulonephritis. At 18 weeks of age, wild-type NZB/W F1 mice are injected intraperitoneally with anti-MASP-2 inhibitory agents, such as anti-MASP-2 monoclonal antibodies (in a dosage range of from .01 mg/kg to 10 mg/kg) at a frequency of weekly or biweekly. The above-mentioned histopathological and biochemical markers of glomerulonephritis are used to evaluate disease development in the mice and to identify useful MASP-2 inhibitory agents for the treatment of this disease.

### Reference EXAMPLE 22

This example describes the use of a tubing loop as a model for testing MASP-2 inhibitory agents useful to prevent tissue damage resulting from extracorporeal circulation (ECC) such as a cardiopulmonary bypass (CPB) circuit.

Background and Rationale: As discussed above, patients undergoing ECC during CPB suffer a systemic inflammatory reaction, which is partly caused by exposure of blood to the artificial surfaces of the extracorporeal circuit, but also by surface-independent factors like surgical trauma and ischemia-reperfusion injury (Butler, J., et al., Ann. Thorac. Surg. 55:552-9, 1993; Edmunds, L.H., Ann. Thorac. Surg. 66(Suppl):S12-6, 1998; Asimakopoulos, G., Perfusion 14:269-77, 1999). It has further been shown that the alternative complement pathway plays a predominant role in complement activation in CPB circuits, resulting from the interaction of blood with the artificial surfaces of the CPB circuits (see Kirklin et al., 1983, 1986, discussed *supra*). Therefore, based on the observations described herein that MASP-2 plays an essential role in the initiation of both the lectin and alternative pathways, the tubing loop model is useful to screen for MASP-2 inhibitory agents that are effective for use as therapeutic agents to prevent or treat an extracorporeal exposure-triggered inflammatory reaction.

Methods: A modification of a previously described tubing loop model for cardiopulmonary bypass circuits is utilized (see Gong et al., J. Clinical Immunol. 16(4):222-229 (1996)) as described in Gupta-Bansal et al., Molecular Immunol. 37:191-201 (2000). Briefly, blood is freshly collected from a healthy subject in a 7ml vacutainer tube (containing 7 units of heparin per ml of whole blood). Polyethylene tubing similar to what is used during CPB procedures (e.g., I.D. 2.92 mm; O.D. 3.73 mm, length: 45 cm) is filled with 1 ml of blood and closed into a loop with a short piece of silicone tubing. A control tubing containing heparinized blood with 10 mM EDTA was included in the study as a background control. Sample and control tubings were rotated vertically in a water bath for 1 hour at 37°C. After incubation, the blood samples were transferred into 1.7ml microfuge tubes containing EDTA, resulting in a final concentration of 20mM EDTA. The samples were centrifuged and the plasma was collected. MASP-2 inhibitory agents, such as anti-MASP-2 antibodies are added to the heparinized blood immediately before rotation. The plasma samples are then subjected to assays to measure the concentration C3a and soluble C5b-9 as described in Gupta-Bansal et al., 2000, *supra.*

### Reference EXAMPLE 23

This example describes the use of a rodent caecal ligation and puncture (CLP) model system for testing MASP-2 inhibitory agents useful to treat sepsis or a condition resulting from sepsis, including severe sepsis, septic shock, acute respiratory distress syndrome resulting from sepsis and systemic inflammatory response syndrome.

Background and Rationale: As discussed above, complement activation has been shown in numerous studies to have a major role in the pathogenesis of sepsis (see Bone, R.C., Annals. Internal. Med. 115:457-469, 1991). The CLP rodent model is a recognized model that mimics the clinical course of sepsis in humans and is considered to be a reasonable surrogate model for sepsis in humans (see Ward, P., Nature Review Immunology Vol 4: 133-142 (2004). A recent study has shown that treatment of CLP animals with anti-C5a antibodies resulted in reduced bacteremia and greatly improved survival Huber-Lang et al., J. of Immunol. 169: 3223-3231 (2002). Therefore, based on the observations described herein that MASP-2 plays an essential role in the initiation of both the lectin and alternative pathways, the CLP rodent model is useful to screen for MASP-2 inhibitory agents that are effective for use as therapeutic agents to prevent or treat sepsis or a condition resulting from sepsis.

Methods: The CLP model is adapted from the model described in Huber-Lang et al., 2004, *supra* as follows. MASP-2-/- and MASP-2+/+ animals are anesthetized. A 2cm midline abdominal incision is made and the cecum is tightly ligated below the ileocecal valve, avoiding bowel obstruction. The cecum is then punctured through and through with a 21-gauge needle. The abdominal incision was then closed in layers with silk suture and skin clips (Ethicon, Summerville, NJ). Immediately after CLP, animals receive an injection of a MASP-2 inhibitory agent such as anti-MASP-2 monoclonal antibodies (in a dosage range of from .01 mg/kg to 10 mg/kg). Anti-hMASP-2 monoclonal antibodies as therapeutic agents can be easily evaluated in a MASP-2-/-, hMASP-+/+ knock-in CLP mouse model. The plasma of the mice are then analyzed for levels of complement-derived anaphylatoxins and respiratory burst using the assays described in Huber-Lang et al., 2004, *supra.*

## Claims

1. A MASP-2 inhibitory anti-MASP-2 antibody or fragment thereof that specifically binds to a portion of SEQ ID NO:6 and selectively inhibits MASP-2-dependent complement activation without substantially inhibiting C1q-dependent complement activation for use in the treatment of a subject suffering from diabetic angiopathy.

2. A MASP-2 inhibitory antibody or fragment for use as claimed in claim 1, wherein the MASP-2 inhibitory antibody specifically binds to a polypeptide comprising SEQ ID NO:6.

3. A MASP-2 inhibitory antibody or fragment for use as claimed in any preceding claim, wherein the MASP-2 inhibitory antibody specifically binds to a polypeptide comprising SEQ ID NO: 6 with an affinity of at least 10 times greater than it binds to a different antigen in the complement system.

4. A MASP-2 inhibitory antibody or fragment for use as claimed in any preceding claim, wherein the MASP-2 inhibitory antibody binds to the the polypeptide at a location within amino acid residues 1-176 of SEQ ID NO:6.

5. A MASP-2 inhibitory antibody or fragment for use as claimed in Claim 1, wherein the anti-MASP-2 antibody or fragment thereof is monoclonal.

6. A MASP-2 inhibitory antibody or fragment for use as claimed in any preceding claim, wherein the anti-MASP-2 antibody or fragment thereof is recombinant.

7. A MASP-2 inhibitory antibody or fragment for use as claimed in any preceding claim, wherein the anti-MASP-2 antibody or fragment thereof has reduced effector function.

8. A MASP-2 inhibitory antibody or fragment for use as claimed in any preceding claim, wherein the anti-MASP-2 antibody or fragment thereof is chimeric, humanized or human.

9. A MASP-2 inhibitory antibody or fragment for use as claimed in any preceding claim, wherein the anti-MASP-2 antibody or fragment thereof is produced in a MASP-2 deficient transgenic animal.

10. A pharmaceutical composition comprising a MASP-2 inhibitory anti-MASP-2 antibody or fragment thereof that specifically binds to a portion of SEQ ID NO:6 and selectively inhibits MASP-2-dependent complement activation without substantially inhibiting C1q-dependent complement activation and a pharmaceutically acceptable carrier for use in the treatment of diabetic angiopathy.

11. A pharmaceutical composition for use as claimed in claim 10, wherein the MASP-2 inhibitory antibody is as set forth in any of claims 2 to 9.

12. A pharmaceutical composition for use as claimed in any of claims 10 to 11, wherein the composition is formulated for intra-arterial, intravenous, intracranial, intramuscular, subcutaneous administration or administration as an inhalant.

13. A pharmaceutical composition for use as claimed in any of claims 10 to 12, wherein the composition is formulated for subcutaneous administration.

## Patentansprüche

1. MASP-2-hemmender Anti-MASP-2-Antikörper oder Fragment davon, der/das spezifisch an einen Abschnitt von SEQ ID NO:6 bindet und selektiv MASP-2-abhängige Komplementaktivierung hemmt, ohne im Wesentlichen C1q-abhängige Komplementaktivierung zu hemmen, zur Verwendung beim Behandeln eines Subjekts, das an diabetischer Angiopathie leidet.

2. MASP-2-hemmender Antikörper oder Fragment zur Verwendung nach Anspruch 1, wobei der MASP-2-hemmende Antikörper spezifisch an ein Polypeptid, das SEQ ID NO:6 umfasst, bindet.

3. MASP-2-hemmender Antikörper oder Fragment zur Verwendung nach einem der vorhergehenden Ansprüche, wobei der MASP-2-hemmende Antikörper mit einer wenigstens 10-mal größeren Affinität spezifisch an ein Polypeptid, das SEQ ID NO: 6 umfasst, als an ein anderes Antigen in dem Komplementsystem bindet.

4. MASP-2-hemmender Antikörper oder Fragment zur Verwendung nach einem der vorhergehenden Ansprüche, wobei der MASP-2-hemmende Antikörper an einer Stelle in Aminosäureresten 1-176 von SEQ ID NO:6 an das Polypeptid bindet.

5. MASP-2-hemmender Antikörper oder Fragment zur Verwendung nach Anspruch 1, wobei der Anti-MASP-2-Antikörper oder das Fragment davon monoklonal ist.

6. MASP-2-hemmender Antikörper oder Fragment zur Verwendung nach einem der vorhergehenden Ansprüche, wobei der Anti-MASP-2-Antikörper oder das Fragment davon rekombinant ist.

7. MASP-2-hemmender Antikörper oder Fragment zur Verwendung nach einem der vorhergehenden Ansprüche, wobei der Anti-MASP-2-Antikörper oder das Fragment davon eine reduzierte Effektorfunktion aufweist.

8. MASP-2-hemmender Antikörper oder Fragment zur Verwendung nach einem der vorhergehenden Ansprüche, wobei der Anti-MASP-2-Antikörper oder das Fragment davon chimär, humanisiert oder human ist.

9. MASP-2-hemmender Antikörper oder Fragment zur Verwendung nach einem der vorhergehenden Ansprüche, wobei der Anti-MASP-2-Antikörper oder das Fragment davon in einem MASP-2-defizienten transgenen Tier produziert worden ist.

10. Pharmazeutische Zusammensetzung, umfassend einen MASP-2-hemmenden Anti-MASP-2-Antikörper oder ein Fragment davon, der/das spezifisch an einen Abschnitt von SEQ ID NO:6 bindet und selektiv MASP-2-abhängige Komplementaktivierung hemmt, ohne im Wesentlichen C1q-abhängige Komplementaktivierung zu hemmen, und einen pharmazeutisch unbedenklichen Träger zur Verwendung beim Behandeln von diabetischer Angiopathie.

11. Pharmazeutische Zusammensetzung zur Verwendung nach Anspruch 10, wobei der MASP-2-hemmende Antikörper nach einem der Ansprüche 2 bis 9 dargelegt ist.

12. Pharmazeutische Zusammensetzung zur Verwendung nach einem der Ansprüche 10 bis 11, wobei die Zusammensetzung zum intraarteriellen, intravenösen, intrakraniellen, intramuskulären, subkutanen Verabreichen oder zum Verabreichen als ein Inhalationsmittel formuliert ist.

13. Pharmazeutische Zusammensetzung zur Verwendung nach einem der Ansprüche 10 bis 12, wobei die Zusammensetzung zum subkutanen Verabreichen formuliert ist.

## Revendications

1. Anticorps anti-MASP-2 d'inhibition de MASP-2 ou fragment de celui-ci qui se lie spécifiquement à une partie de SEQ ID n° : 6 et inhibe sélectivement l'activation du complément dépendante de MASP-2 sans sensiblement inhiber l'activation du complément dépendante de C1q pour une utilisation dans le traitement d'un patient souffrant d'angiopathie diabétique.

2. Anticorps d'inhibition de MASP-2 ou fragment de celui-ci pour une utilisation selon la revendication 1, lequel anticorps d'inhibition de MASP-2 se lie spécifiquement à un polypeptide comprenant SEQ ID n° : 6.

3. Anticorps d'inhibition de MASP-2 ou fragment de celui-ci pour une utilisation selon l'une quelconque des revendications précédentes, lequel anticorps d'inhibition de MASP-2 se lie spécifiquement à un polypeptide comprenant SEQ ID n° : 6 avec une affinité au moins 10 fois plus élevée qu'il ne se lie à un antigène différent dans le système complémentaire.

4. Anticorps d'inhibition de MASP-2 ou fragment de celui-ci pour une utilisation selon l'une quelconque des revendications précédentes, lequel anticorps d'inhibition de MASP-2 se lie au polypeptide au niveau d'un emplacement dans les résidus d'acides aminés 1 à 176 de SEQ ID n° : 6.

5. Anticorps d'inhibition de MASP-2 ou fragment de celui-ci pour une utilisation selon la revendication 1, lequel anticorps anti-MASP-2 ou fragment de celui-ci est monoclonal.

6. Anticorps d'inhibition de MASP-2 ou fragment de celui-ci pour une utilisation selon l'une quelconque des revendications précédentes, lequel anticorps anti-MASP-2 ou fragment de celui-ci est recombinant.

7. Anticorps d'inhibition de MASP-2 ou fragment de celui-ci pour une utilisation selon l'une quelconque des revendications précédentes, lequel anticorps anti-MASP-2 ou fragment de celui-ci a une fonction effectrice réduite.

8. Anticorps d'inhibition de MASP-2 ou fragment de celui-ci pour une utilisation selon l'une quelconque des revendications précédentes, lequel anticorps anti-MASP-2 ou fragment de celui-ci est chimérique, humanisé ou humain.

9. Anticorps d'inhibition de MASP-2 ou fragment de celui-ci pour une utilisation selon l'une quelconque des revendications précédentes, lequel anticorps anti-MASP-2 ou fragment de celui-ci est produit chez un animal transgénique déficient en MASP-2.

10. Composition pharmaceutique comprenant un anticorps anti-MASP-2 d'inhibition de MASP-2 ou un fragment de celui-ci qui se lie spécifiquement à une partie de SEQ ID n° : 6 et inhibe sélectivement l'activation du complément dépendante de MASP-2 sans sensiblement inhiber l'activation du complément dépendante de C1q et un vecteur pharmaceutiquement acceptable pour une utilisation dans le traitement de l'angiopathie diabétique.

11. Composition pharmaceutique pour une utilisation selon la revendication 10, dans laquelle l'anticorps d'inhibition de MASP-2 est tel que décrit dans l'une quelconque des revendications 2 à 9.

12. Composition pharmaceutique pour une utilisation selon l'une quelconque des revendications 10 à 11, laquelle composition est formulée pour une administration par voie intra-artérielle, intraveineuse, intracrânienne, intramusculaire, sous-cutanée ou une administration sous forme de présentation pour inhalation.

13. Composition pharmaceutique pour une utilisation selon l'une quelconque des revendications 10 à 12, laquelle composition est formulée pour une administration par voie sous-cutanée.


### SEQUENCE LISTING

<110> Omeros Corporation University of Leicester
<120> METHODS FOR TREATING CONDITIONS ASSOCIATED WITH MASP-2 DEPENDENT COMPLEMENT ACTIVATION
<130> P101240EP60
<150> US 60/578,847
   <151> 2004-06-10
<160> 65
<170> PatentIn version 3.2
<210> 1
   <211> 725
   <212> DNA
   <213> Homo sapien
<220>
   <221> CDS
   <222> (27)..(584)
<400> 1
   ggccaggcca gctggacggg cacacc atg agg ctg ctg acc ctc ctg ggc ctt      53
                                Met Arg Leu Leu Thr Leu Leu Gly Leu
                                1               5
   ctg tgt ggc tcg gtg gcc acc ccc ttg ggc ccg aag tgg cct gaa cct      101
   Leu Cys Gly Ser Val Ala Thr Pro Leu Gly Pro Lys Trp Pro Glu Pro
   10                  15                  20                  25
   gtg ttc ggg cgc ctg gca tcc ccc ggc ttt cca ggg gag tat gcc aat      149
   Val Phe Gly Arg Leu Ala Ser Pro Gly Phe Pro Gly Glu Tyr Ala Asn
                   30                  35                  40
   gac cag gag cgg cgc tgg acc ctg act gca ccc ccc ggc tac cgc ctg      197
   Asp Gln Glu Arg Arg Trp Thr Leu Thr Ala Pro Pro Gly Tyr Arg Leu
               45                  50                  55
   cgc ctc tac ttc acc cac ttc gac ctg gag ctc tcc cac ctc tgc gag      245
   Arg Leu Tyr Phe Thr His Phe Asp Leu Glu Leu Ser His Leu Cys Glu
           60                  65                  70
   tac gac ttc gtc aag ctg agc tcg ggg gcc aag gtg ctg gcc acg ctg      293
   Tyr Asp Phe Val Lys Leu Ser Ser Gly Ala Lys Val Leu Ala Thr Leu
       75                  80                  85
   tgc ggg cag gag agc aca gac acg gag cgg gcc cct ggc aag gac act      341
   Cys Gly Gln Glu Ser Thr Asp Thr Glu Arg Ala Pro Gly Lys Asp Thr
   90                  95                  100                 105
   ttc tac tcg ctg ggc tcc agc ctg gac att acc ttc cgc tcc gac tac      389
   Phe Tyr Ser Leu Gly Ser Ser Leu Asp Ile Thr Phe Arg Ser Asp Tyr
                   110                 115                 120
   tcc aac gag aag ccg ttc acg ggg ttc gag gcc ttc tat gca gcc gag      437
   Ser Asn Glu Lys Pro Phe Thr Gly Phe Glu Ala Phe Tyr Ala Ala Glu
               125                 130                 135
   gac att gac gag tgc cag gtg gcc ccg gga gag gcg ccc acc tgc gac      485
   Asp Ile Asp Glu Cys Gln Val Ala Pro Gly Glu Ala Pro Thr Cys Asp
           140                 145                 150
   cac cac tgc cac aac cac ctg ggc ggt ttc tac tgc tcc tgc cgc gca      533
   His His Cys His Asn His Leu Gly Gly Phe Tyr Cys Ser Cys Arg Ala
       155                 160                 165
   ggc tac gtc ctg cac cgt aac aag cgc acc tgc tca gag cag agc ctc      581
   Gly Tyr Val Leu His Arg Asn Lys Arg Thr Cys Ser Glu Gln Ser Leu
   170                 175                 180                 185
   tag cctcccctgg agctccggcc tgcccagcag gtcagaagcc agagccagcc          634
   tgctggcctc agctccgggt tgggctgaga tggctgtgcc ccaactccca ttcacccacc    694
   atggacccaa taataaacct ggccccaccc c                                  725
<210> 2
   <211> 185
   <212> PRT
   <213> Homo sapien
<400> 2
   Met Arg Leu Leu Thr Leu Leu Gly Leu Leu Cys Gly Ser Val Ala Thr
   1               5                   10                  15
   Pro Leu Gly Pro Lys Trp Pro Glu Pro Val Phe Gly Arg Leu Ala Ser
               20                  25                  30
   Pro Gly Phe Pro Gly Glu Tyr Ala Asn Asp Gln Glu Arg Arg Trp Thr
           35                  40                  45
   Leu Thr Ala Pro Pro Gly Tyr Arg Leu Arg Leu Tyr Phe Thr His Phe
       50                  55                  60
   Asp Leu Glu Leu Ser His Leu Cys Glu Tyr Asp Phe Val Lys Leu Ser
   65                  70                  75                  80
   Ser Gly Ala Lys Val Leu Ala Thr Leu Cys Gly Gln Glu Ser Thr Asp
                   85                  90                  95
   Thr Glu Arg Ala Pro Gly Lys Asp Thr Phe Tyr Ser Leu Gly Ser Ser
               100                 105                 110
   Leu Asp Ile Thr Phe Arg Ser Asp Tyr Ser Asn Glu Lys Pro Phe Thr
           115                 120                 125
   Gly Phe Glu Ala Phe Tyr Ala Ala Glu Asp Ile Asp Glu Cys Gln Val
       130                 135                 140
   Ala Pro Gly Glu Ala Pro Thr Cys Asp His His Cys His Asn His Leu
   145                 150                 155                 160
   Gly Gly Phe Tyr Cys Ser Cys Arg Ala Gly Tyr Val Leu His Arg Asn
                   165                 170                 175
   Lys Arg Thr Cys Ser Glu Gln Ser Leu
               180                 185
<210> 3
   <211> 170
   <212> PRT
   <213> Homo sapien
<400> 3
   Thr Pro Leu Gly Pro Lys Trp Pro Glu Pro Val Phe Gly Arg Leu Ala
   1               5                   10                  15
   Ser Pro Gly Phe Pro Gly Glu Tyr Ala Asn Asp Gln Glu Arg Arg Trp
               20                  25                  30
   Thr Leu Thr Ala Pro Pro Gly Tyr Arg Leu Arg Leu Tyr Phe Thr His
           35                  40                  45
   Phe Asp Leu Glu Leu Ser His Leu Cys Glu Tyr Asp Phe Val Lys Leu
       50                  55                  60
   Ser Ser Gly Ala Lys Val Leu Ala Thr Leu Cys Gly Gln Glu Ser Thr
   65                  70                  75                  80
   Asp Thr Glu Arg Ala Pro Gly Lys Asp Thr Phe Tyr Ser Leu Gly Ser
                   85                  90                  95
   Ser Leu Asp Ile Thr Phe Arg Ser Asp Tyr Ser Asn Glu Lys Pro Phe
               100                 105                 110
   Thr Gly Phe Glu Ala Phe Tyr Ala Ala Glu Asp Ile Asp Glu Cys Gln
           115                 120                 125
   Val Ala Pro Gly Glu Ala Pro Thr Cys Asp His His Cys His Asn His
       130                 135                 140
   Leu Gly Gly Phe Tyr Cys Ser Cys Arg Ala Gly Tyr Val Leu His Arg
   145                 150                 155                 160
   Asn Lys Arg Thr Cys Ser Glu Gln Ser Leu
                   165                 170
<210> 4
   <211> 2460
   <212> DNA
   <213> Homo sapien
<220>
   <221> CDS
   <222> (22)..(2082)
<400> 4
   ggccagctgg acgggcacac c atg agg ctg ctg acc ctc ctg ggc ctt ctg       51
                           Met Arg Leu Leu Thr Leu Leu Gly Leu Leu
                           1               5                   10
   tgt ggc tcg gtg gcc acc ccc ttg ggc ccg aag tgg cct gaa cct gtg       99
   Cys Gly Ser Val Ala Thr Pro Leu Gly Pro Lys Trp Pro Glu Pro Val
                   15                  20                  25
   ttc ggg cgc ctg gca tcc ccc ggc ttt cca ggg gag tat gcc aat gac      147
   Phe Gly Arg Leu Ala Ser Pro Gly Phe Pro Gly Glu Tyr Ala Asn Asp
               30                  35                  40
   cag gag cgg cgc tgg acc ctg act gca ccc ccc ggc tac cgc ctg cgc      195
   Gln Glu Arg Arg Trp Thr Leu Thr Ala Pro Pro Gly Tyr Arg Leu Arg
           45                  50                  55
   ctc tac ttc acc cac ttc gac ctg gag ctc tcc cac ctc tgc gag tac      243
   Leu Tyr Phe Thr His Phe Asp Leu Glu Leu Ser His Leu Cys Glu Tyr
       60                  65                  70
   gac ttc gtc aag ctg agc tcg ggg gcc aag gtg ctg gcc acg ctg tgc      291
   Asp Phe Val Lys Leu Ser Ser Gly Ala Lys Val Leu Ala Thr Leu Cys
   75                  80                  85                  90
   ggg cag gag agc aca gac acg gag cgg gcc cct ggc aag gac act ttc      339
   Gly Gln Glu Ser Thr Asp Thr Glu Arg Ala Pro Gly Lys Asp Thr Phe
                   95                  100                 105
   tac tcg ctg ggc tcc agc ctg gac att acc ttc cgc tcc gac tac tcc      387
   Tyr Ser Leu Gly Ser Ser Leu Asp Ile Thr Phe Arg Ser Asp Tyr Ser
               110                 115                 120
   aac gag aag ccg ttc acg ggg ttc gag gcc ttc tat gca gcc gag gac      435
   Asn Glu Lys Pro Phe Thr Gly Phe Glu Ala Phe Tyr Ala Ala Glu Asp
           125                 130                 135
   att gac gag tgc cag gtg gcc ccg gga gag gcg ccc acc tgc gac cac      483
   Ile Asp Glu Cys Gln Val Ala Pro Gly Glu Ala Pro Thr Cys Asp His
       140                 145                 150
   cac tgc cac aac cac ctg ggc ggt ttc tac tgc tcc tgc cgc gca ggc      531
   His Cys His Asn His Leu Gly Gly Phe Tyr Cys Ser Cys Arg Ala Gly
   155                 160                 165                 170
   tac gtc ctg cac cgt aac aag cgc acc tgc tca gcc ctg tgc tcc ggc      579
   Tyr Val Leu His Arg Asn Lys Arg Thr Cys Ser Ala Leu Cys Ser Gly
                   175                 180                 185
   cag gtc ttc acc cag agg tct ggg gag ctc agc agc cct gaa tac cca      627
   Gln Val Phe Thr Gln Arg Ser Gly Glu Leu Ser Ser Pro Glu Tyr Pro
               190                 195                 200
   cgg ccg tat ccc aaa ctc tcc agt tgc act tac agc atc agc ctg gag      675
   Arg Pro Tyr Pro Lys Leu Ser Ser Cys Thr Tyr Ser Ile Ser Leu Glu
           205                 210                 215
   gag ggg ttc agt gtc att ctg gac ttt gtg gag tcc ttc gat gtg gag      723
   Glu Gly Phe Ser Val Ile Leu Asp Phe Val Glu Ser Phe Asp Val Glu
       220                 225                 230
   aca cac cct gaa acc ctg tgt ccc tac gac ttt ctc aag att caa aca      771
   Thr His Pro Glu Thr Leu Cys Pro Tyr Asp Phe Leu Lys Ile Gln Thr
   235                 240                 245                     250
   gac aga gaa gaa cat ggc cca ttc tgt ggg aag aca ttg ccc cac agg      819
   Asp Arg Glu Glu His Gly Pro Phe Cys Gly Lys Thr Leu Pro His Arg
                   255                 260                 265
   att gaa aca aaa agc aac acg gtg acc atc acc ttt gtc aca gat gaa      867
   Ile Glu Thr Lys Ser Asn Thr Val Thr Ile Thr Phe Val Thr Asp Glu
               270                 275                 280
   tca gga gac cac aca ggc tgg aag atc cac tac acg agc aca gcg cag      915
   Ser Gly Asp His Thr Gly Trp Lys Ile His Tyr Thr Ser Thr Ala Gln
           285                 290                 295
   cct tgc cct tat ccg atg gcg cca cct aat ggc cac gtt tca cct gtg      963
   Pro Cys Pro Tyr Pro Met Ala Pro Pro Asn Gly His Val Ser Pro Val
       300                 305                 310
   caa gcc aaa tac atc ctg aaa gac agc ttc tcc atc ttt tgc gag act     1011
   Gln Ala Lys Tyr Ile Leu Lys Asp Ser Phe Ser Ile Phe Cys Glu Thr
   315                 320                 325                     330
   ggc tat gag ctt ctg caa ggt cac ttg ccc ctg aaa tcc ttt act gca     1059
   Gly Tyr Glu Leu Leu Gln Gly His Leu Pro Leu Lys Ser Phe Thr Ala
                   335                 340                 345
   gtt tgt cag aaa gat gga tct tgg gac cgg cca atg ccc gcg tgc agc     1107
   Val Cys Gln Lys Asp Gly Ser Trp Asp Arg Pro Met Pro Ala Cys Ser
               350                 355                 360
   att gtt gac tgt ggc cct cct gat gat cta ccc agt ggc cga gtg gag     1155
   Ile Val Asp Cys Gly Pro Pro Asp Asp Leu Pro Ser Gly Arg Val Glu
           365                 370                 375
   tac atc aca ggt cct gga gtg acc acc tac aaa gct gtg att cag tac     1203
   Tyr Ile Thr Gly Pro Gly Val Thr Thr Tyr Lys Ala Val Ile Gln Tyr
       380                 385                 390
   agc tgt gaa gag acc ttc tac aca atg aaa gtg aat gat ggt aaa tat     1251
   Ser Cys Glu Glu Thr Phe Tyr Thr Met Lys Val Asn Asp Gly Lys Tyr
   395                 400                 405                     410
   gtg tgt gag gct gat gga ttc tgg acg agc tcc aaa gga gaa aaa tca     1299
   Val Cys Glu Ala Asp Gly Phe Trp Thr Ser Ser Lys Gly Glu Lys Ser
                   415                 420                 425
   ctc cca gtc tgt gag cct gtt tgt gga cta tca gcc cgc aca aca gga     1347
   Leu Pro Val Cys Glu Pro Val Cys Gly Leu Ser Ala Arg Thr Thr Gly
               430                 435                 440
   ggg cgt ata tat gga ggg caa aag gca aaa cct ggt gat ttt cct tgg     1395
   Gly Arg Ile Tyr Gly Gly Gln Lys Ala Lys Pro Gly Asp Phe Pro Trp
           445                 450                 455
   caa gtc ctg ata tta ggt gga acc aca gca gca ggt gca ctt tta tat      1443
   Gln Val Leu Ile Leu Gly Gly Thr Thr Ala Ala Gly Ala Leu Leu Tyr
       460                 465                 470
   gac aac tgg gtc cta aca gct gct cat gcc gtc tat gag caa aaa cat      1491
   Asp Asn Trp Val Leu Thr Ala Ala His Ala Val Tyr Glu Gln Lys His
   475                 480                 485                 490
   gat gca tcc gcc ctg gac att cga atg ggc acc ctg aaa aga cta tca      1539
   Asp Ala Ser Ala Leu Asp Ile Arg Met Gly Thr Leu Lys Arg Leu Ser
                   495                 500                 505
   cct cat tat aca caa gcc tgg tct gaa gct gtt ttt ata cat gaa ggt      1587
   Pro His Tyr Thr Gln Ala Trp Ser Glu Ala Val Phe Ile His Glu Gly
               510                 515                 520
   tat act cat gat gct ggc ttt gac aat gac ata gca ctg att aaa ttg      1635
   Tyr Thr His Asp Ala Gly Phe Asp Asn Asp Ile Ala Leu Ile Lys Leu
           525                 530                 535
   aat aac aaa gtt gta atc aat agc aac atc acg cct att tgt ctg cca      1683
   Asn Asn Lys Val Val Ile Asn Ser Asn Ile Thr Pro Ile Cys Leu Pro
       540                 545                 550
   aga aaa gaa gct gaa tcc ttt atg agg aca gat gac att gga act gca      1731
   Arg Lys Glu Ala Glu Ser Phe Met Arg Thr Asp Asp Ile Gly Thr Ala
   555                 560                 565                 570
   tct gga tgg gga tta acc caa agg ggt ttt ctt gct aga aat cta atg      1779
   Ser Gly Trp Gly Leu Thr Gln Arg Gly Phe Leu Ala Arg Asn Leu Met
                   575                 580                 585
   tat gtc gac ata ccg att gtt gac cat caa aaa tgt act gct gca tat      1827
   Tyr Val Asp Ile Pro Ile Val Asp His Gln Lys Cys Thr Ala Ala Tyr
               590                 595                 600
   gaa aag cca ccc tat cca agg gga agt gta act gct aac atg ctt tgt      1875
   Glu Lys Pro Pro Tyr Pro Arg Gly Ser Val Thr Ala Asn Met Leu Cys
           605                 610                 615
   gct ggc tta gaa agt ggg ggc aag gac agc tgc aga ggt gac agc gga      1923
   Ala Gly Leu Glu Ser Gly Gly Lys Asp Ser Cys Arg Gly Asp Ser Gly
       620                 625                 630
   ggg gca ctg gtg ttt cta gat agt gaa aca gag agg tgg ttt gtg gga      1971
   Gly Ala Leu Val Phe Leu Asp Ser Glu Thr Glu Arg Trp Phe Val Gly
   635                 640                 645                 650
   gga ata gtg tcc tgg ggt tcc atg aat tgt ggg gaa gca ggt cag tat      2019
   Gly Ile Val Ser Trp Gly Ser Met Asn Cys Gly Glu Ala Gly Gln Tyr
                   655                 660                 665
   gga gtc tac aca aaa gtt att aac tat att ccc tgg atc gag aac ata      2067
   Gly Val Tyr Thr Lys Val Ile Asn Tyr Ile Pro Trp Ile Glu Asn Ile
               670                 675                 680
   att agt gat ttt taa cttgcgtgtc tgcagtcaag gattcttcat ttttagaaat     2122
   Ile Ser Asp Phe
           685
   gcctgtgaag accttggcag cgacgtggct cgagaagcat tcatcattac tgtggacatg    2182
   gcagttgttg ctccacccaa aaaaacagac tccaggtgag gctgctgtca tttctccact    2242
   tgccagttta attccagcct tacccattga ctcaagggga cataaaccac gagagtgaca    2302
   gtcatctttg cccacccagt gtaatgtcac tgctcaaatt acatttcatt accttaaaaa 2362
   gccagtctct tttcatactg gctgttggca tttctgtaaa ctgcctgtcc atgctctttg 2422
   tttttaaact tgttcttatt gaaaaaaaaa aaaaaaaa 2460
<210> 5
   <211> 686
   <212> PRT
   <213> Homo sapien
<400> 5
   Met Arg Leu Leu Thr Leu Leu Gly Leu Leu Cys Gly Ser Val Ala Thr
   1 5 10 15
   Pro Leu Gly Pro Lys Trp Pro Glu Pro Val Phe Gly Arg Leu Ala Ser
   20 25 30
   Pro Gly Phe Pro Gly Glu Tyr Ala Asn Asp Gln Glu Arg Arg Trp Thr
   35 40 45
   Leu Thr Ala Pro Pro Gly Tyr Arg Leu Arg Leu Tyr Phe Thr His Phe
   50 55 60
   Asp Leu Glu Leu Ser His Leu Cys Glu Tyr Asp Phe Val Lys Leu Ser
   65 70 75 80
   Ser Gly Ala Lys Val Leu Ala Thr Leu Cys Gly Gln Glu Ser Thr Asp
   85 90 95
   Thr Glu Arg Ala Pro Gly Lys Asp Thr Phe Tyr Ser Leu Gly Ser Ser
   100 105 110
   Leu Asp Ile Thr Phe Arg Ser Asp Tyr Ser Asn Glu Lys Pro Phe Thr
   115 120 125
   Gly Phe Glu Ala Phe Tyr Ala Ala Glu Asp Ile Asp Glu Cys Gln Val
   130 135 140
   Ala Pro Gly Glu Ala Pro Thr Cys Asp His His Cys His Asn His Leu
   145 150 155 160
   Gly Gly Phe Tyr Cys Ser Cys Arg Ala Gly Tyr Val Leu His Arg Asn
   165 170 175
   Lys Arg Thr Cys Ser Ala Leu Cys Ser Gly Gln Val Phe Thr Gln Arg
   180 185 190
   Ser Gly Glu Leu Ser Ser Pro Glu Tyr Pro Arg Pro Tyr Pro Lys Leu
   195 200 205
   Ser Ser Cys Thr Tyr Ser Ile Ser Leu Glu Glu Gly Phe Ser Val Ile
   210 215 220
   Leu Asp Phe Val Glu Ser Phe Asp Val Glu Thr His Pro Glu Thr Leu
   225 230 235 240
   Cys Pro Tyr Asp Phe Leu Lys Ile Gln Thr Asp Arg Glu Glu His Gly
   245 250 255
   Pro Phe Cys Gly Lys Thr Leu Pro His Arg Ile Glu Thr Lys Ser Asn
   260 265 270
   Thr Val Thr Ile Thr Phe Val Thr Asp Glu Ser Gly Asp His Thr Gly
   275 280 285
   Trp Lys Ile His Tyr Thr Ser Thr Ala Gln Pro Cys Pro Tyr Pro Met
   290 295 300
   Ala Pro Pro Asn Gly His Val Ser Pro Val Gln Ala Lys Tyr Ile Leu
   305 310 315 320
   Lys Asp Ser Phe Ser Ile Phe Cys Glu Thr Gly Tyr Glu Leu Leu Gln
   325 330 335
   Gly His Leu Pro Leu Lys Ser Phe Thr Ala Val Cys Gln Lys Asp Gly
   340 345 350
   Ser Trp Asp Arg Pro Met Pro Ala Cys Ser Ile Val Asp Cys Gly Pro
   355 360 365
   Pro Asp Asp Leu Pro Ser Gly Arg Val Glu Tyr Ile Thr Gly Pro Gly
   370 375 380
   Val Thr Thr Tyr Lys Ala Val Ile Gln Tyr Ser Cys Glu Glu Thr Phe
   385 390 395 400
   Tyr Thr Met Lys Val Asn Asp Gly Lys Tyr Val Cys Glu Ala Asp Gly
   405 410 415
   Phe Trp Thr Ser Ser Lys Gly Glu Lys Ser Leu Pro Val Cys Glu Pro
   420 425 430
   Val Cys Gly Leu Ser Ala Arg Thr Thr Gly Gly Arg Ile Tyr Gly Gly
           435                 440                 445
   Gln Lys Ala Lys Pro Gly Asp Phe Pro Trp Gln Val Leu Ile Leu Gly
       450                 455                 460
   Gly Thr Thr Ala Ala Gly Ala Leu Leu Tyr Asp Asn Trp Val Leu Thr
   465                 470                 475                 480
   Ala Ala His Ala Val Tyr Glu Gln Lys His Asp Ala Ser Ala Leu Asp
                   485                 490                 495
   Ile Arg Met Gly Thr Leu Lys Arg Leu Ser Pro His Tyr Thr Gln Ala
               500                 505                 510
   Trp Ser Glu Ala Val Phe Ile His Glu Gly Tyr Thr His Asp Ala Gly
           515                 520                 525
   Phe Asp Asn Asp Ile Ala Leu Ile Lys Leu Asn Asn Lys Val Val Ile
       530                 535                 540
   Asn Ser Asn Ile Thr Pro Ile Cys Leu Pro Arg Lys Glu Ala Glu Ser
   545                 550                 555                 560
   Phe Met Arg Thr Asp Asp Ile Gly Thr Ala Ser Gly Trp Gly Leu Thr
                   565                 570                 575
   Gln Arg Gly Phe Leu Ala Arg Asn Leu Met Tyr Val Asp Ile Pro Ile
               580                 585                 590
   Val Asp His Gln Lys Cys Thr Ala Ala Tyr Glu Lys Pro Pro Tyr Pro
           595                 600                 605
   Arg Gly Ser Val Thr Ala Asn Met Leu Cys Ala Gly Leu Glu Ser Gly
       610                 615                 620
   Gly Lys Asp Ser Cys Arg Gly Asp Ser Gly Gly Ala Leu Val Phe Leu
   625                 630                 635                 640
   Asp Ser Glu Thr Glu Arg Trp Phe Val Gly Gly Ile Val Ser Trp Gly
                   645                 650                 655
   Ser Met Asn Cys Gly Glu Ala Gly Gln Tyr Gly Val Tyr Thr Lys Val
               660                 665                 670
   Ile Asn Tyr Ile Pro Trp Ile Glu Asn Ile Ile Ser Asp Phe
           675                 680                 685
<210> 6
   <211> 671
   <212> PRT
   <213> Homo sapien
<400> 6
   Thr Pro Leu Gly Pro Lys Trp Pro Glu Pro Val Phe Gly Arg Leu Ala
   1               5                   10                  15
   Ser Pro Gly Phe Pro Gly Glu Tyr Ala Asn Asp Gln Glu Arg Arg Trp
               20                  25                  30
   Thr Leu Thr Ala Pro Pro Gly Tyr Arg Leu Arg Leu Tyr Phe Thr His
           35                  40                  45
   Phe Asp Leu Glu Leu Ser His Leu Cys Glu Tyr Asp Phe Val Lys Leu
       50                  55                  60
   Ser Ser Gly Ala Lys Val Leu Ala Thr Leu Cys Gly Gln Glu Ser Thr
   65                  70                  75                  80
   Asp Thr Glu Arg Ala Pro Gly Lys Asp Thr Phe Tyr Ser Leu Gly Ser
                   85                  90                  95
   Ser Leu Asp Ile Thr Phe Arg Ser Asp Tyr Ser Asn Glu Lys Pro Phe
               100                 105                 110
   Thr Gly Phe Glu Ala Phe Tyr Ala Ala Glu Asp Ile Asp Glu Cys Gln
           115                 120                 125
   Val Ala Pro Gly Glu Ala Pro Thr Cys Asp His His Cys His Asn His
       130                 135                 140
   Leu Gly Gly Phe Tyr Cys Ser Cys Arg Ala Gly Tyr Val Leu His Arg
   145                 150                 155                 160
   Asn Lys Arg Thr Cys Ser Ala Leu Cys Ser Gly Gln Val Phe Thr Gln
                   165                 170                 175
   Arg Ser Gly Glu Leu Ser Ser Pro Glu Tyr Pro Arg Pro Tyr Pro Lys
               180                 185                 190
   Leu Ser Ser Cys Thr Tyr Ser Ile Ser Leu Glu Glu Gly Phe Ser Val
           195                 200                 205
   Ile Leu Asp Phe Val Glu Ser Phe Asp Val Glu Thr His Pro Glu Thr
       210                 215                 220
   Leu Cys Pro Tyr Asp Phe Leu Lys Ile Gln Thr Asp Arg Glu Glu His
   225 230 235 240
   Gly Pro Phe Cys Gly Lys Thr Leu Pro His Arg Ile Glu Thr Lys Ser
   245 250 255
   Asn Thr Val Thr Ile Thr Phe Val Thr Asp Glu Ser Gly Asp His Thr
   260 265 270
   Gly Trp Lys Ile His Tyr Thr Ser Thr Ala Gln Pro Cys Pro Tyr Pro
   275 280 285
   Met Ala Pro Pro Asn Gly His Val Ser Pro Val Gln Ala Lys Tyr Ile
   290 295 300
   Leu Lys Asp Ser Phe Ser Ile Phe Cys Glu Thr Gly Tyr Glu Leu Leu
   305 310 315 320
   Gln Gly His Leu Pro Leu Lys Ser Phe Thr Ala Val Cys Gln Lys Asp
   325 330 335
   Gly Ser Trp Asp Arg Pro Met Pro Ala Cys Ser Ile Val Asp Cys Gly
   340 345 350
   Pro Pro Asp Asp Leu Pro Ser Gly Arg Val Glu Tyr Ile Thr Gly Pro
   355 360 365
   Gly Val Thr Thr Tyr Lys Ala Val Ile Gln Tyr Ser Cys Glu Glu Thr
   370 375 380
   Phe Tyr Thr Met Lys Val Asn Asp Gly Lys Tyr Val Cys Glu Ala Asp
   385 390 395 400
   Gly Phe Trp Thr Ser Ser Lys Gly Glu Lys Ser Leu Pro Val Cys Glu
   405 410 415
   Pro Val Cys Gly Leu Ser Ala Arg Thr Thr Gly Gly Arg Ile Tyr Gly
   420 425 430
   Gly Gln Lys Ala Lys Pro Gly Asp Phe Pro Trp Gln Val Leu Ile Leu
   435 440 445
   Gly Gly Thr Thr Ala Ala Gly Ala Leu Leu Tyr Asp Asn Trp Val Leu
   450 455 460
   Thr Ala Ala His Ala Val Tyr Glu Gln Lys His Asp Ala Ser Ala Leu
   465 470 475 480
   Asp Ile Arg Met Gly Thr Leu Lys Arg Leu Ser Pro His Tyr Thr Gln
   485 490 495
   Ala Trp Ser Glu Ala Val Phe Ile His Glu Gly Tyr Thr His Asp Ala
   500 505 510
   Gly Phe Asp Asn Asp Ile Ala Leu Ile Lys Leu Asn Asn Lys Val Val
   515 520 525
   Ile Asn Ser Asn Ile Thr Pro Ile Cys Leu Pro Arg Lys Glu Ala Glu
   530 535 540
   Ser Phe Met Arg Thr Asp Asp Ile Gly Thr Ala Ser Gly Trp Gly Leu
   545 550 555 560
   Thr Gln Arg Gly Phe Leu Ala Arg Asn Leu Met Tyr Val Asp Ile Pro
   565 570 575
   Ile Val Asp His Gln Lys Cys Thr Ala Ala Tyr Glu Lys Pro Pro Tyr
   580 585 590
   Pro Arg Gly Ser Val Thr Ala Asn Met Leu Cys Ala Gly Leu Glu Ser
   595 600 605
   Gly Gly Lys Asp Ser Cys Arg Gly Asp Ser Gly Gly Ala Leu Val Phe
   610 615 620
   Leu Asp Ser Glu Thr Glu Arg Trp Phe Val Gly Gly Ile Val Ser Trp
   625 630 635 640
   Gly Ser Met Asn Cys Gly Glu Ala Gly Gln Tyr Gly Val Tyr Thr Lys
   645 650 655
   Val Ile Asn Tyr Ile Pro Trp Ile Glu Asn Ile Ile Ser Asp Phe
   660 665 670
<210> 7
   <211> 4900
   <212> DNA
   <213> Homo sapien
<400> 7
   cctgtcctgc ctgcctggaa ctctgagcag gctggagtca tggagtcgat tcccagaatc 60
   ccagagtcag ggaggctggg ggcaggggca ggtcactgga caaacagatc aaaggtgaga 120
   ccagcgtagg actgcagacc aggccaggcc agctggacgg gcacaccatg aggtaggtgg 180
   gcgccacagc ctccctgcag ggtgtggggt gggagcacag gcctgggcct caccgcccct 240
   gccctgccca taggctgctg accctcctgg gccttctgtg tggctcggtg gccaccccct 300
   taggcccgaa gtggcctgaa cctgtgttcg ggcgcctggc atcccccggc tttccagggg 360
   agtatgccaa tgaccaggag cggcgctgga ccctgactgc accccccggc taccgcctgc 420
   gcctctactt cacccacttc gacctggagc tctcccacct ctgcgagtac gacttcgtca 480
   aggtgccgtc agacgggagg gctggggttt ctcagggtcg gggggtcccc aaggagtagc 540
   cagggttcag ggacacctgg gagcaggggc caggcttggc caggagggag atcaggcctg 600
   ggtcttgcct tcactccctg tgacacctga ccccacagct gagctcgggg gccaaggtgc 660
   tggccacgct gtgcgggcag gagagcacag acacggagcg ggcccctggc aaggacactt 720
   tctactcgct gggctccagc ctggacatta ccttccgctc cgactactcc aacgagaagc 780
   cgttcacggg gttcgaggcc ttctatgcag ccgagggtga gccaagaggg gtcctgcaac 840
   atctcagtct gcgcagctgg ctgtgggggt aactctgtct taggccaggc agccctgcct 900
   tcagtttccc cacctttccc agggcagggg agaggcctct ggcctgacat catccacaat 960
   gcaaagacca aaacagccgt gacctccatt cacatgggct gagtgccaac tctgagccag 1020
   ggatctgagg acagcatcgc ctcaagtgac gcagggactg gccgggcgcg gcagctcacg 1080
   cctgtaattc cagcactttg ggaggccgag gctggcttga taatttgagg gtcaggagtt 1140
   caaggccagc cagggcaaca cggtgaaact ctatctccac taaaactaca aaaattagct 1200
   gggcgtggtg gtgcgcacct ggaatcccag ctactaggga ggctgaggca ggagaattgc 1260
   ttgaacctgc gaggtggagg ctgcagtgaa cagagattgc accactacac tccacctggg 1320
   cgacagacta gactccgtct caaaaaacaa aaaacaaaaa ccacgcaggg ccgagggccc 1380
   atttacaagc tgacaaagtg ggccctgcca gcgggagcgc tgcaggatgt ttgattttca 1440
   gatcccagtc cctgcagaga ccaactgtgt gacctctggc aagtggctca atttctctgc 1500
   tccttagaag ctgctgcaag ggttcagcgc tgtagccccg ccccctgggt ttgattgact 1560
   cccctcatta gctgggtgac ctcggccgga cactgaaact cccactggtt taacagaggt 1620
   gatgtttgca tctttctccc agcgctgctg ggagcttgca gcgaccctag gcctgtaagg 1680
   tgattggccc ggcaccagtc ccgcacccta gacaggacct aggcctcctc tgaggtccac 1740
   tctgaggtca tggatctcct gggaggagtc caggctggat cccgcctctt tccctcctga 1800
   cggcctgcct ggccctgcct ctcccccaga cattgacgag tgccaggtgg ccccgggaga 1860
   ggcgcccacc tgcgaccacc actgccacaa ccacctgggc ggtttctact gctcctgccg 1920
   cgcaggctac gtcctgcacc gtaacaagcg cacctgctca ggtgagggag gctgcctggg 1980
   ccccaacgca ccctctcctg ggatacccgg ggctcctcag ggccattgct gctctgccca 2040
   ggggtgcgga gggcctgggc ctggacactg ggtgcttcta ggccctgctg cctccagctc 2100
   cccttctcag ccctgcttcc cctctcagca gccaggctca tcagtgccac cctgccctag 2160
   cactgagact aattctaaca tcccactgtg tacctggttc cacctgggct ctgggaaccc 2220
   ctcatgtagc cacgggagag tcggggtatc taccctcgtt ccttggactg ggttcctgtt 2280
   ccctgcactg ggggacgggc cagtgctctg gggcgtgggc agccccaccc tgtggcgctg 2340
   accctgctcc cccgactcgg tttctcctct cggggtctct ccttgcctct ctgatctctc 2400
   ttccagagca gagcctctag cctcccctgg agctccggct gcccagcagg tcagaagcca 2460
   gagccaggct gctggcctca gctccgggtt gggctgagat gctgtgcccc aactcccatt 2520
   cacccaccat ggacccaata ataaacctgg ccccacccca cctgctgccg cgtgtctctg 2580
   gggtgggagg gtcgggaggc ggtggggcgc gctcctctct gcctaccctc ctcacagcct 2640
   catgaacccc aggtctgtgg gagcctcctc catggggcca cacggtcctt ggcctcaccc 2700
   cctgttttga agatggggca ctgaggccgg agaggggtaa ggcctcgctc gagtccaggt 2760
   ccccagaggc tgagcccaga gtaatcttga accaccccca ttcagggtct ggcctggagg 2820
   agcctgaccc acagaggaga caccctggga gatattcatt gaggggtaat ctggtccccc 2880
   gcaaatccag gggtgattcc cactgcccca taggcacagc cacgtggaag aaggcaggca 2940
   atgttggggc tcctcacttc ctagaggcct cacaactcaa atgcccccca ctgcagctgg 3000
   gggtggggtg gtggtatggg atggggacca agccttcctt gaaggataga gcccagccca 3060
   acaccccgcc ccgtggcagc agcatcacgt gttccagcga ggaaggagag caccagactc 3120
   agtcatgatc actgttgcct tgaacttcca agaacagccc cagggcaagg gtcaaaacag 3180
   gggaaagggg gtgatgagag atccttcttc cggatgttcc tccaggaacc agggggctgg 3240
   ctggtcttgg ctgggttcgg gtaggagacc catgatgaat aaacttggga atcactgggg 3300
   tggctgtaag ggaatttagg ggagctccga aggggccctt aggctcgagg agatgctcct 3360
   ctcttttccc gaattcccag ggacccagga gagtgtccct tcttcctctt cctgtgtgtc 3420
   catccacccc cgccccccgc cctggcagag ctggtggaac tcagtgctct agcccctacc 3480
   ctggggttgc gactctggct caggacacca ccacgctccc tgggggtgtg agtgagggcc 3540
   tgtgcgctcc atcccgagtg ctgcctgttt cagctaaagc ctcaaagcaa gagaaacccc 3600
   ctctctaagc ggcccctcag ccatcgggtg ggtcgtttgg tttctgggta ggcctcaggg 3660
   gctggccacc tgcagggccc agcccaaccc agggatgcag atgtcccagc cacatccctg 3720
   tcccagtttc ctgctcccca aggcatccac cctgctgttg gtgcgagggc tgatagaggg 3780
   cacgccaagt cactcccctg cccttccctc cttccagccc tgtgctccgg ccaggtcttc 3840
   acccagaggt ctggggagct cagcagccct gaatacccac ggccgtatcc caaactctcc 3900
   agttgcactt acagcatcag cctggaggag gggttcagtg tcattctgga ctttgtggag 3960
   tccttcgatg tggagacaca ccctgaaacc ctgtgtccct acgactttct caaggtctgg 4020
   ctcctgggcc cctcatcttg tcccagatcc tcccccttca gcccagctgc accccctact    4080
   tcctgcagca tggcccccac cacgttcccg tcaccctcgg tgaccccacc tcttcaggtg    4140
   ctctatggag gtcaaggctg gggcttcgag tacaagtgtg ggaggcagag tggggagggg    4200
   caccccaatc catggcctgg gttggcctca ttggctgtcc ctgaaatgct gaggaggtgg    4260
   gttacttccc tccgcccagg ccagacccag gcagctgctc cccagctttc atgagcttct    4320
   ttctcagatt caaacagaca gagaagaaca tggcccattc tgtgggaaga cattgcccca    4380
   caggattgaa acaaaaagca acacggtgac catcaccttt gtcacagatg aatcaggaga    4440
   ccacacaggc tggaagatcc actacacgag cacagtgagc aagtgggctc agatccttgg    4500
   tggaagcgca gagctgcctc tctctggagt gcaaggagct gtagagtgta gggctcttct    4560
   gggcaggact aggaagggac accaggttta gtggtgctga ggtctgaggc agcagcttct    4620
   aaggggaagc acccgtgccc tcctcagcag cacccagcat cttcaccact cattcttcaa    4680
   ccacccattc acccatcact catcttttac ccacccaccc tttgccactc atccttctgt    4740
   ccctcatcct tccaaccatt catcaatcac ccacccatcc atcctttgcc acacaaccat    4800
   ccacccattc ttctacctac ccatcctatc catccatcct tctatcagca tccttctacc    4860
   acccatcctt cgttcggtca tccatcatca tccatccatc                          4900
<210> 8
   <211> 136
   <212> PRT
   <213> Homo sapien
<400> 8
   Met Arg Leu Leu Thr Leu Leu Gly Leu Leu Cys Gly Ser Val Ala Thr
   1               5                   10                  15
   Pro Leu Gly Pro Lys Trp Pro Glu Pro Val Phe Gly Arg Leu Ala Ser
               20                  25                  30
   Pro Gly Phe Pro Gly Glu Tyr Ala Asn Asp Gln Glu Arg Arg Trp Thr
           35                  40                  45
   Leu Thr Ala Pro Pro Gly Tyr Arg Leu Arg Leu Tyr Phe Thr His Phe
       50                  55                  60
   Asp Leu Glu Leu Ser His Leu Cys Glu Tyr Asp Phe Val Lys Leu Ser
   65                  70                  75                  80
   Ser Gly Ala Lys Val Leu Ala Thr Leu Cys Gly Gln Glu Ser Thr Asp
                   85                  90                  95
   Thr Glu Arg Ala Pro Gly Lys Asp Thr Phe Tyr Ser Leu Gly Ser Ser
   100 105 110
   Leu Asp Ile Thr Phe Arg Ser Asp Tyr Ser Asn Glu Lys Pro Phe Thr
   115 120 125
   Gly Phe Glu Ala Phe Tyr Ala Ala
   130 135
<210> 9
   <211> 181
   <212> PRT
   <213> Homo sapien
<400> 9
   Met Arg Leu Leu Thr Leu Leu Gly Leu Leu Cys Gly Ser Val Ala Thr
   1 5 10 15
   Pro Leu Gly Pro Lys Trp Pro Glu Pro Val Phe Gly Arg Leu Ala Ser
   20 25 30
   Pro Gly Phe Pro Gly Glu Tyr Ala Asn Asp Gln Glu Arg Arg Trp Thr
   35 40 45
   Leu Thr Ala Pro Pro Gly Tyr Arg Leu Arg Leu Tyr Phe Thr His Phe
   50 55 60
   Asp Leu Glu Leu Ser His Leu Cys Glu Tyr Asp Phe Val Lys Leu Ser
   65 70 75 80
   Ser Gly Ala Lys Val Leu Ala Thr Leu Cys Gly Gln Glu Ser Thr Asp
   85 90 95
   Thr Glu Arg Ala Pro Gly Lys Asp Thr Phe Tyr Ser Leu Gly Ser Ser
   100 105 110
   Leu Asp Ile Thr Phe Arg Ser Asp Tyr Ser Asn Glu Lys Pro Phe Thr
   115 120 125
   Gly Phe Glu Ala Phe Tyr Ala Ala Glu Asp Ile Asp Glu Cys Gln Val
   130 135 140
   Ala Pro Gly Glu Ala Pro Thr Cys Asp His His Cys His Asn His Leu
   145 150 155 160
   Gly Gly Phe Tyr Cys Ser Cys Arg Ala Gly Tyr Val Leu His Arg Asn
   165 170 175
   Lys Arg Thr Cys Ser
   180
<210> 10
   <211> 293
   <212> PRT
   <213> Homo sapien
<400> 10
   Met Arg Leu Leu Thr Leu Leu Gly Leu Leu Cys Gly Ser Val Ala Thr
   1               5                   10                  15
   Pro Leu Gly Pro Lys Trp Pro Glu Pro Val Phe Gly Arg Leu Ala Ser
               20                  25                  30
   Pro Gly Phe Pro Gly Glu Tyr Ala Asn Asp Gln Glu Arg Arg Trp Thr
           35                  40                  45
   Leu Thr Ala Pro Pro Gly Tyr Arg Leu Arg Leu Tyr Phe Thr His Phe
       50                  55                  60
   Asp Leu Glu Leu Ser His Leu Cys Glu Tyr Asp Phe Val Lys Leu Ser
   65                  70                  75                  80
   Ser Gly Ala Lys Val Leu Ala Thr Leu Cys Gly Gln Glu Ser Thr Asp
                   85                  90                  95
   Thr Glu Arg Ala Pro Gly Lys Asp Thr Phe Tyr Ser Leu Gly Ser Ser
               100                 105                 110
   Leu Asp Ile Thr Phe Arg Ser Asp Tyr Ser Asn Glu Lys Pro Phe Thr
           115                 120                 125
   Gly Phe Glu Ala Phe Tyr Ala Ala Glu Asp Ile Asp Glu Cys Gln Val
       130                 135                 140
   Ala Pro Gly Glu Ala Pro Thr Cys Asp His His Cys His Asn His Leu
   145                 150                 155                 160
   Gly Gly Phe Tyr Cys Ser Cys Arg Ala Gly Tyr Val Leu His Arg Asn
                   165                 170                 175
   Lys Arg Thr Cys Ser Ala Leu Cys Ser Gly Gln Val Phe Thr Gln Arg
               180                 185                 190
   Ser Gly Glu Leu Ser Ser Pro Glu Tyr Pro Arg Pro Tyr Pro Lys Leu
           195                 200                 205
   Ser Ser Cys Thr Tyr Ser Ile Ser Leu Glu Glu Gly Phe Ser Val Ile
       210                 215                 220
   Leu Asp Phe Val Glu Ser Phe Asp Val Glu Thr His Pro Glu Thr Leu
   225                 230                 235                 240
   Cys Pro Tyr Asp Phe Leu Lys Ile Gln Thr Asp Arg Glu Glu His Gly
                   245                 250                 255
   Pro Phe Cys Gly Lys Thr Leu Pro His Arg Ile Glu Thr Lys Ser Asn
               260                 265                 270
   Thr Val Thr Ile Thr Phe Val Thr Asp Glu Ser Gly Asp His Thr Gly
           275                 280                 285
   Trp Lys Ile His Tyr
       290
<210> 11
   <211> 41
   <212> PRT
   <213> Homo sapien
<400> 11
   Glu Asp Ile Asp Glu Cys Gln Val Ala Pro Gly Glu Ala Pro Thr Cys
   1               5                   10                  15
   Asp His His Cys His Asn His Leu Gly Gly Phe Tyr Cys Ser Cys Arg
               20                  25                  30
   Ala Gly Tyr Val Leu His Arg Asn Lys
           35                  40
<210> 12
   <211> 242
   <212> PRT
   <213> Homo sapien
<400> 12
   Ile Tyr Gly Gly Gln Lys Ala Lys Pro Gly Asp Phe Pro Trp Gln Val
   1               5                   10                  15
   Leu Ile Leu Gly Gly Thr Thr Ala Ala Gly Ala Leu Leu Tyr Asp Asn
               20                  25                  30
   Trp Val Leu Thr Ala Ala His Ala Val Tyr Glu Gln Lys His Asp Ala
           35                  40                  45
   Ser Ala Leu Asp Ile Arg Met Gly Thr Leu Lys Arg Leu Ser Pro His
   50 55 60
   Tyr Thr Gln Ala Trp Ser Glu Ala Val Phe Ile His Glu Gly Tyr Thr
   65 70 75 80
   His Asp Ala Gly Phe Asp Asn Asp Ile Ala Leu Ile Lys Leu Asn Asn
   85 90 95
   Lys Val Val Ile Asn Ser Asn Ile Thr Pro Ile Cys Leu Pro Arg Lys
   100 105 110
   Glu Ala Glu Ser Phe Met Arg Thr Asp Asp Ile Gly Thr Ala Ser Gly
   115 120 125
   Trp Gly Leu Thr Gln Arg Gly Phe Leu Ala Arg Asn Leu Met Tyr Val
   130 135 140
   Asp Ile Pro Ile Val Asp His Gln Lys Cys Thr Ala Ala Tyr Glu Lys
   145 150 155 160
   Pro Pro Tyr Pro Arg Gly Ser Val Thr Ala Asn Met Leu Cys Ala Gly
   165 170 175
   Leu Glu Ser Gly Gly Lys Asp Ser Cys Arg Gly Asp Ser Gly Gly Ala
   180 185 190
   Leu Val Phe Leu Asp Ser Glu Thr Glu Arg Trp Phe Val Gly Gly Ile
   195 200 205
   Val Ser Trp Gly Ser Met Asn Cys Gly Glu Ala Gly Gln Tyr Gly Val
   210 215 220
   Tyr Thr Lys Val Ile Asn Tyr Ile Pro Trp Ile Glu Asn Ile Ile Ser
   225 230 235 240
   Asp Phe
<210> 13
   <211> 16
   <212> PRT
   <213> Artificial Sequence
<220>
   <223> Synthetic peptide
<400> 13
   Gly Lys Asp Ser Cys Arg Gly Asp Ala Gly Gly Ala Leu Val Phe Leu
   1 5 10 15
<210> 14
   <211> 15
   <212> PRT
   <213> Artificial Sequence
<220>
   <223> Synthetic peptide
<400> 14
   Thr Pro Leu Gly Pro Lys Trp Pro Glu Pro Val Phe Gly Arg Leu
   1 5 10 15
<210> 15
   <211> 43
   <212> PRT
   <213> Artificial Sequence
<220>
   <223> Synthetic peptide
<400> 15
   Thr Ala Pro Pro Gly Tyr Arg Leu Arg Leu Tyr Phe Thr His Phe Asp
   1 5 10 15
   Leu Glu Leu Ser His Leu Cys Glu Tyr Asp Phe Val Lys Leu Ser Ser
   20 25 30
   Gly Ala Lys Val Leu Ala Thr Leu Cys Gly Gln
   35 40
<210> 16
   <211> 8
   <212> PRT
   <213> Artificial
<220>
   <223> Synthetic peptide
<400> 16
   Thr Phe Arg Ser Asp Tyr Ser Asn
   1 5
<210> 17
   <211> 25
   <212> PRT
   <213> Artificial Sequence
<220>
   <223> Synthetic peptide
<400> 17
   Phe Tyr Ser Leu Gly Ser Ser Leu Asp Ile Thr Phe Arg Ser Asp Tyr
   1 5 10 15
   Ser Asn Glu Lys Pro Phe Thr Gly Phe
   20 25
<210> 18
   <211> 9
   <212> PRT
   <213> Artificial
<220>
   <223> Synthetic peptide
<400> 18
   Ile Asp Glu Cys Gln Val Ala Pro Gly
   1 5
<210> 19
   <211> 25
   <212> PRT
   <213> Artificial Sequence
<220>
   <223> Synthetic peptide
<400> 19
   Ala Asn Met Leu Cys Ala Gly Leu Glu Ser Gly Gly Lys Asp Ser Cys
   1 5 10 15
   Arg Gly Asp Ser Gly Gly Ala Leu Val
   20 25
<210> 20
   <211> 960
   <212> DNA
   <213> Homo sapien
<220>
   <221> CDS
   <222> (51)..(797)
<400> 20
   attaactgag attaaccttc cctgagtttt ctcacaccaa ggtgaggacc atg tcc        56
                                                          Met Ser
                                                          1
   ctg ttt cca tca ctc cct ctc ctt ctc ctg agt atg gtg gca gcg tct      104
   Leu Phe Pro Ser Leu Pro Leu Leu Leu Leu Ser Met Val Ala Ala Ser
           5                   10                  15
   tac tca gaa act gtg acc tgt gag gat gcc caa aag acc tgc cct gca      152
   Tyr Ser Glu Thr Val Thr Cys Glu Asp Ala Gln Lys Thr Cys Pro Ala
       20                  25                  30
   gtg att gcc tgt agc tct cca ggc atc aac ggc ttc cca ggc aaa gat      200
   Val Ile Ala Cys Ser Ser Pro Gly Ile Asn Gly Phe Pro Gly Lys Asp
   35                  40                  45                  50
   ggg cgt gat ggc acc aag gga gaa aag ggg gaa cca ggc caa ggg ctc     248
   Gly Arg Asp Gly Thr Lys Gly Glu Lys Gly Glu Pro Gly Gln Gly Leu
                   55                  60                  65
   aga ggc tta cag ggc ccc cct gga aag ttg ggg cct cca gga aat cca     296
   Arg Gly Leu Gln Gly Pro Pro Gly Lys Leu Gly Pro Pro Gly Asn Pro
               70                  75                  80
   ggg cct tct ggg tca cca gga cca aag ggc caa aaa gga gac cct gga     344
   Gly Pro Ser Gly Ser Pro Gly Pro Lys Gly Gln Lys Gly Asp Pro Gly
           85                  90                  95
   aaa agt ccg gat ggt gat agt agc ctg gct gcc tca gaa aga aaa gct     392
   Lys Ser Pro Asp Gly Asp Ser Ser Leu Ala Ala Ser Glu Arg Lys Ala
       100                 105                 110
   ctg caa aca gaa atg gca cgt atc aaa aag tgg ctc acc ttc tct ctg     440
   Leu Gln Thr Glu Met Ala Arg Ile Lys Lys Trp Leu Thr Phe Ser Leu
   115                 120                 125                 130
   ggc aaa caa gtt ggg aac aag ttc ttc ctg acc aat ggt gaa ata atg     488
   Gly Lys Gln Val Gly Asn Lys Phe Phe Leu Thr Asn Gly Glu Ile Met
                   135                 140                 145
   acc ttt gaa aaa gtg aag gcc ttg tgt gtc aag ttc cag gcc tct gtg     536
   Thr Phe Glu Lys Val Lys Ala Leu Cys Val Lys Phe Gln Ala Ser Val
               150                 155                 160
   gcc acc ccc agg aat gct gca gag aat gga gcc att cag aat ctc atc     584
   Ala Thr Pro Arg Asn Ala Ala Glu Asn Gly Ala Ile Gln Asn Leu Ile
           165                 170                 175
   aag gag gaa gcc ttc ctg ggc atc act gat gag aag aca gaa ggg cag     632
   Lys Glu Glu Ala Phe Leu Gly Ile Thr Asp Glu Lys Thr Glu Gly Gln
       180                 185                 190
   ttt gtg gat ctg aca gga aat aga ctg acc tac aca aac tgg aac gag     680
   Phe Val Asp Leu Thr Gly Asn Arg Leu Thr Tyr Thr Asn Trp Asn Glu
   195                 200                 205                 210
   ggt gaa ccc aac aat gct ggt tct gat gaa gat tgt gta ttg cta ctg     728
   Gly Glu Pro Asn Asn Ala Gly Ser Asp Glu Asp Cys Val Leu Leu Leu
                   215                 220                 225
   aaa aat ggc cag tgg aat gac gtc ccc tgc tcc acc tcc cat ctg gcc     776
   Lys Asn Gly Gln Trp Asn Asp Val Pro Cys Ser Thr Ser His Leu Ala
               230                 235                 240
   gtc tgt gag ttc cct atc tga agggtcatat cactcaggcc ctccttgtct        827
   Val Cys Glu Phe Pro Ile
           245
   ttttactgca acccacaggc ccacagtatg cttgaaaaga taaattatat caatttcctc   887
   atatccagta ttgttccttt tgtgggcaat cactaaaaat gatcactaac agcaccaaca   947
   aagcaataat agt                                                      960
<210> 21
   <211> 248
   <212> PRT
   <213> Homo sapien
<400> 21
   Met Ser Leu Phe Pro Ser Leu Pro Leu Leu Leu Leu Ser Met Val Ala
   1               5                   10                  15
   Ala Ser Tyr Ser Glu Thr Val Thr Cys Glu Asp Ala Gln Lys Thr Cys
               20                  25                  30
   Pro Ala Val Ile Ala Cys Ser Ser Pro Gly Ile Asn Gly Phe Pro Gly
           35                  40                  45
   Lys Asp Gly Arg Asp Gly Thr Lys Gly Glu Lys Gly Glu Pro Gly Gln
       50                  55                  60
   Gly Leu Arg Gly Leu Gln Gly Pro Pro Gly Lys Leu Gly Pro Pro Gly
   65                  70                  75                  80
   Asn Pro Gly Pro Ser Gly Ser Pro Gly Pro Lys Gly Gln Lys Gly Asp
                   85                  90                  95
   Pro Gly Lys Ser Pro Asp Gly Asp Ser Ser Leu Ala Ala Ser Glu Arg
               100                 105                 110
   Lys Ala Leu Gln Thr Glu Met Ala Arg Ile Lys Lys Trp Leu Thr Phe
           115                 120                 125
   Ser Leu Gly Lys Gln Val Gly Asn Lys Phe Phe Leu Thr Asn Gly Glu
       130                 135                 140
   Ile Met Thr Phe Glu Lys Val Lys Ala Leu Cys Val Lys Phe Gln Ala
   145                 150                 155                 160
   Ser Val Ala Thr Pro Arg Asn Ala Ala Glu Asn Gly Ala Ile Gln Asn
                   165                 170                 175
   Leu Ile Lys Glu Glu Ala Phe Leu Gly Ile Thr Asp Glu Lys Thr Glu
               180                 185                 190
   Gly Gln Phe Val Asp Leu Thr Gly Asn Arg Leu Thr Tyr Thr Asn Trp
           195                 200                 205
   Asn Glu Gly Glu Pro Asn Asn Ala Gly Ser Asp Glu Asp Cys Val Leu
       210                 215                 220
   Leu Leu Lys Asn Gly Gln Trp Asn Asp Val Pro Cys Ser Thr Ser His
   225                 230                 235                 240
   Leu Ala Val Cys Glu Phe Pro Ile
                   245
<210> 22
   <211> 6
   <212> PRT
   <213> Artificial Sequence
<220>
   <223> Synthetic peptide consensus sequence
<220>
   <221> MISC_FEATURE
   <222> (1)..(1)
   <223> Wherein X at position 1 represents hydroxyproline
<220>
   <221> MISC_FEATURE
   <222> (4)..(4)
   <223> wherein x at position 4 represents hydrophobic residue
<400> 22
   Xaa Gly Lys Xaa Gly Pro
   1               5
<210> 23
   <211> 5
   <212> PRT
   <213> Artificial Sequence
<220>
   <223> Synthetic peptide
<220>
   <221> MISC_FEATURE
   <222> (1)..(1)
   <223> wherein X represents hydroxyproline
<400> 23
   Xaa Gly Lys Leu Gly
   1               5
<210> 24
   <211> 16
   <212> PRT
   <213> Artificial Sequence
<220>
   <223> Synthetic peptide
<220>
   <221> MISC_FEATURE
   <222> (9)..(15)
   <223> Wherein X at positions 9 and 15 represents hydroxyproline
<400> 24
   Gly Leu Arg Gly Leu Gln Gly Pro Xaa Gly Lys Leu Gly Pro Xaa Gly
   1               5                   10                  15
<210> 25
   <211> 27
   <212> PRT
   <213> Artificial Sequence
<220>
   <223> Synthetic peptide
<220>
   <221> MISC_FEATURE
   <222> (3)..(27)
   <223> wherein X at positions 3, 6, 15, 21, 24, 27 represents hydroxyproline
<400> 25
   Gly Pro Xaa Gly Pro Xaa Gly Leu Arg Gly Leu Gln Gly Pro Xaa Gly
   1               5                   10                  15
   Lys Leu Gly Pro Xaa Gly Pro Xaa Gly Pro Xaa
               20                  25
<210> 26
   <211> 53
   <212> PRT
   <213> Artificial Sequence
<220>
   <223> Synthetic peptide
<220>
   <221> misc_feature
   <222> (26)..(26)
   <223> Xaa can be any naturally occurring amino acid
<220>
   <221> misc_feature
   <222> (32)..(32)
   <223> Xaa can be any naturally occurring amino acid
<220>
   <221> misc_feature
   <222> (35)..(35)
   <223> Xaa can be any naturally occurring amino acid
<220>
   <221> misc_feature
   <222> (41)..(41)
   <223> Xaa can be any naturally occurring amino acid
<220>
   <221> misc_feature
   <222> (50)..(50)
   <223> Xaa can be any naturally occurring amino acid
<400> 26
   Gly Lys Asp Gly Arg Asp Gly Thr Lys Gly Glu Lys Gly Glu Pro Gly
   1 5 10 15
   Gln Gly Leu Arg Gly Leu Gln Gly Pro Xaa Gly Lys Leu Gly Pro Xaa
   20 25 30
   Gly Asn Xaa Gly Pro Ser Gly Ser Xaa Gly Pro Lys Gly Gln Lys Gly
   35 40 45
   Asp Xaa Gly Lys Ser
   50
<210> 27
   <211> 33
   <212> PRT
   <213> Artificial Sequence
<220>
   <223> Synthetic peptide
<220>
   <221> MISC_FEATURE
   <222> (3)..(33)
   <223> wherein X at positions 3, 6, 12, 18, 21, 30, 33 represents hydroxyproline
<400> 27
   Gly Ala Xaa Gly Ser Xaa Gly Glu Lys Gly Ala Xaa Gly Pro Gln Gly
   1 5 10 15
   Pro Xaa Gly Pro Xaa Gly Lys Met Gly Pro Lys Gly Glu Xaa Gly Asp
   20 25 30
   Xaa
<210> 28
   <211> 45
   <212> PRT
   <213> Artificial Sequence
<220>
   <223> Synthetic peptide
<220>
   <221> MISC_FEATURE
   <222> (3)..(45)
   <223> Wherein X at positions 3, 6, 9, 27, 30, 36, 42, 45 represents hydroxyproline
<400> 28
   Gly Cys Xaa Gly Leu Xaa Gly Ala Xaa Gly Asp Lys Gly Glu Ala Gly
   1 5 10 15
   Thr Asn Gly Lys Arg Gly Glu Arg Gly Pro Xaa Gly Pro Xaa Gly Lys
   20 25 30
   Ala Gly Pro Xaa Gly Pro Asn Gly Ala Xaa Gly Glu Xaa
   35 40 45
<210> 29
   <211> 24
   <212> PRT
   <213> Artificial Sequence
<220>
   <223> Synthetic peptide
<400> 29
   Leu Gln Arg Ala Leu Glu Ile Leu Pro Asn Arg Val Thr Ile Lys Ala
   1 5 10 15
   Asn Arg Pro Phe Leu Val Phe Ile
   20
<210> 30
   <211> 559
   <212> DNA
   <213> Homo sapien
<400> 30
   atgaggctgc tgaccctcct gggccttctg tgtggctcgg tggccacccc cttgggcccg 60
   aagtggcctg aacctgtgtt cgggcgcctg gcatcccccg gctttccagg ggagtatgcc 120
   aatgaccagg agcggcgctg gaccctgact gcaccccccg gctaccgcct gcgcctctac 180
   ttcacccact tcgacctgga gctctcccac ctctgcgagt acgacttcgt caagctgagc 240
   tcgggggcca aggtgctggc cacgctgtgc gggcaggaga gcacagacac ggagcgggcc 300
   cctggcaagg acactttcta ctcgctgggc tccagcctgg acattacctt ccgctccgac 360
   tactccaacg agaagccgtt cacggggttc gaggccttct atgcagccga ggacattgac 420
   gagtgccagg tggccccggg agaggcgccc acctgcgacc accactgcca caaccacctg 480
   ggcggtttct actgctcctg ccgcgcaggc tacgtcctgc accgtaacaa gcgcacctgc 540
   tcagccctgt gctccggcc 559
<210> 31
   <211> 34
   <212> DNA
   <213> Artificial Sequence
<220>
   <223> Synthetic oligo
<400> 31
   cgggcacacc atgaggctgc tgaccctcct gggc 34
<210> 32
   <211> 33
   <212> DNA
   <213> Artificial Sequence
<220>
   <223> Synthetic oligo
<400> 32
   gacattacct tccgctccga ctccaacgag aag 33
<210> 33
   <211> 33
   <212> DNA
   <213> Artificial Sequence
<220>
   <223> Synthetic oligo
<400> 33
   agcagccctg aatacccacg gccgtatccc aaa 33
<210> 34
   <211> 26
   <212> DNA
   <213> Artificial Sequence
<220>
   <223> Synthetic oligo
<400> 34
   cgggatccat gaggctgctg accctc 26
<210> 35
   <211> 19
   <212> DNA
   <213> Artificial Sequence
<220>
   <223> Synthetic oligo
<400> 35
   ggaattccta ggctgcata 19
<210> 36
   <211> 19
   <212> DNA
   <213> Artificial Sequence
<220>
   <223> Synthetic oligo
<400> 36
   ggaattccta cagggcgct 19
<210> 37
   <211> 19
   <212> DNA
   <213> Artificial Sequence
<220>
   <223> Synthetic oligo
<400> 37
   ggaattccta gtagtggat 19
<210> 38
   <211> 25
   <212> DNA
   <213> Artificial Sequence
<220>
   <223> Synthetic oligo
<400> 38
   tgcggccgct gtaggtgctg tcttt 25
<210> 39
   <211> 23
   <212> DNA
   <213> Artificial Sequence
<220>
   <223> Synthetic oligo
<400> 39
   ggaattcact cgttattctc gga 23
<210> 40
   <211> 17
   <212> DNA
   <213> Artificial Sequence
<220>
   <223> Synthetic oligo
<400> 40
   tccgagaata acgagtg 17
<210> 41
   <211> 29
   <212> DNA
   <213> Artificial Sequence
<220>
   <223> Synthetic oligo
<400> 41
   cattgaaagc tttggggtag aagttgttc 29
<210> 42
   <211> 27
   <212> DNA
   <213> Artificial Sequence
<220>
   <223> Synthetic oligo
<400> 42
   cgcggccgca gctgctcaga gtgtaga 27
<210> 43
   <211> 28
   <212> DNA
   <213> Artificial Sequence
<220>
   <223> Synthetic oligo
<400> 43
   cggtaagctt cactggctca gggaaata 28
<210> 44
   <211> 37
   <212> DNA
   <213> Artificial Sequence
<220>
   <223> Synthetic oligo
<400> 44
   aagaagcttg ccgccaccat ggattggctg tggaact 37
<210> 45
   <211> 31
   <212> DNA
   <213> Artificial Sequence
<220>
   <223> Synthetic oligo
<400> 45
   cgggatcctc aaactttctt gtccaccttg g 31
<210> 46
   <211> 36
   <212> DNA
   <213> Artificial Sequence
<220>
   <223> Synthetic oligo
<400> 46
   aagaaagctt gccgccacca tgttctcact agctct 36
<210> 47
   <211> 26
   <212> DNA
   <213> Artificial Sequence
<220>
   <223> Synthetic oligo
<400> 47
   cgggatcctt ctccctctaa cactct 26
<210> 48
   <211> 9
   <212> PRT
   <213> Artificial Sequence
<220>
   <223> Synthetic peptide
<400> 48
   Glu Pro Lys Ser Cys Asp Lys Thr His
   1 5
<210> 49
   <211> 4960
   <212> DNA
   <213> Homo Sapien
<400> 49
   ccggacgtgg tggcgcatgc ctgtaatccc agctactcgg gaggctgagg caggagaatt 60
   gctcgaaccc cggaggcaga ggtttggtgg ctcacacctg taatcccagc actttgcgag 120
   gctgaggcag gtgcatcgct ttggctcagg agttcaagac cagcctgggc aacacaggga 180
   gacccccatc tctacaaaaa acaaaaacaa atataaaggg gataaaaaaa aaaaaaagac 240
   aagacatgaa tccatgagga cagagtgtgg aagaggaagc agcagcctca aagttctgga 300
   agctggaaga acagataaac aggtgtgaaa taactgcctg gaaagcaact tctttttttt 360
   tttttttttt tttgaggtgg agtctcactc tgtcgtccag gctggagtgc agtggtgcga 420
   tctcggatca ctgcaacctc cgcctcccag gctcaagcaa ttctcctgcc tcagcctccc 480
   gagtagctgg gattataagt gcgcgctgcc acacctggat gatttttgta tttttagtag 540
   agatgggatt tcaccatgtt ggtcaggctg gtctcaaact cccaacctcg tgatccaccc 600
   accttggcct cccaaagtgc tgggattaca ggtataagcc accgagccca gccaaaagcg 660
   acttctaagc ctgcaaggga atcgggaatt ggtggcacca ggtccttctg acagggttta 720
   agaaattagc cagcctgagg ctgggcacgg tggctcacac ctgtaatccc agcactttgg 780
   gaggctaagg caggtggatc acctgagggc aggagttcaa gaccagcctg accaacatgg 840
   agaaacccca tccctaccaa aaataaaaaa ttagccaggt gtggtggtgc tcgcctgtaa 900
   tcccagctac ttgggaggct gaggtgggag gattgcttga acacaggaag tagaggctgc 960
   agtgagctat gattgcagca ctgcactgaa gccggggcaa cagaacaaga tccaaaaaaa 1020
   agggaggggt gaggggcaga gccaggattt gtttccaggc tgttgttacc taggtccgac 1080
   tcctggctcc cagagcagcc tgtcctgcct gcctggaact ctgagcaggc tggagtcatg 1140
   gagtcgattc ccagaatccc agagtcaggg aggctggggg caggggcagg tcactggaca 1200
   aacagatcaa aggtgagacc agcgtagggc tgcagaccag gccaggccag ctggacgggc 1260
   acaccatgag gtaggtgggc gcccacagcc tccctgcagg gtgtggggtg ggagcacagg 1320
   cctgggccct caccgcccct gccctgccca taggctgctg accctcctgg gccttctgtg 1380
   tggctcggtg gccaccccct tgggcccgaa gtggcctgaa cctgtgttcg ggcgcctggc 1440
   atcccccggc tttccagggg agtatgccaa tgaccaggag cggcgctgga ccctgactgc 1500
   accccccggc taccgcctgc gcctctactt cacccacttc gacctggagc tctcccacct 1560
   ctgcgagtac gacttcgtca aggtgccgtc aggacgggag ggctggggtt tctcagggtc 1620
   ggggggtccc caaggagtag ccagggttca gggacacctg ggagcagggg ccaggcttgg 1680
   ccaggaggga gatcaggcct gggtcttgcc ttcactccct gtgacacctg accccacagc 1740
   tgagctcggg ggccaaggtg ctggccacgc tgtgcgggca ggagagcaca gacacggagc 1800
   gggcccctgg caaggacact ttctactcgc tgggctccag cctggacatt accttccgct 1860
   ccgactactc caacgagaag ccgttcacgg ggttcgaggc cttctatgca gccgagggtg 1920
   agccaagagg ggtcctgcaa catctcagtc tgcgcagctg gctgtggggg taactctgtc 1980
   ttaggccagg cagccctgcc ttcagtttcc ccacctttcc cagggcaggg gagaggcctc 2040
   tggcctgaca tcatccacaa tgcaaagacc aaaacagccg tgacctccat tcacatgggc 2100
   tgagtgccaa ctctgagcca gggatctgag gacagcatcg cctcaagtga cgcagggact 2160
   ggccgggcgc agcagctcac gcctgtaatt ccagcacttt gggaggccga ggctggctga 2220
   tcatttgagg tcaggagttc aaggccagcc agggcaacac ggtgaaactc tatctccact 2280
   aaaactacaa aaattagctg ggcgtggtgg tgcgcacctg gaatcccagc tactagggag 2340
   gctgaggcag gagaattgct tgaacctgcg aggtggaggc tgcagtgaac agagattgca 2400
   ccactacact ccagcctggg cgacagagct agactccgtc tcaaaaaaca aaaaacaaaa 2460
   acgacgcagg ggccgagggc cccatttaca gctgacaaag tggggccctg ccagcgggag 2520
   cgctgccagg atgtttgatt tcagatccca gtccctgcag agaccaactg tgtgacctct 2580
   ggcaagtggc tcaatttctc tgctccttag gaagctgctg caagggttca gcgctgtagc 2640
   cccgccccct gggtttgatt gactcccctc attagctggg tgacctcggg ccggacactg 2700
   aaactcccac tggtttaaca gaggtgatgt ttgcatcttt ctcccagcgc tgctgggagc 2760
   ttgcagcgac cctaggcctg taaggtgatt ggcccggcac cagtcccgca ccctagacag 2820
   gacgaggcct cctctgaggt ccactctgag gtcatggatc tcctgggagg agtccaggct 2880
   ggatcccgcc tctttccctc ctgacggcct gcctggccct gcctctcccc cagacattga 2940
   cgagtgccag gtggccccgg gagaggcgcc cacctgcgac caccactgcc acaaccacct 3000
   gggcggtttc tactgctcct gccgcgcagg ctacgtcctg caccgtaaca agcgcacctg 3060
   ctcagccctg tgctccggcc aggtcttcac ccagaggtct ggggagctca gcagccctga 3120
   atacccacgg ccgtatccca aactctccag ttgcacttac agcatcagcc tggaggaggg 3180
   gttcagtgtc attctggact ttgtggagtc cttcgatgtg gagacacacc ctgaaaccct 3240
   gtgtccctac gactttctca agattcaaac agacagagaa gaacatggcc cattctgtgg 3300
   gaagacattg ccccacagga ttgaaacaaa aagcaacacg gtgaccatca cctttgtcac 3360
   agatgaatca ggagaccaca caggctggaa gatccactac acgagcacag cgcacgcttg 3420
   cccttatccg atggcgccac ctaatggcca cgtttcacct gtgcaagcca aatacatcct 3480
   gaaagacagc ttctccatct tttgcgagac tggctatgag cttctgcaag gtcacttgcc 3540
   cctgaaatcc tttactgcag tttgtcagaa agatggatct tgggaccggc caatgcccgc 3600
   gtgcagcatt gttgactgtg gccctcctga tgatctaccc agtggccgag tggagtacat 3660
   cacaggtcct ggagtgacca cctacaaagc tgtgattcag tacagctgtg aagagacctt 3720
   ctacacaatg aaagtgaatg atggtaaata tgtgtgtgag gctgatggat tctggacgag 3780
   ctccaaagga gaaaaatcac tcccagtctg tgagcctgtt tgtggactat cagcccgcac 3840
   aacaggaggg cgtatatatg gagggcaaaa ggcaaaacct ggtgattttc cttggcaagt 3900
   cctgatatta ggtggaacca cagcagcagg tgcactttta tatgacaact gggtcctaac 3960
   agctgctcat gccgtctatg agcaaaaaca tgatgcatcc gccctggaca ttcgaatggg 4020
   caccctgaaa agactatcac ctcattatac acaagcctgg tctgaagctg tttttataca 4080
   tgaaggttat actcatgatg ctggctttga caatgacata gcactgatta aattgaataa 4140
   caaagttgta atcaatagca acatcacgcc tatttgtctg ccaagaaaag aagctgaatc 4200
   ctttatgagg acagatgaca ttggaactgc atctggatgg ggattaaccc aaaggggttt 4260
   tcttgctaga aatctaatgt atgtcgacat accgattgtt gaccatcaaa aatgtactgc 4320
   tgcatatgaa aagccaccct atccaagggg aagtgtaact gctaacatgc tttgtgctgg 4380
   cttagaaagt gggggcaagg acagctgcag aggtgacagc ggaggggcac tggtgtttct 4440
   agatagtgaa acagagaggt ggtttgtggg aggaatagtg tcctggggtt ccatgaattg 4500
   tggggaagca ggtcagtatg gagtctacac aaaagttatt aactatattc cctggatcga 4560
   gaacataatt agtgattttt aacttgcgtg tctgcagtca aggattcttc atttttagaa 4620
   atgcctgtga agaccttggc agcgacgtgg ctcgagaagc attcatcatt actgtggaca    4680
   tggcagttgt tgctccaccc aaaaaaacag actccaggtg aggctgctgt catttctcca    4740
   cttgccagtt taattccagc cttacccatt gactcaaggg gacataaacc acgagagtga    4800
   cagtcatctt tgcccaccca gtgtaatgtc actgctcaaa ttacatttca ttaccttaaa    4860
   aagccagtct cttttcatac tggctgttgg catttctgta aactgcctgt ccatgctctt    4920
   tgtttttaaa cttgttctta ttgaaaaaaa aaaaaaaaaa                          4960
<210> 50
   <211> 2090
   <212> DNA
   <213> Murine MASP-2 CDS
<220>
   <221> CDS
   <222> (33)..(2090)
<400> 50
   ggcgctggac tgcagagcta tggtggcaca cc atg agg cta ctc atc ttc ctg       53
                                       Met Arg Leu Leu Ile Phe Leu
                                       1               5
   ggt ctg ctg tgg agt ttg gtg gcc aca ctt ctg ggt tca aag tgg cct      101
   Gly Leu Leu Trp Ser Leu Val Ala Thr Leu Leu Gly Ser Lys Trp Pro
           10                  15                  20
   gaa cct gta ttc ggg cgc ctg gtg tcc cct ggc ttc cca gag aag tat      149
   Glu Pro Val Phe Gly Arg Leu Val Ser Pro Gly Phe Pro Glu Lys Tyr
       25                  30                  35
   gct gac cat caa gat cga tcc tgg aca ctg act gca ccc cct ggc tac      197
   Ala Asp His Gln Asp Arg Ser Trp Thr Leu Thr Ala Pro Pro Gly Tyr
   40                  45                  50                  55
   cgc ctg cgc ctc tac ttc acc cac ttt gac ctg gaa ctc tct tac cgc      245
   Arg Leu Arg Leu Tyr Phe Thr His Phe Asp Leu Glu Leu Ser Tyr Arg
                   60                  65                  70
   tgc gag tat gac ttt gtc aag ttg agc tca ggg acc aag gtg ctg gcc      293
   Cys Glu Tyr Asp Phe Val Lys Leu Ser Ser Gly Thr Lys Val Leu Ala
               75                  80                  85
   aca ctg tgt ggg cag gag agt aca gac act gag cag gca cct ggc aat      341
   Thr Leu Cys Gly Gln Glu Ser Thr Asp Thr Glu Gln Ala Pro Gly Asn
           90                  95                  100
   gac acc ttc tac tca ctg ggt ccc agc cta aag gtc acc ttc cac tcc      389
   Asp Thr Phe Tyr Ser Leu Gly Pro Ser Leu Lys Val Thr Phe His Ser
       105                 110                 115
   gac tac tcc aat gag aag ccg ttc aca ggg ttt gag gcc ttc tat gca      437
   Asp Tyr Ser Asn Glu Lys Pro Phe Thr Gly Phe Glu Ala Phe Tyr Ala
   120                 125                 130                 135
   gcg gag gat gtg gat gaa tgc aga gtg tct ctg gga gac tca gtc cct      485
   Ala Glu Asp Val Asp Glu Cys Arg Val Ser Leu Gly Asp Ser Val Pro
                   140                 145                 150
   tgt gac cat tat tgc cac aac tac ttg ggc ggc tac tat tgc tcc tgc      533
   Cys Asp His Tyr Cys His Asn Tyr Leu Gly Gly Tyr Tyr Cys Ser Cys
               155                 160                 165
   aga gcg ggc tac att ctc cac cag aac aag cac acg tgc tca gcc ctt      581
   Arg Ala Gly Tyr Ile Leu His Gln Asn Lys His Thr Cys Ser Ala Leu
           170                 175                 180
   tgt tca ggc cag gtg ttc aca gga aga tct ggg tat ctc agt agc cct      629
   Cys Ser Gly Gln Val Phe Thr Gly Arg Ser Gly Tyr Leu Ser Ser Pro
       185                 190                 195
   gag tac ccg cag cca tac ccc aag ctc tcc agc tgc acc tac agc atc      677
   Glu Tyr Pro Gln Pro Tyr Pro Lys Leu Ser Ser Cys Thr Tyr Ser Ile
   200                 205                 210                 215
   cgc ctg gag gac ggc ttc agt gtc atc ctg gac ttc gtg gag tcc ttc      725
   Arg Leu Glu Asp Gly Phe Ser Val Ile Leu Asp Phe Val Glu Ser Phe
                   220                 225                 230
   gat gtg gag acg cac cct gaa gcc cag tgc ccc tat gac tcc ctc aag      773
   Asp Val Glu Thr His Pro Glu Ala Gln Cys Pro Tyr Asp Ser Leu Lys
               235                 240                 245
   att caa aca gac aag ggg gaa cac ggc cca ttt tgt ggg aag acg ctg      821
   Ile Gln Thr Asp Lys Gly Glu His Gly Pro Phe Cys Gly Lys Thr Leu
           250                 255                 260
   cct ccc agg att gaa act gac agc cac aag gtg acc atc acc ttt gcc      869
   Pro Pro Arg Ile Glu Thr Asp Ser His Lys Val Thr Ile Thr Phe Ala
       265                 270                 275
   act gac gag tcg ggg aac cac aca ggc tgg aag ata cac tac aca agc      917
   Thr Asp Glu Ser Gly Asn His Thr Gly Trp Lys Ile His Tyr Thr Ser
   280                 285                 290                 295
   aca gca cgg ccc tgc cct gat cca acg gcg cca cct aat ggc agc att      965
   Thr Ala Arg Pro Cys Pro Asp Pro Thr Ala Pro Pro Asn Gly Ser Ile
                   300                 305                 310
   tca cct gtg caa gcc acg tat gtc ctg aag gac agg ttt tct gtc ttc     1013
   Ser Pro Val Gln Ala Thr Tyr Val Leu Lys Asp Arg Phe Ser Val Phe
               315                 320                 325
   tgc aag aca ggc ttc gag ctt ctg caa ggt tct gtc ccc ctg aaa tca     1061
   Cys Lys Thr Gly Phe Glu Leu Leu Gln Gly Ser Val Pro Leu Lys Ser
           330                 335                 340
   ttc act gct gtc tgt cag aaa gat gga tct tgg gac cgg ccg atg cca     1109
   Phe Thr Ala Val Cys Gln Lys Asp Gly Ser Trp Asp Arg Pro Met Pro
       345                 350                 355
   gag tgc agc att att gat tgt ggc cct ccc gat gac cta ccc aat ggc     1157
   Glu Cys Ser Ile Ile Asp Cys Gly Pro Pro Asp Asp Leu Pro Asn Gly
   360                 365                 370                 375
   cat gtg gac tat atc aca ggc cct caa gtg act acc tac aaa gct gtg     1205
   His Val Asp Tyr Ile Thr Gly Pro Gln Val Thr Thr Tyr Lys Ala Val
                   380                 385                 390
   att cag tac agc tgt gaa gag act ttc tac aca atg agc agc aat ggt     1253
   Ile Gln Tyr Ser Cys Glu Glu Thr Phe Tyr Thr Met Ser Ser Asn Gly
               395                 400                 405
   aaa tat gtg tgt gag gct gat gga ttc tgg acg agc tcc aaa gga gaa      1301
   Lys Tyr Val Cys Glu Ala Asp Gly Phe Trp Thr Ser Ser Lys Gly Glu
           410                 415                 420
   aaa ctc ccc ccg gtt tgt gag cct gtt tgt ggg ctg tcc aca cac act      1349
   Lys Leu Pro Pro Val Cys Glu Pro Val Cys Gly Leu Ser Thr His Thr
       425                 430                 435
   ata gga gga cgc ata gtt gga ggg cag cct gca aag cct ggt gac ttt      1397
   Ile Gly Gly Arg Ile Val Gly Gly Gln Pro Ala Lys Pro Gly Asp Phe
   440                 445                 450                 455
   cct tgg caa gtc ttg ttg ctg ggt caa act aca gca gca gca ggt gca      1445
   Pro Trp Gln Val Leu Leu Leu Gly Gln Thr Thr Ala Ala Ala Gly Ala
                   460                 465                 470
   ctt ata cat gac aat tgg gtc cta aca gcc gct cat gct gta tat gag      1493
   Leu Ile His Asp Asn Trp Val Leu Thr Ala Ala His Ala Val Tyr Glu
               475                 480                 485
   aaa aga atg gca gcg tcc tcc ctg aac atc cga atg ggc atc ctc aaa      1541
   Lys Arg Met Ala Ala Ser Ser Leu Asn Ile Arg Met Gly Ile Leu Lys
           490                 495                 500
   agg ctc tca cct cat tac act caa gcc tgg ccc gag gaa atc ttt ata      1589
   Arg Leu Ser Pro His Tyr Thr Gln Ala Trp Pro Glu Glu Ile Phe Ile
       505                 510                 515
   cat gaa ggc tac act cac ggt gct ggt ttt gac aat gat ata gca ttg      1637
   His Glu Gly Tyr Thr His Gly Ala Gly Phe Asp Asn Asp Ile Ala Leu
   520                 525                 530                 535
   att aaa ctc aag aac aaa gtc aca atc aac gga agc atc atg cct gtt      1685
   Ile Lys Leu Lys Asn Lys Val Thr Ile Asn Gly Ser Ile Met Pro Val
                   540                 545                 550
   tgc cta ccg cga aaa gaa gct gca tcc tta atg aga aca gac ttc act      1733
   Cys Leu Pro Arg Lys Glu Ala Ala Ser Leu Met Arg Thr Asp Phe Thr
               555                 560                 565
   gga act gtg gct ggc tgg ggg tta acc cag aag ggg ctt ctt gct aga      1781
   Gly Thr Val Ala Gly Trp Gly Leu Thr Gln Lys Gly Leu Leu Ala Arg
           570                 575                 580
   aac cta atg ttt gtg gac ata cca att gct gac cac caa aaa tgt acc      1829
   Asn Leu Met Phe Val Asp Ile Pro Ile Ala Asp His Gln Lys Cys Thr
       585                 590                 595
   acc gtg tat gaa aag ctc tat cca gga gta aga gta agc gct aac atg      1877
   Thr Val Tyr Glu Lys Leu Tyr Pro Gly Val Arg Val Ser Ala Asn Met
   600                 605                 610                 615
   ctc tgt gct ggc tta gag act ggt ggc aag gac agc tgc aga ggt gac      1925
   Leu Cys Ala Gly Leu Glu Thr Gly Gly Lys Asp Ser Cys Arg Gly Asp
                   620                 625                 630
   agt ggg ggg gca tta gtg ttt cta gat aat gag aca cag cga tgg ttt      1973
   Ser Gly Gly Ala Leu Val Phe Leu Asp Asn Glu Thr Gln Arg Trp Phe
               635                 640                 645
   gtg gga gga ata gtt tcc tgg ggt tcc att aat tgt ggg gcg gca ggc      2021
   Val Gly Gly Ile Val Ser Trp Gly Ser Ile Asn Cys Gly Ala Ala Gly
           650                 655                 660
   cag tat ggg gtc tac aca aaa gtc atc aac tat att ccc tgg aat gag      2069
   Gln Tyr Gly Val Tyr Thr Lys Val Ile Asn Tyr Ile Pro Trp Asn Glu
       665                 670                 675
   aac ata ata agt aat ttc taa                                          2090
   Asn Ile Ile Ser Asn Phe
   680                 685
<210> 51
   <211> 685
   <212> PRT
   <213> Murine MASP-2 CDS
<400> 51
   Met Arg Leu Leu Ile Phe Leu Gly Leu Leu Trp Ser Leu Val Ala Thr
   1               5                   10                  15
   Leu Leu Gly Ser Lys Trp Pro Glu Pro Val Phe Gly Arg Leu Val Ser
               20                  25                  30
   Pro Gly Phe Pro Glu Lys Tyr Ala Asp His Gln Asp Arg Ser Trp Thr
           35                  40                  45
   Leu Thr Ala Pro Pro Gly Tyr Arg Leu Arg Leu Tyr Phe Thr His Phe
       50                  55                  60
   Asp Leu Glu Leu Ser Tyr Arg Cys Glu Tyr Asp Phe Val Lys Leu Ser
   65                  70                  75                  80
   Ser Gly Thr Lys Val Leu Ala Thr Leu Cys Gly Gln Glu Ser Thr Asp
                   85                  90                  95
   Thr Glu Gln Ala Pro Gly Asn Asp Thr Phe Tyr Ser Leu Gly Pro Ser
               100                 105                 110
   Leu Lys Val Thr Phe His Ser Asp Tyr Ser Asn Glu Lys Pro Phe Thr
           115                 120                 125
   Gly Phe Glu Ala Phe Tyr Ala Ala Glu Asp Val Asp Glu Cys Arg Val
       130                 135                 140
   Ser Leu Gly Asp Ser Val Pro Cys Asp His Tyr Cys His Asn Tyr Leu
   145                 150                 155                 160
   Gly Gly Tyr Tyr Cys Ser Cys Arg Ala Gly Tyr Ile Leu His Gln Asn
                   165                 170                 175
   Lys His Thr Cys Ser Ala Leu Cys Ser Gly Gln Val Phe Thr Gly Arg
   180 185 190
   Ser Gly Tyr Leu Ser Ser Pro Glu Tyr Pro Gln Pro Tyr Pro Lys Leu
   195 200 205
   Ser Ser Cys Thr Tyr Ser Ile Arg Leu Glu Asp Gly Phe Ser Val Ile
   210 215 220
   Leu Asp Phe Val Glu Ser Phe Asp Val Glu Thr His Pro Glu Ala Gln
   225 230 235 240
   Cys Pro Tyr Asp Ser Leu Lys Ile Gln Thr Asp Lys Gly Glu His Gly
   245 250 255
   Pro Phe Cys Gly Lys Thr Leu Pro Pro Arg Ile Glu Thr Asp Ser His
   260 265 270
   Lys Val Thr Ile Thr Phe Ala Thr Asp Glu Ser Gly Asn His Thr Gly
   275 280 285
   Trp Lys Ile His Tyr Thr Ser Thr Ala Arg Pro Cys Pro Asp Pro Thr
   290 295 300
   Ala Pro Pro Asn Gly Ser Ile Ser Pro Val Gln Ala Thr Tyr Val Leu
   305 310 315 320
   Lys Asp Arg Phe Ser Val Phe Cys Lys Thr Gly Phe Glu Leu Leu Gln
   325 330 335
   Gly Ser Val Pro Leu Lys Ser Phe Thr Ala Val Cys Gln Lys Asp Gly
   340 345 350
   Ser Trp Asp Arg Pro Met Pro Glu Cys Ser Ile Ile Asp Cys Gly Pro
   355 360 365
   Pro Asp Asp Leu Pro Asn Gly His Val Asp Tyr Ile Thr Gly Pro Gln
   370 375 380
   Val Thr Thr Tyr Lys Ala Val Ile Gln Tyr Ser Cys Glu Glu Thr Phe
   385 390 395 400
   Tyr Thr Met Ser Ser Asn Gly Lys Tyr Val Cys Glu Ala Asp Gly Phe
   405 410 415
   Trp Thr Ser Ser Lys Gly Glu Lys Leu Pro Pro Val Cys Glu Pro Val
   420 425 430
   Cys Gly Leu Ser Thr His Thr Ile Gly Gly Arg Ile Val Gly Gly Gln
           435                 440                 445
   Pro Ala Lys Pro Gly Asp Phe Pro Trp Gln Val Leu Leu Leu Gly Gln
       450                 455                 460
   Thr Thr Ala Ala Ala Gly Ala Leu Ile His Asp Asn Trp Val Leu Thr
   465                 470                 475                 480
   Ala Ala His Ala Val Tyr Glu Lys Arg Met Ala Ala Ser Ser Leu Asn
                   485                 490                 495
   Ile Arg Met Gly Ile Leu Lys Arg Leu Ser Pro His Tyr Thr Gln Ala
               500                 505                 510
   Trp Pro Glu Glu Ile Phe Ile His Glu Gly Tyr Thr His Gly Ala Gly
           515                 520                 525
   Phe Asp Asn Asp Ile Ala Leu Ile Lys Leu Lys Asn Lys Val Thr Ile
       530                 535                 540
   Asn Gly Ser Ile Met Pro Val Cys Leu Pro Arg Lys Glu Ala Ala Ser
   545                 550                 555                 560
   Leu Met Arg Thr Asp Phe Thr Gly Thr Val Ala Gly Trp Gly Leu Thr
                   565                 570                 575
   Gln Lys Gly Leu Leu Ala Arg Asn Leu Met Phe Val Asp Ile Pro Ile
               580                 585                 590
   Ala Asp His Gln Lys Cys Thr Thr Val Tyr Glu Lys Leu Tyr Pro Gly
           595                 600                 605
   Val Arg Val Ser Ala Asn Met Leu Cys Ala Gly Leu Glu Thr Gly Gly
       610                 615                 620
   Lys Asp Ser Cys Arg Gly Asp Ser Gly Gly Ala Leu Val Phe Leu Asp
   625                 630                 635                 640
   Asn Glu Thr Gln Arg Trp Phe Val Gly Gly Ile Val Ser Trp Gly Ser
                   645                 650                 655
   Ile Asn Cys Gly Ala Ala Gly Gln Tyr Gly Val Tyr Thr Lys Val Ile
               660                 665                 670
   Asn Tyr Ile Pro Trp Asn Glu Asn Ile Ile Ser Asn Phe
           675                 680                 685
<210> 52
   <211> 670
   <212> PRT
   <213> Murine MASP-2 mature protein
<400> 52
   Thr Leu Leu Gly Ser Lys Trp Pro Glu Pro Val Phe Gly Arg Leu Val
   1               5                   10                  15
   Ser Pro Gly Phe Pro Glu Lys Tyr Ala Asp His Gln Asp Arg Ser Trp
               20                  25                  30
   Thr Leu Thr Ala Pro Pro Gly Tyr Arg Leu Arg Leu Tyr Phe Thr His
           35                  40                  45
   Phe Asp Leu Glu Leu Ser Tyr Arg Cys Glu Tyr Asp Phe Val Lys Leu
       50                  55                  60
   Ser Ser Gly Thr Lys Val Leu Ala Thr Leu Cys Gly Gln Glu Ser Thr
   65                  70                  75                  80
   Asp Thr Glu Gln Ala Pro Gly Asn Asp Thr Phe Tyr Ser Leu Gly Pro
                   85                  90                  95
   Ser Leu Lys Val Thr Phe His Ser Asp Tyr Ser Asn Glu Lys Pro Phe
               100                 105                 110
   Thr Gly Phe Glu Ala Phe Tyr Ala Ala Glu Asp Val Asp Glu Cys Arg
           115                 120                 125
   Val Ser Leu Gly Asp Ser Val Pro Cys Asp His Tyr Cys His Asn Tyr
       130                 135                 140
   Leu Gly Gly Tyr Tyr Cys Ser Cys Arg Ala Gly Tyr Ile Leu His Gln
   145                 150                 155                 160
   Asn Lys His Thr Cys Ser Ala Leu Cys Ser Gly Gln Val Phe Thr Gly
                   165                 170                 175
   Arg Ser Gly Tyr Leu Ser Ser Pro Glu Tyr Pro Gln Pro Tyr Pro Lys
               180                 185                 190
   Leu Ser Ser Cys Thr Tyr Ser Ile Arg Leu Glu Asp Gly Phe Ser Val
           195                 200                 205
   Ile Leu Asp Phe Val Glu Ser Phe Asp Val Glu Thr His Pro Glu Ala
       210                 215                 220
   Gln Cys Pro Tyr Asp Ser Leu Lys Ile Gln Thr Asp Lys Gly Glu His
   225 230 235 240
   Gly Pro Phe Cys Gly Lys Thr Leu Pro Pro Arg Ile Glu Thr Asp Ser
   245 250 255
   His Lys Val Thr Ile Thr Phe Ala Thr Asp Glu Ser Gly Asn His Thr
   260 265 270
   Gly Trp Lys Ile His Tyr Thr Ser Thr Ala Arg Pro Cys Pro Asp Pro
   275 280 285
   Thr Ala Pro Pro Asn Gly Ser Ile Ser Pro Val Gln Ala Thr Tyr Val
   290 295 300
   Leu Lys Asp Arg Phe Ser Val Phe Cys Lys Thr Gly Phe Glu Leu Leu
   305 310 315 320
   Gln Gly Ser Val Pro Leu Lys Ser Phe Thr Ala Val Cys Gln Lys Asp
   325 330 335
   Gly Ser Trp Asp Arg Pro Met Pro Glu Cys Ser Ile Ile Asp Cys Gly
   340 345 350
   Pro Pro Asp Asp Leu Pro Asn Gly His Val Asp Tyr Ile Thr Gly Pro
   355 360 365
   Gln Val Thr Thr Tyr Lys Ala Val Ile Gln Tyr Ser Cys Glu Glu Thr
   370 375 380
   Phe Tyr Thr Met Ser Ser Asn Gly Lys Tyr Val Cys Glu Ala Asp Gly
   385 390 395 400
   Phe Trp Thr Ser Ser Lys Gly Glu Lys Leu Pro Pro Val Cys Glu Pro
   405 410 415
   Val Cys Gly Leu Ser Thr His Thr Ile Gly Gly Arg Ile Val Gly Gly
   420 425 430
   Gln Pro Ala Lys Pro Gly Asp Phe Pro Trp Gln Val Leu Leu Leu Gly
   435 440 445
   Gln Thr Thr Ala Ala Ala Gly Ala Leu Ile His Asp Asn Trp Val Leu
   450 455 460
   Thr Ala Ala His Ala Val Tyr Glu Lys Arg Met Ala Ala Ser Ser Leu
   465 470 475 480
   Asn Ile Arg Met Gly Ile Leu Lys Arg Leu Ser Pro His Tyr Thr Gln
   485 490 495
   Ala Trp Pro Glu Glu Ile Phe Ile His Glu Gly Tyr Thr His Gly Ala
   500 505 510
   Gly Phe Asp Asn Asp Ile Ala Leu Ile Lys Leu Lys Asn Lys Val Thr
   515 520 525
   Ile Asn Gly Ser Ile Met Pro Val Cys Leu Pro Arg Lys Glu Ala Ala
   530 535 540
   Ser Leu Met Arg Thr Asp Phe Thr Gly Thr Val Ala Gly Trp Gly Leu
   545 550 555 560
   Thr Gln Lys Gly Leu Leu Ala Arg Asn Leu Met Phe Val Asp Ile Pro
   565 570 575
   Ile Ala Asp His Gln Lys Cys Thr Thr Val Tyr Glu Lys Leu Tyr Pro
   580 585 590
   Gly Val Arg Val Ser Ala Asn Met Leu Cys Ala Gly Leu Glu Thr Gly
   595 600 605
   Gly Lys Asp Ser Cys Arg Gly Asp Ser Gly Gly Ala Leu Val Phe Leu
   610 615 620
   Asp Asn Glu Thr Gln Arg Trp Phe Val Gly Gly Ile Val Ser Trp Gly
   625 630 635 640
   Ser Ile Asn Cys Gly Ala Ala Gly Gln Tyr Gly Val Tyr Thr Lys Val
   645 650 655
   Ile Asn Tyr Ile Pro Trp Asn Glu Asn Ile Ile Ser Asn Phe
   660 665 670
<210> 53
   <211> 2091
   <212> DNA
   <213> Rat MASP-2 CDS
<220>
   <221> CDS
   <222> (10)..(2067)
<400> 53
   tggcacaca atg agg cta ctg atc gtc ctg ggt ctg ctt tgg agt ttg gtg 51
               Met Arg Leu Leu Ile Val Leu Gly Leu Leu Trp Ser Leu Val
               1               5                   10
   gcc aca ctt ttg ggc tcc aag tgg cct gag cct gta ttc ggg cgc ctg      99
   Ala Thr Leu Leu Gly Ser Lys Trp Pro Glu Pro Val Phe Gly Arg Leu
   15                  20                  25                  30
   gtg tcc ctg gcc ttc cca gag aag tat ggc aac cat cag gat cga tcc     147
   Val Ser Leu Ala Phe Pro Glu Lys Tyr Gly Asn His Gln Asp Arg Ser
                   35                  40                  45
   tgg acg ctg act gca ccc cct ggc ttc cgc ctg cgc ctc tac ttc acc     195
   Trp Thr Leu Thr Ala Pro Pro Gly Phe Arg Leu Arg Leu Tyr Phe Thr
               50                  55                  60
   cac ttc aac ctg gaa ctc tct tac cgc tgc gag tat gac ttt gtc aag     243
   His Phe Asn Leu Glu Leu Ser Tyr Arg Cys Glu Tyr Asp Phe Val Lys
           65                  70                  75
   ttg acc tca ggg acc aag gtg cta gcc acg ctg tgt ggg cag gag agt     291
   Leu Thr Ser Gly Thr Lys Val Leu Ala Thr Leu Cys Gly Gln Glu Ser
       80                  85                  90
   aca gat act gag cgg gca cct ggc aat gac acc ttc tac tca ctg ggt     339
   Thr Asp Thr Glu Arg Ala Pro Gly Asn Asp Thr Phe Tyr Ser Leu Gly
   95                  100                 105                 110
   ccc agc cta aag gtc acc ttc cac tcc gac tac tcc aat gag aag cca     387
   Pro Ser Leu Lys Val Thr Phe His Ser Asp Tyr Ser Asn Glu Lys Pro
                   115                 120                 125
   ttc aca gga ttt gag gcc ttc tat gca gcg gag gat gtg gat gaa tgc     435
   Phe Thr Gly Phe Glu Ala Phe Tyr Ala Ala Glu Asp Val Asp Glu Cys
               130                 135                 140
   aga aca tcc ctg gga gac tca gtc cct tgt gac cat tat tgc cac aac     483
   Arg Thr Ser Leu Gly Asp Ser Val Pro Cys Asp His Tyr Cys His Asn
           145                 150                 155
   tac ctg ggc ggc tac tac tgc tcc tgc cga gtg ggc tac att ctg cac     531
   Tyr Leu Gly Gly Tyr Tyr Cys Ser Cys Arg Val Gly Tyr Ile Leu His
       160                 165                 170
   cag aac aag cat acc tgc tca gcc ctt tgt tca ggc cag gtg ttc act     579
   Gln Asn Lys His Thr Cys Ser Ala Leu Cys Ser Gly Gln Val Phe Thr
   175                 180                 185                 190
   ggg agg tct ggc ttt ctc agt agc cct gag tac cca cag cca tac ccc     627
   Gly Arg Ser Gly Phe Leu Ser Ser Pro Glu Tyr Pro Gln Pro Tyr Pro
                   195                 200                 205
   aaa ctc tcc agc tgc gcc tac aac atc cgc ctg gag gaa ggc ttc agt     675
   Lys Leu Ser Ser Cys Ala Tyr Asn Ile Arg Leu Glu Glu Gly Phe Ser
               210                 215                 220
   atc acc ctg gac ttc gtg gag tcc ttt gat gtg gag atg cac cct gaa     723
   Ile Thr Leu Asp Phe Val Glu Ser Phe Asp Val Glu Met His Pro Glu
           225                 230                 235
   gcc cag tgc ccc tac gac tcc ctc aag att caa aca gac aag agg gaa     771
   Ala Gln Cys Pro Tyr Asp Ser Leu Lys Ile Gln Thr Asp Lys Arg Glu
       240                 245                 250
   tac ggc ccg ttt tgt ggg aag acg ctg ccc ccc agg att gaa act gac      819
   Tyr Gly Pro Phe Cys Gly Lys Thr Leu Pro Pro Arg Ile Glu Thr Asp
   255                 260                 265                 270
   agc aac aag gtg acc att acc ttt acc acc gac gag tca ggg aac cac      867
   Ser Asn Lys Val Thr Ile Thr Phe Thr Thr Asp Glu Ser Gly Asn His
                   275                 280                 285
   aca ggc tgg aag ata cac tac aca agc aca gca cag ccc tgc cct gat      915
   Thr Gly Trp Lys Ile His Tyr Thr Ser Thr Ala Gln Pro Cys Pro Asp
               290                 295                 300
   cca acg gcg cca cct aat ggt cac att tca cct gtg caa gcc acg tat      963
   Pro Thr Ala Pro Pro Asn Gly His Ile Ser Pro Val Gln Ala Thr Tyr
           305                 310                 315
   gtc ctg aag gac agc ttt tct gtc ttc tgc aag act ggc ttc gag ctt      1011
   Val Leu Lys Asp Ser Phe Ser Val Phe Cys Lys Thr Gly Phe Glu Leu
       320                 325                 330
   ctg caa ggt tct gtc ccc ctg aag tca ttc act gct gtc tgt cag aaa      1059
   Leu Gln Gly Ser Val Pro Leu Lys Ser Phe Thr Ala Val Cys Gln Lys
   335                 340                 345                 350
   gat gga tct tgg gac cgg ccg ata cca gag tgc agc att att gac tgt      1107
   Asp Gly Ser Trp Asp Arg Pro Ile Pro Glu Cys Ser Ile Ile Asp Cys
                   355                 360                 365
   ggc cct ccc gat gac cta ccc aat ggc cac gtg gac tat atc aca ggc      1155
   Gly Pro Pro Asp Asp Leu Pro Asn Gly His Val Asp Tyr Ile Thr Gly
               370                 375                 380
   cct gaa gtg acc acc tac aaa gct gtg att cag tac agc tgt gaa gag      1203
   Pro Glu Val Thr Thr Tyr Lys Ala Val Ile Gln Tyr Ser Cys Glu Glu
           385                 390                 395
   act ttc tac aca atg agc agc aat ggt aaa tat gtg tgt gag gct gat      1251
   Thr Phe Tyr Thr Met Ser Ser Asn Gly Lys Tyr Val Cys Glu Ala Asp
       400                 405                 410
   gga ttc tgg acg agc tcc aaa gga gaa aaa tcc ctc ccg gtt tgc aag      1299
   Gly Phe Trp Thr Ser Ser Lys Gly Glu Lys Ser Leu Pro Val Cys Lys
   415                 420                 425                 430
   cct gtc tgt gga ctg tcc aca cac act tca gga ggc cgt ata att gga      1347
   Pro Val Cys Gly Leu Ser Thr His Thr Ser Gly Gly Arg Ile Ile Gly
                   435                 440                 445
   gga cag cct gca aag cct ggt gac ttt cct tgg caa gtc ttg tta ctg      1395
   Gly Gln Pro Ala Lys Pro Gly Asp Phe Pro Trp Gln Val Leu Leu Leu
               450                 455                 460
   ggt gaa act aca gca gca ggt gct ctt ata cat gac gac tgg gtc cta      1443
   Gly Glu Thr Thr Ala Ala Gly Ala Leu Ile His Asp Asp Trp Val Leu
           465                 470                 475
   aca gcg gct cat gct gta tat ggg aaa aca gag gcg atg tcc tcc ctg      1491
   Thr Ala Ala His Ala Val Tyr Gly Lys Thr Glu Ala Met Ser Ser Leu
       480                 485                 490
   gac atc cgc atg ggc atc ctc aaa agg ctc tcc ctc att tac act caa      1539
   Asp Ile Arg Met Gly Ile Leu Lys Arg Leu Ser Leu Ile Tyr Thr Gln
   495                 500                 505                 510
   gcc tgg cca gag gct gtc ttt atc cat gaa ggc tac act cac gga gct      1587
   Ala Trp Pro Glu Ala Val Phe Ile His Glu Gly Tyr Thr His Gly Ala
                   515                 520                 525
   ggt ttt gac aat gat ata gca ctg att aaa ctc aag aac aaa gtc aca      1635
   Gly Phe Asp Asn Asp Ile Ala Leu Ile Lys Leu Lys Asn Lys Val Thr
               530                 535                 540
   atc aac aga aac atc atg ccg att tgt cta cca aga aaa gaa gct gca      1683
   Ile Asn Arg Asn Ile Met Pro Ile Cys Leu Pro Arg Lys Glu Ala Ala
           545                 550                 555
   tcc tta atg aaa aca gac ttc gtt gga act gtg gct ggc tgg ggg tta      1731
   Ser Leu Met Lys Thr Asp Phe Val Gly Thr Val Ala Gly Trp Gly Leu
       560                 565                 570
   acc cag aag ggg ttt ctt gct aga aac cta atg ttt gtg gac ata cca      1779
   Thr Gln Lys Gly Phe Leu Ala Arg Asn Leu Met Phe Val Asp Ile Pro
   575                 580                 585                 590
   att gtt gac cac caa aaa tgt gct act gcg tat aca aag cag ccc tac      1827
   Ile Val Asp His Gln Lys Cys Ala Thr Ala Tyr Thr Lys Gln Pro Tyr
                   595                 600                 605
   cca gga gca aaa gtg act gtt aac atg ctc tgt gct ggc cta gac cgc      1875
   Pro Gly Ala Lys Val Thr Val Asn Met Leu Cys Ala Gly Leu Asp Arg
               610                 615                 620
   ggt ggc aag gac agc tgc aga ggt gac agc gga ggg gca tta gtg ttt      1923
   Gly Gly Lys Asp Ser Cys Arg Gly Asp Ser Gly Gly Ala Leu Val Phe
           625                 630                 635
   cta gac aat gaa aca cag aga tgg ttt gtg gga gga ata gtt tcc tgg      1971
   Leu Asp Asn Glu Thr Gln Arg Trp Phe Val Gly Gly Ile Val Ser Trp
       640                 645                 650
   ggt tct att aac tgt ggg ggg tca gaa cag tat ggg gtc tac acg aaa      2019
   Gly Ser Ile Asn Cys Gly Gly Ser Glu Gln Tyr Gly Val Tyr Thr Lys
   655                 660                 665                 670
   gtc acg aac tat att ccc tgg att gag aac ata ata aat aat ttc taa      2067
   Val Thr Asn Tyr Ile Pro Trp Ile Glu Asn Ile Ile Asn Asn Phe
                   675                 680                 685
   tttgcaaaaa aaaaaaaaaa aaaa                                           2091
<210> 54
   <211> 685
   <212> PRT
   <213> Rat MASP-2 CDS
<400> 54
   Met Arg Leu Leu Ile Val Leu Gly Leu Leu Trp Ser Leu Val Ala Thr
   1               5                   10                  15
   Leu Leu Gly Ser Lys Trp Pro Glu Pro Val Phe Gly Arg Leu Val Ser
               20                  25                  30
   Leu Ala Phe Pro Glu Lys Tyr Gly Asn His Gln Asp Arg Ser Trp Thr
   35 40 45
   Leu Thr Ala Pro Pro Gly Phe Arg Leu Arg Leu Tyr Phe Thr His Phe
   50 55 60
   Asn Leu Glu Leu Ser Tyr Arg Cys Glu Tyr Asp Phe Val Lys Leu Thr
   65 70 75 80
   Ser Gly Thr Lys Val Leu Ala Thr Leu Cys Gly Gln Glu Ser Thr Asp
   85 90 95
   Thr Glu Arg Ala Pro Gly Asn Asp Thr Phe Tyr Ser Leu Gly Pro Ser
   100 105 110
   Leu Lys Val Thr Phe His Ser Asp Tyr Ser Asn Glu Lys Pro Phe Thr
   115 120 125
   Gly Phe Glu Ala Phe Tyr Ala Ala Glu Asp Val Asp Glu Cys Arg Thr
   130 135 140
   Ser Leu Gly Asp Ser Val Pro Cys Asp His Tyr Cys His Asn Tyr Leu
   145 150 155 160
   Gly Gly Tyr Tyr Cys Ser Cys Arg Val Gly Tyr Ile Leu His Gln Asn
   165 170 175
   Lys His Thr Cys Ser Ala Leu Cys Ser Gly Gln Val Phe Thr Gly Arg
   180 185 190
   Ser Gly Phe Leu Ser Ser Pro Glu Tyr Pro Gln Pro Tyr Pro Lys Leu
   195 200 205
   Ser Ser Cys Ala Tyr Asn Ile Arg Leu Glu Glu Gly Phe Ser Ile Thr
   210 215 220
   Leu Asp Phe Val Glu Ser Phe Asp Val Glu Met His Pro Glu Ala Gln
   225 230 235 240
   Cys Pro Tyr Asp Ser Leu Lys Ile Gln Thr Asp Lys Arg Glu Tyr Gly
   245 250 255
   Pro Phe Cys Gly Lys Thr Leu Pro Pro Arg Ile Glu Thr Asp Ser Asn
   260 265 270
   Lys Val Thr Ile Thr Phe Thr Thr Asp Glu Ser Gly Asn His Thr Gly
   275 280 285
   Trp Lys Ile His Tyr Thr Ser Thr Ala Gln Pro Cys Pro Asp Pro Thr
       290                 295                 300
   Ala Pro Pro Asn Gly His Ile Ser Pro Val Gln Ala Thr Tyr Val Leu
   305                 310                 315                 320
   Lys Asp Ser Phe Ser Val Phe Cys Lys Thr Gly Phe Glu Leu Leu Gln
                   325                 330                 335
   Gly Ser Val Pro Leu Lys Ser Phe Thr Ala Val Cys Gln Lys Asp Gly
               340                 345                 350
   Ser Trp Asp Arg Pro Ile Pro Glu Cys Ser Ile Ile Asp Cys Gly Pro
           355                 360                 365
   Pro Asp Asp Leu Pro Asn Gly His Val Asp Tyr Ile Thr Gly Pro Glu
       370                 375                 380
   Val Thr Thr Tyr Lys Ala Val Ile Gln Tyr Ser Cys Glu Glu Thr Phe
   385                 390                 395                 400
   Tyr Thr Met Ser Ser Asn Gly Lys Tyr Val Cys Glu Ala Asp Gly Phe
                   405                 410                 415
   Trp Thr Ser Ser Lys Gly Glu Lys Ser Leu Pro Val Cys Lys Pro Val
               420                 425                 430
   Cys Gly Leu Ser Thr His Thr Ser Gly Gly Arg Ile Ile Gly Gly Gln
           435                 440                 445
   Pro Ala Lys Pro Gly Asp Phe Pro Trp Gln Val Leu Leu Leu Gly Glu
       450                 455                 460
   Thr Thr Ala Ala Gly Ala Leu Ile His Asp Asp Trp Val Leu Thr Ala
   465                 470                 475                 480
   Ala His Ala Val Tyr Gly Lys Thr Glu Ala Met Ser Ser Leu Asp Ile
                   485                 490                 495
   Arg Met Gly Ile Leu Lys Arg Leu Ser Leu Ile Tyr Thr Gln Ala Trp
               500                 505                 510
   Pro Glu Ala Val Phe Ile His Glu Gly Tyr Thr His Gly Ala Gly Phe
           515                 520                 525
   Asp Asn Asp Ile Ala Leu Ile Lys Leu Lys Asn Lys Val Thr Ile Asn
       530                 535                 540
   Arg Asn Ile Met Pro Ile Cys Leu Pro Arg Lys Glu Ala Ala Ser Leu
   545 550 555 560
   Met Lys Thr Asp Phe Val Gly Thr Val Ala Gly Trp Gly Leu Thr Gln
   565 570 575
   Lys Gly Phe Leu Ala Arg Asn Leu Met Phe Val Asp Ile Pro Ile Val
   580 585 590
   Asp His Gln Lys Cys Ala Thr Ala Tyr Thr Lys Gln Pro Tyr Pro Gly
   595 600 605
   Ala Lys Val Thr Val Asn Met Leu Cys Ala Gly Leu Asp Arg Gly Gly
   610 615 620
   Lys Asp Ser Cys Arg Gly Asp Ser Gly Gly Ala Leu Val Phe Leu Asp
   625 630 635 640
   Asn Glu Thr Gln Arg Trp Phe Val Gly Gly Ile Val Ser Trp Gly Ser
   645 650 655
   Ile Asn Cys Gly Gly Ser Glu Gln Tyr Gly Val Tyr Thr Lys Val Thr
   660 665 670
   Asn Tyr Ile Pro Trp Ile Glu Asn Ile Ile Asn Asn Phe
   675 680 685
<210> 55
   <211> 670
   <212> PRT
   <213> Rat MASP-2 mature protein
<400> 55
   Thr Leu Leu Gly Ser Lys Trp Pro Glu Pro Val Phe Gly Arg Leu Val
   1 5 10 15
   Ser Leu Ala Phe Pro Glu Lys Tyr Gly Asn His Gln Asp Arg Ser Trp
   20 25 30
   Thr Leu Thr Ala Pro Pro Gly Phe Arg Leu Arg Leu Tyr Phe Thr His
   35 40 45
   Phe Asn Leu Glu Leu Ser Tyr Arg Cys Glu Tyr Asp Phe Val Lys Leu
   50 55 60
   Thr Ser Gly Thr Lys Val Leu Ala Thr Leu Cys Gly Gln Glu Ser Thr
   65 70 75 80
   Asp Thr Glu Arg Ala Pro Gly Asn Asp Thr Phe Tyr Ser Leu Gly Pro
   85 90 95
   Ser Leu Lys Val Thr Phe His Ser Asp Tyr Ser Asn Glu Lys Pro Phe
   100 105 110
   Thr Gly Phe Glu Ala Phe Tyr Ala Ala Glu Asp Val Asp Glu Cys Arg
   115 120 125
   Thr Ser Leu Gly Asp Ser Val Pro Cys Asp His Tyr Cys His Asn Tyr
   130 135 140
   Leu Gly Gly Tyr Tyr Cys Ser Cys Arg Val Gly Tyr Ile Leu His Gln
   145 150 155 160
   Asn Lys His Thr Cys Ser Ala Leu Cys Ser Gly Gln Val Phe Thr Gly
   165 170 175
   Arg Ser Gly Phe Leu Ser Ser Pro Glu Tyr Pro Gln Pro Tyr Pro Lys
   180 185 190
   Leu Ser Ser Cys Ala Tyr Asn Ile Arg Leu Glu Glu Gly Phe Ser Ile
   195 200 205
   Thr Leu Asp Phe Val Glu Ser Phe Asp Val Glu Met His Pro Glu Ala
   210 215 220
   Gln Cys Pro Tyr Asp Ser Leu Lys Ile Gln Thr Asp Lys Arg Glu Tyr
   225 230 235 240
   Gly Pro Phe Cys Gly Lys Thr Leu Pro Pro Arg Ile Glu Thr Asp Ser
   245 250 255
   Asn Lys Val Thr Ile Thr Phe Thr Thr Asp Glu Ser Gly Asn His Thr
   260 265 270
   Gly Trp Lys Ile His Tyr Thr Ser Thr Ala Gln Pro Cys Pro Asp Pro
   275 280 285
   Thr Ala Pro Pro Asn Gly His Ile Ser Pro Val Gln Ala Thr Tyr Val
   290 295 300
   Leu Lys Asp Ser Phe Ser Val Phe Cys Lys Thr Gly Phe Glu Leu Leu
   305 310 315 320
   Gln Gly Ser Val Pro Leu Lys Ser Phe Thr Ala Val Cys Gln Lys Asp
   325 330 335
   Gly Ser Trp Asp Arg Pro Ile Pro Glu Cys Ser Ile Ile Asp Cys Gly
   340 345 350
   Pro Pro Asp Asp Leu Pro Asn Gly His Val Asp Tyr Ile Thr Gly Pro
   355 360 365
   Glu Val Thr Thr Tyr Lys Ala Val Ile Gln Tyr Ser Cys Glu Glu Thr
   370 375 380
   Phe Tyr Thr Met Ser Ser Asn Gly Lys Tyr Val Cys Glu Ala Asp Gly
   385 390 395 400
   Phe Trp Thr Ser Ser Lys Gly Glu Lys Ser Leu Pro Val Cys Lys Pro
   405 410 415
   Val Cys Gly Leu Ser Thr His Thr Ser Gly Gly Arg Ile Ile Gly Gly
   420 425 430
   Gln Pro Ala Lys Pro Gly Asp Phe Pro Trp Gln Val Leu Leu Leu Gly
   435 440 445
   Glu Thr Thr Ala Ala Gly Ala Leu Ile His Asp Asp Trp Val Leu Thr
   450 455 460
   Ala Ala His Ala Val Tyr Gly Lys Thr Glu Ala Met Ser Ser Leu Asp
   465 470 475 480
   Ile Arg Met Gly Ile Leu Lys Arg Leu Ser Leu Ile Tyr Thr Gln Ala
   485 490 495
   Trp Pro Glu Ala Val Phe Ile His Glu Gly Tyr Thr His Gly Ala Gly
   500 505 510
   Phe Asp Asn Asp Ile Ala Leu Ile Lys Leu Lys Asn Lys Val Thr Ile
   515 520 525
   Asn Arg Asn Ile Met Pro Ile Cys Leu Pro Arg Lys Glu Ala Ala Ser
   530 535 540
   Leu Met Lys Thr Asp Phe Val Gly Thr Val Ala Gly Trp Gly Leu Thr
   545 550 555 560
   Gln Lys Gly Phe Leu Ala Arg Asn Leu Met Phe Val Asp Ile Pro Ile
   565 570 575
   Val Asp His Gln Lys Cys Ala Thr Ala Tyr Thr Lys Gln Pro Tyr Pro
   580 585 590
   Gly Ala Lys Val Thr Val Asn Met Leu Cys Ala Gly Leu Asp Arg Gly
   595 600 605
   Gly Lys Asp Ser Cys Arg Gly Asp Ser Gly Gly Ala Leu Val Phe Leu
   610 615 620
   Asp Asn Glu Thr Gln Arg Trp Phe Val Gly Gly Ile Val Ser Trp Gly
   625 630 635 640
   Ser Ile Asn Cys Gly Gly Ser Glu Gln Tyr Gly Val Tyr Thr Lys Val
   645 650 655
   Thr Asn Tyr Ile Pro Trp Ile Glu Asn Ile Ile Asn Asn Phe
   660 665 670
<210> 56
   <211> 28
   <212> DNA
   <213> Artificial Sequence
<220>
   <223> Homo Sapien MASP-2 PCR primer
<400> 56
   atgaggctgc tgaccctcct gggccttc 28
<210> 57
   <211> 23
   <212> DNA
   <213> Artificial Sequence
<220>
   <223> Homo Sapien MASP-2 PCR primer
<400> 57
   gtgcccctcc tgcgtcacct ctg 23
<210> 58
   <211> 23
   <212> DNA
   <213> Artificial Sequence
<220>
   <223> Homo Sapien MASP-2 PCR primer
<400> 58
   cagaggtgac gcaggagggg cac 23
<210> 59
   <211> 27
   <212> DNA
   <213> Artificial Sequence
<220>
   <223> Homo Sapien MASP-2 PCR primer
<400> 59
   ttaaaatcac taattatgtt ctcgatc 27
<210> 60
   <211> 22
   <212> DNA
   <213> Artificial Sequence
<220>
   <223> Murine MASP-2 PCR primer
<400> 60
   atgaggctac tcatcttcct gg 22
<210> 61
   <211> 23
   <212> DNA
   <213> Artificial Sequence
<220>
   <223> Murine MASP-2 PCR primer
<400> 61
   ctgcagaggt gacgcagggg ggg 23
<210> 62
   <211> 23
   <212> DNA
   <213> Artificial Sequence
<220>
   <223> Murine MASP-2 PCR primer
<400> 62
   ccccccctgc gtcacctctg cag 23
<210> 63
   <211> 29
   <212> DNA
   <213> Artificial Sequence
<220>
   <223> Murine MASP-2 PCR primer
<400> 63
   ttagaaatta cttattatgt tctcaatcc 29
<210> 64
   <211> 29
   <212> DNA
   <213> Artificial Sequence
<220>
   <223> oligonucleotide from rat MASP-2
<400> 64
   gaggtgacgc aggaggggca ttagtgttt 29
<210> 65
   <211> 37
   <212> DNA
   <213> Artificial Sequence
<220>
   <223> oligonucleotide from rat MASP-2
<400> 65
   ctagaaacac taatgcccct cctgcgtcac ctctgca 37